# EUROPEAN PATENT APPLICATION

(11) **EP 4 019 536 A1**
(43) Date of publication of application: **29.06.2022**
(21) Application number: 20855549.0
(22) Date of filing: 19.08.2020
(51) Int. Cl.: C07K 14/54, C07K 19/00, C07K 16/28, C07K 14/715, A61K 39/00, C12N 15/12, C12N 15/63, A61P 35/00

(54) **IMMUNOCYTOKINE, PREPARATION FOR SAME, AND USES THEREOF**

(30) Priority: 19.08.2019 CN 201910764762
(71) Applicant: Nantong Yichen Biopharma. Co. Ltd., Nantong, Jiangsu 226001 (CN)
(72) Inventor: WANG, Feng, Shanghai 200233 (CN); ZHENG, Huayang, Shanghai 200233 (CN); ZHANG, Yuhan, Shanghai 200233 (CN)
(74) Representative: Novagraaf Technologies
(86) International application number: PCT/CN2020/109986
(87) International publication number: WO 2021/032116

(57) **Abstract**

The present disclosure provides an immunocytokine with IL-15 and IL-15Ra fused to an antibody targeting a tumor cell surface antigen, which can effectively and specifically target the complex of IL-15 and IL-15Ra to tumor microenvironment, and activate relevant immune cells within or in the vicinity of a tumor, thereby achieving the goal of tumor-specific killing, and at the same time, preventing immunotoxicity induced by the systemic hyperactivation of NK cells.

SEQUENCE LISTING

<110>  SHANGHAI YICHEN BIOMED CO.LTD
       WANG,Feng
       ZHENG,Huayang
       ZHANG,Yuhan

<120>  Immunocytokine, Preparation for the Same, and Uses Thereof

<160>  120

<170>  PatentIn version 3.5

<210>  1
<211>  1737
<212>  DNA
<213>  artificial sequence

<400>  1
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg caggtgaccc tgcgcgagtc cggccctgca     420

ctggtgaagc ccacccagac cctgaccctg acctgcacct tctccggctt ctccctgtcc     480

acctccggca tgtccgtggg ctggatccgg cagcctcccg gcaaggccct ggagtggctg     540

gctgacatct ggtgggacga caagaaggac tacaacccct ccctgaagtc ccgcctgacc     600

atctccaagg acacctccaa gaaccaggtg gtgctgaagg tgaccaacat ggaccccgcc     660

gacaccgcca cctactactg cgcccgctca atgattacca actggtactt cgacgtgtgg     720

ggagccggta ccaccgtgac cgtgtcttcc gcctccacca agggcccatc ggtcttcccc     780

ctggcaccct cctccaagag cacctctggg ggcacagcgg ccctgggctg cctggtcaag     840

gactacttcc ccgaaccggt gacggtgtcg tggaactcag gcgccctgac cagcggcgtg     900

cacaccttcc cggctgtcct acagtcctca ggactctact ccctcagcag cgtggtgact     960

gtgccctcta gcagcttggg cacccagacc tacatctgca acgtgaatca caagcccagc    1020

aacaccaagg tggacaagaa agttgaaccc aaatcttgcg acaaaactca cacatgccca    1080

ccgtgcccag cacctccagt cgccggaccg tcagtcttcc tcttccctcc aaaacccaag    1140

gacaccctca tgatctcccg gacccctgag gtcacatgcg tggtggtgga cgtgagccac    1200

gaagaccctg aggtcaagtt caactggtac gtggacggcg tggaggtgca taatgccaag    1260

acaaagccgc gggaggagca gtacaacagc acgtaccgtg tggtcagcgt cctcaccgtc    1320

ctgcaccagg actggctgaa tggcaaggag tacaagtgca aggtctccaa caaaggcctc 1380

ccaagctcca tcgagaaaac catctccaaa gccaaagggc agccccgaga accacaggtg 1440

tacaccctgc ctccatcccg ggatgagctg accaagaacc aggtcagcct gacctgcctg 1500

gtcaaaggct tctatcccag cgacatcgcc gtggagtggg agagcaatgg gcagccggag 1560

aacaactaca agaccacgcc tcccgtgctg gactccgacg gctccttctt cctctacagc 1620

aagctcaccg tggacaagag caggtggcag caggggaacg tcttctcatg ctccgtgatg 1680

catgaggctc tgcacaacca ctacacgcag aagagcctct ccctgtctcc gggtaaa 1737

<210> 2
<211> 579
<212> PRT
<213> artificial sequence

<400> 2

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Gln Val Thr Leu Arg Glu Ser Gly Pro Ala Leu Val Lys Pro
    130                 135                 140

Thr Gln Thr Leu Thr Leu Thr Cys Thr Phe Ser Gly Phe Ser Leu Ser
145                 150                 155                 160

Thr Ser Gly Met Ser Val Gly Trp Ile Arg Gln Pro Pro Gly Lys Ala

165                 170                 175

Leu Glu Trp Leu Ala Asp Ile Trp Trp Asp Asp Lys Lys Asp Tyr Asn
            180                 185                 190

Pro Ser Leu Lys Ser Arg Leu Thr Ile Ser Lys Asp Thr Ser Lys Asn
        195                 200                 205

Gln Val Val Leu Lys Val Thr Asn Met Asp Pro Ala Asp Thr Ala Thr
    210                 215                 220

Tyr Tyr Cys Ala Arg Ser Met Ile Thr Asn Trp Tyr Phe Asp Val Trp
225                 230                 235                 240

Gly Ala Gly Thr Thr Val Thr Val Ser Ser Ala Ser Thr Lys Gly Pro
                245                 250                 255

Ser Val Phe Pro Leu Ala Pro Ser Ser Lys Ser Thr Ser Gly Gly Thr
            260                 265                 270

Ala Ala Leu Gly Cys Leu Val Lys Asp Tyr Phe Pro Glu Pro Val Thr
        275                 280                 285

Val Ser Trp Asn Ser Gly Ala Leu Thr Ser Gly Val His Thr Phe Pro
    290                 295                 300

Ala Val Leu Gln Ser Ser Gly Leu Tyr Ser Leu Ser Ser Val Val Thr
305                 310                 315                 320

Val Pro Ser Ser Ser Leu Gly Thr Gln Thr Tyr Ile Cys Asn Val Asn
                325                 330                 335

His Lys Pro Ser Asn Thr Lys Val Asp Lys Lys Val Glu Pro Lys Ser
            340                 345                 350

Cys Asp Lys Thr His Thr Cys Pro Pro Cys Pro Ala Pro Pro Val Ala
        355                 360                 365

Gly Pro Ser Val Phe Leu Phe Pro Pro Lys Pro Lys Asp Thr Leu Met
    370                 375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val
                405                 410                 415

His Asn Ala Lys Thr Lys Pro Arg Glu Glu Gln Tyr Asn Ser Thr Tyr
            420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Gly Leu Pro Ser Ser Ile
    450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Asp Glu Leu Thr Lys Asn Gln Val Ser
                485                 490                 495

Leu Thr Cys Leu Val Lys Gly Phe Tyr Pro Ser Asp Ile Ala Val Glu
            500                 505                 510

Trp Glu Ser Asn Gly Gln Pro Glu Asn Asn Tyr Lys Thr Thr Pro Pro
        515                 520                 525

Val Leu Asp Ser Asp Gly Ser Phe Phe Leu Tyr Ser Lys Leu Thr Val
    530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys

<210>  3
<211>  1029
<212>  DNA
<213>  artificial sequence

<400>  3
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg gacatccaga tgacccagtc cccctccacc     420

ctgtccgcct ccgtgggcga ccgcgtgacc atcacctgca agtgccagct gtccgtgggc 480

tacatgcact ggtaccagca gaagcccggc aaggccccca agctgctgat ctacgacacc 540

tccaagctgg cctccggcgt gccctcccgc ttctccggct ccggctccgg caccgagttc 600

accctgacca tctcctccct gcagcccgac gacttcgcca cctactactg cttccagggc 660

tccggctacc ccttcacctt cggcggcggc accaagctgg agatcaaacg aactgtggct 720

gcaccatctg tcttcatctt cccgccatct gatgagcagt tgaaatctgg aactgcctct 780

gtcgtgtgcc tgctgaataa cttctatccc agagaggcca aagtacagtg gaaggtggat 840

aacgccctcc aatcgggtaa ctcccaggag agtgtcacag agcaggacag caaggacagc 900

acctacagcc tcagcagcac cctgacgctg agcaaagcag actacgagaa acacaaagtc 960

tacgcctgcg aagtcaccca tcagggcctg tcctcgcccg tcacaaagag cttcaacagg 1020

ggagagtgt 1029

<210> 4
<211> 343
<212> PRT
<213> artificial sequence

<400> 4

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Asp Ile Gln Met Thr Gln Ser Pro Ser Thr Leu Ser Ala Ser
    130                 135                 140

Val Gly Asp Arg Val Thr Ile Thr Cys Lys Cys Gln Leu Ser Val Gly
145                 150                 155                 160

Tyr Met His Trp Tyr Gln Gln Lys Pro Gly Lys Ala Pro Lys Leu Leu
                165                 170                 175

Ile Tyr Asp Thr Ser Lys Leu Ala Ser Gly Val Pro Ser Arg Phe Ser
            180                 185                 190

Gly Ser Gly Ser Gly Thr Glu Phe Thr Leu Thr Ile Ser Ser Leu Gln
        195                 200                 205

Pro Asp Asp Phe Ala Thr Tyr Tyr Cys Phe Gln Gly Ser Gly Tyr Pro
    210                 215                 220

Phe Thr Phe Gly Gly Gly Thr Lys Leu Glu Ile Lys Arg Thr Val Ala
225                 230                 235                 240

Ala Pro Ser Val Phe Ile Phe Pro Pro Ser Asp Glu Gln Leu Lys Ser
                245                 250                 255

Gly Thr Ala Ser Val Val Cys Leu Leu Asn Asn Phe Tyr Pro Arg Glu
            260                 265                 270

Ala Lys Val Gln Trp Lys Val Asp Asn Ala Leu Gln Ser Gly Asn Ser
        275                 280                 285

Gln Glu Ser Val Thr Glu Gln Asp Ser Lys Asp Ser Thr Tyr Ser Leu
    290                 295                 300

Ser Ser Thr Leu Thr Leu Ser Lys Ala Asp Tyr Glu Lys His Lys Val
305                 310                 315                 320

Tyr Ala Cys Glu Val Thr His Gln Gly Leu Ser Ser Pro Val Thr Lys
                325                 330                 335

Ser Phe Asn Arg Gly Glu Cys
            340

<210>  5
<211>  1590
<212>  DNA
<213>  artificial sequence

<400>  5
atcacctgcc cacctcccat gagcgtggag cacgccgaca tctgggtgaa gagctacagc        60

ctgtacagcc gcgagcgcta catctgcaac agcggcttca agcgcaaggc cggcaccagc 120

agcctgaccg agtgcgtgct gaacaaggcc accaacgtgg cccactggac cacccccagc 180

ctgaagtgca tccgcggcgg aggcggatcc ggaggcggag gttccggcgg gggtgggagc 240

gggcaggtga ccctgcgcga gtccggccct gcactggtga agcccaccca gaccctgacc 300

ctgacctgca ccttctccgg cttctccctg tccacctccg gcatgtccgt gggctggatc 360

cggcagcctc ccggcaaggc cctggagtgg ctggctgaca tctggtggga cgacaagaag 420

gactacaacc cctccctgaa gtcccgcctg accatctcca aggacacctc caagaaccag 480

gtggtgctga aggtgaccaa catggacccc gccgacaccg ccacctacta ctgcgcccgc 540

tcaatgatta ccaactggta cttcgacgtg tggggagccg gtaccaccgt gaccgtgtct 600

tccgcctcca ccaagggccc atcggtcttc cccctggcac cctcctccaa gagcacctct 660

gggggcacag cggccctggg ctgcctggtc aaggactact tccccgaacc ggtgacggtg 720

tcgtggaact caggcgccct gaccagcggc gtgcacacct tcccggctgt cctacagtcc 780

tcaggactct actccctcag cagcgtggtg actgtgccct ctagcagctt gggcacccag 840

acctacatct gcaacgtgaa tcacaagccc agcaacacca aggtggacaa gaaagttgaa 900

cccaaatctt gcgacaaaac tcacacatgc ccaccgtgcc cagcacctcc agtcgccgga 960

ccgtcagtct tcctcttccc tccaaaaccc aaggacaccc tcatgatctc ccggacccct 1020

gaggtcacat gcgtggtggt ggacgtgagc cacgaagacc ctgaggtcaa gttcaactgg 1080

tacgtggacg gcgtggaggt gcataatgcc aagacaaagc cgcgggagga gcagtacaac 1140

agcacgtacc gtgtggtcag cgtcctcacc gtcctgcacc aggactggct gaatggcaag 1200

gagtacaagt gcaaggtctc caacaaaggc ctcccaagct ccatcgagaa aaccatctcc 1260

aaagccaaag ggcagccccg agaaccacag gtgtacaccc tgcctccatc ccgggatgag 1320

ctgaccaaga accaggtcag cctgacctgc ctggtcaaag gcttctatcc cagcgacatc 1380

gccgtggagt gggagagcaa tgggcagccg gagaacaact acaagaccac gcctcccgtg 1440

ctggactccg acggctcctt cttcctctac agcaagctca ccgtggacaa gagcaggtgg 1500

cagcagggga acgtcttctc atgctccgtg atgcatgagg ctctgcacaa ccactacacg 1560

cagaagagcc tctccctgtc tccgggtaaa 1590

<210> 6
<211> 530
<212> PRT
<213> artificial sequence

<400> 6

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Gln Val Thr Leu Arg Glu Ser Gly Pro Ala Leu Val Lys Pro Thr
                85                  90                  95

Gln Thr Leu Thr Leu Thr Cys Thr Phe Ser Gly Phe Ser Leu Ser Thr
            100                 105                 110

Ser Gly Met Ser Val Gly Trp Ile Arg Gln Pro Pro Gly Lys Ala Leu
        115                 120                 125

Glu Trp Leu Ala Asp Ile Trp Trp Asp Asp Lys Lys Asp Tyr Asn Pro
    130                 135                 140

Ser Leu Lys Ser Arg Leu Thr Ile Ser Lys Asp Thr Ser Lys Asn Gln
145                 150                 155                 160

Val Val Leu Lys Val Thr Asn Met Asp Pro Ala Asp Thr Ala Thr Tyr
                165                 170                 175

Tyr Cys Ala Arg Ser Met Ile Thr Asn Trp Tyr Phe Asp Val Trp Gly
            180                 185                 190

Ala Gly Thr Thr Val Thr Val Ser Ser Ala Ser Thr Lys Gly Pro Ser
        195                 200                 205

Val Phe Pro Leu Ala Pro Ser Ser Lys Ser Thr Ser Gly Gly Thr Ala
    210                 215                 220

Ala Leu Gly Cys Leu Val Lys Asp Tyr Phe Pro Glu Pro Val Thr Val
225                 230                 235                 240

Ser Trp Asn Ser Gly Ala Leu Thr Ser Gly Val His Thr Phe Pro Ala
                245                 250                 255

Val Leu Gln Ser Ser Gly Leu Tyr Ser Leu Ser Ser Val Val Thr Val

260                 265                 270

Pro Ser Ser Ser Leu Gly Thr Gln Thr Tyr Ile Cys Asn Val Asn His
        275                 280                 285

Lys Pro Ser Asn Thr Lys Val Asp Lys Lys Val Glu Pro Lys Ser Cys
    290                 295                 300

Asp Lys Thr His Thr Cys Pro Pro Cys Pro Ala Pro Pro Val Ala Gly
305                 310                 315                 320

Pro Ser Val Phe Leu Phe Pro Pro Lys Pro Lys Asp Thr Leu Met Ile
                325                 330                 335

Ser Arg Thr Pro Glu Val Thr Cys Val Val Val Asp Val Ser His Glu
            340                 345                 350

Asp Pro Glu Val Lys Phe Asn Trp Tyr Val Asp Gly Val Glu Val His
        355                 360                 365

Asn Ala Lys Thr Lys Pro Arg Glu Glu Gln Tyr Asn Ser Thr Tyr Arg
    370                 375                 380

Val Val Ser Val Leu Thr Val Leu His Gln Asp Trp Leu Asn Gly Lys
385                 390                 395                 400

Glu Tyr Lys Cys Lys Val Ser Asn Lys Gly Leu Pro Ser Ser Ile Glu
                405                 410                 415

Lys Thr Ile Ser Lys Ala Lys Gly Gln Pro Arg Glu Pro Gln Val Tyr
            420                 425                 430

Thr Leu Pro Pro Ser Arg Asp Glu Leu Thr Lys Asn Gln Val Ser Leu
        435                 440                 445

Thr Cys Leu Val Lys Gly Phe Tyr Pro Ser Asp Ile Ala Val Glu Trp
    450                 455                 460

Glu Ser Asn Gly Gln Pro Glu Asn Asn Tyr Lys Thr Thr Pro Pro Val
465                 470                 475                 480

Leu Asp Ser Asp Gly Ser Phe Phe Leu Tyr Ser Lys Leu Thr Val Asp
                485                 490                 495

Lys Ser Arg Trp Gln Gln Gly Asn Val Phe Ser Cys Ser Val Met His
            500                 505                 510

Glu Ala Leu His Asn His Tyr Thr Gln Lys Ser Leu Ser Leu Ser Pro
        515                 520                 525

Gly Lys
    530

<210>  7
<211>  882
<212>  DNA
<213>  artificial sequence

<400>  7
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

ggggacatcc agatgaccca gtccccctcc accctgtccg cctccgtggg cgaccgcgtg     300

accatcacct gcaagtgcca gctgtccgtg ggctacatgc actggtacca gcagaagccc     360

ggcaaggccc ccaagctgct gatctacgac acctccaagc tggcctccgg cgtgccctcc     420

cgcttctccg gctccggctc cggcaccgag ttcaccctga ccatctcctc cctgcagccc     480

gacgacttcg ccacctacta ctgcttccag ggctccggct accccttcac cttcggcggc     540

ggcaccaagc tggagatcaa acgaactgtg gctgcaccat ctgtcttcat cttcccgcca     600

tctgatgagc agttgaaatc tggaactgcc tctgtcgtgt gcctgctgaa taacttctat     660

cccagagagg ccaaagtaca gtggaaggtg gataacgccc tccaatcggg taactcccag     720

gagagtgtca cagagcagga cagcaaggac agcacctaca gcctcagcag caccctgacg     780

ctgagcaaag cagactacga gaaacacaaa gtctacgcct gcgaagtcac ccatcagggc     840

ctgtcctcgc ccgtcacaaa gagcttcaac aggggagagt gt                        882

<210>  8
<211>  294
<212>  PRT
<213>  artificial sequence

<400>  8

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Asp Ile Gln Met Thr Gln Ser Pro Ser Thr Leu Ser Ala Ser Val
                85                  90                  95

Gly Asp Arg Val Thr Ile Thr Cys Lys Cys Gln Leu Ser Val Gly Tyr
            100                 105                 110

Met His Trp Tyr Gln Gln Lys Pro Gly Lys Ala Pro Lys Leu Leu Ile
        115                 120                 125

Tyr Asp Thr Ser Lys Leu Ala Ser Gly Val Pro Ser Arg Phe Ser Gly
    130                 135                 140

Ser Gly Ser Gly Thr Glu Phe Thr Leu Thr Ile Ser Ser Leu Gln Pro
145                 150                 155                 160

Asp Asp Phe Ala Thr Tyr Tyr Cys Phe Gln Gly Ser Gly Tyr Pro Phe
                165                 170                 175

Thr Phe Gly Gly Gly Thr Lys Leu Glu Ile Lys Arg Thr Val Ala Ala
            180                 185                 190

Pro Ser Val Phe Ile Phe Pro Pro Ser Asp Glu Gln Leu Lys Ser Gly
        195                 200                 205

Thr Ala Ser Val Val Cys Leu Leu Asn Asn Phe Tyr Pro Arg Glu Ala
    210                 215                 220

Lys Val Gln Trp Lys Val Asp Asn Ala Leu Gln Ser Gly Asn Ser Gln
225                 230                 235                 240

Glu Ser Val Thr Glu Gln Asp Ser Lys Asp Ser Thr Tyr Ser Leu Ser
                245                 250                 255

Ser Thr Leu Thr Leu Ser Lys Ala Asp Tyr Glu Lys His Lys Val Tyr
            260                 265                 270

Ala Cys Glu Val Thr His Gln Gly Leu Ser Ser Pro Val Thr Lys Ser
        275                 280                 285

Phe Asn Arg Gly Glu Cys
    290

<210> 9
<211> 1737
<212> DNA
<213> artificial sequence

<400> 9
aactgggtga acgtgatcag cgacctgaag aagatcgagg acctgatcca gagcatgcac 60

atcgacgcca ccctgtacac cgagagcgac gtgcacccca gctgcaaggt gaccgccatg 120

aagtgcttcc tgctggagct gcaggtgatc agcctggaga gcggcgacgc cagcatccac 180

gacaccgtgg agaacctgat catcctggcc aacgacagcc tgagcagcaa cggcaacgtg 240

accgagagcg gctgcaagga gtgcgaggag ctggaggaga agaacatcaa ggagttcctg 300

cagagcttcg tgcacatcgt gcagatgttc atcaacacca gcggcggagg cggatccgga 360

ggcggaggtt ccggcggggg tgggagcggg gaggtgcagc tggtggagtc tggaggaggc 420

ttggtccagc ctggggggtc cctgagactc tcctgtgcag cctctgggtt caatattaag 480

gacacttaca tccactgggt ccgccaggct ccagggaagg ggctggagtg ggtcgcacgt 540

atttatccta ccaatggtta cacacgctac gcagactccg tgaagggccg attcaccatc 600

tccgcagaca cttccaagaa cacggcgtat cttcaaatga acagcctgag agccgaggac 660

acggccgtgt attactgttc gagatggggc ggtgacggct tctatgccat ggactactgg 720

ggccaaggaa ccctggtcac cgtctcctca gcctccacca agggcccatc ggtcttcccc 780

ctggcaccct cctccaagag cacctctggg ggcacagcgg ccctgggctg cctggtcaag 840

gactacttcc ccgaaccggt gacggtgtcg tggaactcag gcgccctgac cagcggcgtg 900

cacaccttcc cggctgtcct acagtcctca ggactctact ccctcagcag cgtggtgact 960

gtgccctcta gcagcttggg cacccagacc tacatctgca acgtgaatca caagcccagc 1020

aacaccaagg tggacaagaa agttgagccc aaatcttgcg acaaaactca cacatgccca 1080

ccgtgcccag cacctccagt cgccggaccg tcagtcttcc tcttccctcc aaaacccaag 1140

gacaccctca tgatctcccg gacccctgag gtcacatgcg tggtggtgga cgtgagccac 1200

gaagaccctg aggtcaagtt caactggtac gtggacggcg tggaggtgca taatgccaag 1260

acaaagccgc gggaggagca gtacaacagc acgtaccgtg tggtcagcgt cctcaccgtc 1320

ctgcaccagg actggctgaa tggcaaggag tacaagtgca aggtctccaa caaaggcctc 1380

ccaagctcca tcgagaaaac catctccaaa gccaaagggc agccccgaga accacaggtg 1440

tacaccctgc ctccatcccg ggatgagctg accaagaacc aggtcagcct gacctgcctg 1500

gtcaaaggct tctatcccag cgacatcgcc gtggagtggg agagcaatgg gcagccggag 1560

aacaactaca agaccacgcc tcccgtgctg gactccgacg gctccttctt cctctacagc 1620

aagctcaccg tggacaagag caggtggcag caggggaacg tcttctcatg ctccgtgatg 1680

catgaggctc tgcacaacca ctacacgcag aagagcctct ccctgtctcc gggtaaa          1737

<210>  10
<211>  579
<212>  PRT
<213>  artificial sequence

<400>  10

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Glu Val Gln Leu Val Glu Ser Gly Gly Gly Leu Val Gln Pro
    130                 135                 140

Gly Gly Ser Leu Arg Leu Ser Cys Ala Ala Ser Gly Phe Asn Ile Lys
145                 150                 155                 160

Asp Thr Tyr Ile His Trp Val Arg Gln Ala Pro Gly Lys Gly Leu Glu
                165                 170                 175

Trp Val Ala Arg Ile Tyr Pro Thr Asn Gly Tyr Thr Arg Tyr Ala Asp
            180                 185                 190

Ser Val Lys Gly Arg Phe Thr Ile Ser Ala Asp Thr Ser Lys Asn Thr
        195                 200                 205

Ala Tyr Leu Gln Met Asn Ser Leu Arg Ala Glu Asp Thr Ala Val Tyr
    210                 215                 220

Tyr Cys Ser Arg Trp Gly Gly Asp Gly Phe Tyr Ala Met Asp Tyr Trp
225                 230                 235                 240

Gly Gln Gly Thr Leu Val Thr Val Ser Ser Ala Ser Thr Lys Gly Pro
                245                 250                 255

Ser Val Phe Pro Leu Ala Pro Ser Ser Lys Ser Thr Ser Gly Gly Thr
            260                 265                 270

Ala Ala Leu Gly Cys Leu Val Lys Asp Tyr Phe Pro Glu Pro Val Thr
        275                 280                 285

Val Ser Trp Asn Ser Gly Ala Leu Thr Ser Gly Val His Thr Phe Pro
    290                 295                 300

Ala Val Leu Gln Ser Ser Gly Leu Tyr Ser Leu Ser Ser Val Val Thr
305                 310                 315                 320

Val Pro Ser Ser Ser Leu Gly Thr Gln Thr Tyr Ile Cys Asn Val Asn
                325                 330                 335

His Lys Pro Ser Asn Thr Lys Val Asp Lys Lys Val Glu Pro Lys Ser
            340                 345                 350

Cys Asp Lys Thr His Thr Cys Pro Pro Cys Pro Ala Pro Pro Val Ala
        355                 360                 365

Gly Pro Ser Val Phe Leu Phe Pro Pro Lys Pro Lys Asp Thr Leu Met
    370                 375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val
                405                 410                 415

His Asn Ala Lys Thr Lys Pro Arg Glu Glu Gln Tyr Asn Ser Thr Tyr
            420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Gly Leu Pro Ser Ser Ile
    450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Asp Glu Leu Thr Lys Asn Gln Val Ser
                485                 490                 495

Leu Thr Cys Leu Val Lys Gly Phe Tyr Pro Ser Asp Ile Ala Val Glu
            500                 505                 510

Trp Glu Ser Asn Gly Gln Pro Glu Asn Asn Tyr Lys Thr Thr Pro Pro
        515                 520                 525

Val Leu Asp Ser Asp Gly Ser Phe Phe Leu Tyr Ser Lys Leu Thr Val
    530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys

<210>  11
<211>  885
<212>  DNA
<213>  artificial sequence

<400>  11
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

ggggacatcc agatgaccca gtctccatcc tccctgtctg catctgtagg agacagagtc     300

accatcactt gccgggcaag tcaggatgtg aataccgcgg tcgcatggta tcagcagaaa     360

ccagggaaag cccctaagct cctgatctat tctgcatcct tcttgtatag tggggtccca     420

tcaaggttca gtggcagtag atctgggaca gatttcactc tcaccatcag cagtctgcaa     480

cctgaagatt ttgcaactta ctactgtcaa cagcattaca ctacccctcc gacgttcggc     540

caaggtacca aggtggagat caaacgaact gtggctgcac catctgtctt catcttcccg     600

ccatctgatg agcagttgaa atctggaact gcctctgtcg tgtgcctgct gaataacttc     660

tatcccagag aggccaaagt acagtggaag gtggataacg ccctccaatc gggtaactcc     720

caggagagtg tcacagagca ggacagcaag gacagcacct acagcctcag cagcaccctg 780

acgctgagca aagcagacta cgagaaacac aaagtctacg cctgcgaagt cacccatcag 840

ggcctgtcct cgcccgtcac aaagagcttc aacaggggag agtgt 885

<210> 12
<211> 295
<212> PRT
<213> artificial sequence

<400> 12

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Asp Ile Gln Met Thr Gln Ser Pro Ser Ser Leu Ser Ala Ser Val
                85                  90                  95

Gly Asp Arg Val Thr Ile Thr Cys Arg Ala Ser Gln Asp Val Asn Thr
            100                 105                 110

Ala Val Ala Trp Tyr Gln Gln Lys Pro Gly Lys Ala Pro Lys Leu Leu
        115                 120                 125

Ile Tyr Ser Ala Ser Phe Leu Tyr Ser Gly Val Pro Ser Arg Phe Ser
    130                 135                 140

Gly Ser Arg Ser Gly Thr Asp Phe Thr Leu Thr Ile Ser Ser Leu Gln
145                 150                 155                 160

Pro Glu Asp Phe Ala Thr Tyr Tyr Cys Gln Gln His Tyr Thr Thr Pro
                165                 170                 175

Pro Thr Phe Gly Gln Gly Thr Lys Val Glu Ile Lys Arg Thr Val Ala
            180                 185                 190

Ala Pro Ser Val Phe Ile Phe Pro Pro Ser Asp Glu Gln Leu Lys Ser

195                 200                 205

Gly Thr Ala Ser Val Val Cys Leu Leu Asn Asn Phe Tyr Pro Arg Glu
    210                 215                 220

Ala Lys Val Gln Trp Lys Val Asp Asn Ala Leu Gln Ser Gly Asn Ser
225                 230                 235                 240

Gln Glu Ser Val Thr Glu Gln Asp Ser Lys Asp Ser Thr Tyr Ser Leu
                245                 250                 255

Ser Ser Thr Leu Thr Leu Ser Lys Ala Asp Tyr Glu Lys His Lys Val
            260                 265                 270

Tyr Ala Cys Glu Val Thr His Gln Gly Leu Ser Ser Pro Val Thr Lys
        275                 280                 285

Ser Phe Asn Arg Gly Glu Cys
    290                 295

<210>  13
<211>  1590
<212>  DNA
<213>  artificial sequence

<400>  13
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

ggggaggtgc agctggtgga gtctggagga ggcttggtcc agcctggggg gtccctgaga     300

ctctcctgtg cagcctctgg gttcaatatt aaggacactt acatccactg ggtccgccag     360

gctccaggga aggggctgga gtgggtcgca cgtatttatc ctaccaatgg ttacacacgc     420

tacgcagact ccgtgaaggg ccgattcacc atctccgcag acacttccaa gaacacggcg     480

tatcttcaaa tgaacagcct gagagccgag gacacggccg tgtattactg ttcgagatgg     540

ggcggtgacg gcttctatgc catggactac tggggccaag gaaccctggt caccgtctcc     600

tcagcctcca ccaagggccc atcggtcttc cccctggcac cctcctccaa gagcacctct     660

gggggcacag cggccctggg ctgcctggtc aaggactact tccccgaacc ggtgacggtg     720

tcgtggaact caggcgccct gaccagcggc gtgcacacct tcccggctgt cctacagtcc     780

tcaggactct actccctcag cagcgtggtg actgtgccct ctagcagctt gggcacccag     840

acctacatct gcaacgtgaa tcacaagccc agcaacacca aggtggacaa gaaagttgag     900

cccaaatctt gcgacaaaac tcacacatgc ccaccgtgcc cagcacctcc agtcgccgga 960

ccgtcagtct tcctcttccc tccaaaaccc aaggacaccc tcatgatctc ccggacccct 1020

gaggtcacat gcgtggtggt ggacgtgagc cacgaagacc ctgaggtcaa gttcaactgg 1080

tacgtggacg gcgtggaggt gcataatgcc aagacaaagc cgcgggagga gcagtacaac 1140

agcacgtacc gtgtggtcag cgtcctcacc gtcctgcacc aggactggct gaatggcaag 1200

gagtacaagt gcaaggtctc caacaaaggc ctcccaagct ccatcgagaa aaccatctcc 1260

aaagccaaag ggcagccccg agaaccacag gtgtacaccc tgcctccatc ccgggatgag 1320

ctgaccaaga accaggtcag cctgacctgc ctggtcaaag gcttctatcc cagcgacatc 1380

gccgtggagt gggagagcaa tgggcagccg gagaacaact acaagaccac gcctcccgtg 1440

ctggactccg acggctcctt cttcctctac agcaagctca ccgtggacaa gagcaggtgg 1500

cagcagggga acgtcttctc atgctccgtg atgcatgagg ctctgcacaa ccactacacg 1560

cagaagagcc tctccctgtc tccgggtaaa 1590

<210> 14
<211> 530
<212> PRT
<213> artificial sequence

<400> 14

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Glu Val Gln Leu Val Glu Ser Gly Gly Gly Leu Val Gln Pro Gly
                85                  90                  95

Gly Ser Leu Arg Leu Ser Cys Ala Ala Ser Gly Phe Asn Ile Lys Asp
            100                 105                 110

Thr Tyr Ile His Trp Val Arg Gln Ala Pro Gly Lys Gly Leu Glu Trp
        115                 120                 125

Val Ala Arg Ile Tyr Pro Thr Asn Gly Tyr Thr Arg Tyr Ala Asp Ser
    130                 135                 140

Val Lys Gly Arg Phe Thr Ile Ser Ala Asp Thr Ser Lys Asn Thr Ala
145                 150                 155                 160

Tyr Leu Gln Met Asn Ser Leu Arg Ala Glu Asp Thr Ala Val Tyr Tyr
                165                 170                 175

Cys Ser Arg Trp Gly Gly Asp Gly Phe Tyr Ala Met Asp Tyr Trp Gly
            180                 185                 190

Gln Gly Thr Leu Val Thr Val Ser Ser Ala Ser Thr Lys Gly Pro Ser
        195                 200                 205

Val Phe Pro Leu Ala Pro Ser Ser Lys Ser Thr Ser Gly Gly Thr Ala
    210                 215                 220

Ala Leu Gly Cys Leu Val Lys Asp Tyr Phe Pro Glu Pro Val Thr Val
225                 230                 235                 240

Ser Trp Asn Ser Gly Ala Leu Thr Ser Gly Val His Thr Phe Pro Ala
                245                 250                 255

Val Leu Gln Ser Ser Gly Leu Tyr Ser Leu Ser Ser Val Val Thr Val
            260                 265                 270

Pro Ser Ser Ser Leu Gly Thr Gln Thr Tyr Ile Cys Asn Val Asn His
        275                 280                 285

Lys Pro Ser Asn Thr Lys Val Asp Lys Lys Val Glu Pro Lys Ser Cys
    290                 295                 300

Asp Lys Thr His Thr Cys Pro Pro Cys Pro Ala Pro Pro Val Ala Gly
305                 310                 315                 320

Pro Ser Val Phe Leu Phe Pro Pro Lys Pro Lys Asp Thr Leu Met Ile
                325                 330                 335

Ser Arg Thr Pro Glu Val Thr Cys Val Val Val Asp Val Ser His Glu
            340                 345                 350

Asp Pro Glu Val Lys Phe Asn Trp Tyr Val Asp Gly Val Glu Val His
        355                 360                 365

Asn Ala Lys Thr Lys Pro Arg Glu Glu Gln Tyr Asn Ser Thr Tyr Arg
    370                 375                 380

Val Val Ser Val Leu Thr Val Leu His Gln Asp Trp Leu Asn Gly Lys
385                 390                 395                 400

Glu Tyr Lys Cys Lys Val Ser Asn Lys Gly Leu Pro Ser Ser Ile Glu
                405                 410                 415

Lys Thr Ile Ser Lys Ala Lys Gly Gln Pro Arg Glu Pro Gln Val Tyr
            420                 425                 430

Thr Leu Pro Pro Ser Arg Asp Glu Leu Thr Lys Asn Gln Val Ser Leu
        435                 440                 445

Thr Cys Leu Val Lys Gly Phe Tyr Pro Ser Asp Ile Ala Val Glu Trp
    450                 455                 460

Glu Ser Asn Gly Gln Pro Glu Asn Asn Tyr Lys Thr Thr Pro Pro Val
465                 470                 475                 480

Leu Asp Ser Asp Gly Ser Phe Phe Leu Tyr Ser Lys Leu Thr Val Asp
                485                 490                 495

Lys Ser Arg Trp Gln Gln Gly Asn Val Phe Ser Cys Ser Val Met His
            500                 505                 510

Glu Ala Leu His Asn His Tyr Thr Gln Lys Ser Leu Ser Leu Ser Pro
        515                 520                 525

Gly Lys
    530

<210>  15
<211>  1032
<212>  DNA
<213>  artificial sequence

<400>  15
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg gacatccaga tgacccagtc tccatcctcc     420

ctgtctgcat ctgtaggaga cagagtcacc atcacttgcc gggcaagtca ggatgtgaat     480

accgcggtcg catggtatca gcagaaacca gggaaagccc ctaagctcct gatctattct 540

gcatccttct tgtatagtgg ggtcccatca aggttcagtg gcagtagatc tgggacagat 600

ttcactctca ccatcagcag tctgcaacct gaagattttg caacttacta ctgtcaacag 660

cattacacta cccctccgac gttcggccaa ggtaccaagg tggagatcaa acgaactgtg 720

gctgcaccat ctgtcttcat cttcccgcca tctgatgagc agttgaaatc tggaactgcc 780

tctgtcgtgt gcctgctgaa taacttctat cccagagagg ccaaagtaca gtggaaggtg 840

gataacgccc tccaatcggg taactcccag gagagtgtca cagagcagga cagcaaggac 900

agcacctaca gcctcagcag caccctgacg ctgagcaaag cagactacga gaaacacaaa 960

gtctacgcct gcgaagtcac ccatcagggc ctgtcctcgc ccgtcacaaa gagcttcaac 1020

aggggagagt gt 1032

<210> 16
<211> 344
<212> PRT
<213> artificial sequence

<400> 16

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Asp Ile Gln Met Thr Gln Ser Pro Ser Ser Leu Ser Ala Ser
    130                 135                 140

Val Gly Asp Arg Val Thr Ile Thr Cys Arg Ala Ser Gln Asp Val Asn
145                 150                 155                 160

Thr Ala Val Ala Trp Tyr Gln Gln Lys Pro Gly Lys Ala Pro Lys Leu
                165                 170                 175

Leu Ile Tyr Ser Ala Ser Phe Leu Tyr Ser Gly Val Pro Ser Arg Phe
            180                 185                 190

Ser Gly Ser Arg Ser Gly Thr Asp Phe Thr Leu Thr Ile Ser Ser Leu
        195                 200                 205

Gln Pro Glu Asp Phe Ala Thr Tyr Tyr Cys Gln Gln His Tyr Thr Thr
    210                 215                 220

Pro Pro Thr Phe Gly Gln Gly Thr Lys Val Glu Ile Lys Arg Thr Val
225                 230                 235                 240

Ala Ala Pro Ser Val Phe Ile Phe Pro Pro Ser Asp Glu Gln Leu Lys
                245                 250                 255

Ser Gly Thr Ala Ser Val Val Cys Leu Leu Asn Asn Phe Tyr Pro Arg
            260                 265                 270

Glu Ala Lys Val Gln Trp Lys Val Asp Asn Ala Leu Gln Ser Gly Asn
        275                 280                 285

Ser Gln Glu Ser Val Thr Glu Gln Asp Ser Lys Asp Ser Thr Tyr Ser
    290                 295                 300

Leu Ser Ser Thr Leu Thr Leu Ser Lys Ala Asp Tyr Glu Lys His Lys
305                 310                 315                 320

Val Tyr Ala Cys Glu Val Thr His Gln Gly Leu Ser Ser Pro Val Thr
                325                 330                 335

Lys Ser Phe Asn Arg Gly Glu Cys
            340

<210>  17
<211>  642
<212>  DNA
<213>  artificial sequence

<400>  17
gacatccaga tgacccagtc tccatcctcc ctgtctgcat ctgtaggaga cagagtcacc      60

atcacttgcc gggcaagtca ggatgtgaat accgcggtcg catggtatca gcagaaacca      120

gggaaagccc ctaagctcct gatctattct gcatccttct tgtatagtgg ggtcccatca 180

aggttcagtg gcagtagatc tgggacagat ttcactctca ccatcagcag tctgcaacct 240

gaagattttg caacttacta ctgtcaacag cattacacta cccctccgac gttcggccaa 300

ggtaccaagg tggagatcaa acgaactgtg gctgcaccat ctgtcttcat cttcccgcca 360

tctgatgagc agttgaaatc tggaactgcc tctgtcgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg 540

ctgagcaaag cagactacga gaaacacaaa gtctacgcct gcgaagtcac ccatcagggc 600

ctgtcctcgc ccgtcacaaa gagcttcaac aggggagagt gt 642

<210> 18
<211> 450
<212> PRT
<213> artificial sequence

<400> 18

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  19
<211>  642
<212>  DNA
<213>  artificial sequence

<400>  19
gacatccaga tgacccagtc tccatcctcc ctgtctgcat ctgtaggaga cagagtcacc      60

atcacttgcc gggcaagtca ggatgtgaat accgcggtcg catggtatca gcagaaacca     120

gggaaagccc ctaagctcct gatctattct gcatccttct tgtatagtgg ggtcccatca     180

aggttcagtg gcagtagatc tgggacagat ttcactctca ccatcagcag tctgcaacct     240

gaagattttg caacttacta ctgtcaacag cattacacta cccctccgac gttcggccaa     300

ggtaccaagg tggagatcaa acgaactgtg gctgcaccat ctgtcttcat cttcccgcca     360

tctgatgagc agttgaaatc tggaactgcc tctgtcgtgt gcctgctgaa taacttctat     420

cccagagagg ccaaagtaca gtggaaggtg gataacgccc tccaatcggg taactcccag     480

gagagtgtca cagagcagga cagcaaggac agcacctaca gcctcagcag caccctgacg     540

ctgagcaaag cagactacga gaaacacaaa gtctacgcct gcgaagtcac ccatcagggc     600

ctgtcctcgc ccgtcacaaa gagcttcaac aggggagagt gt                        642

<210>  20
<211>  214
<212>  PRT
<213>  artificial sequence

<400>  20

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  21
<211>  1737
<212>  DNA
<213>  artificial sequence

<400>  21
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg caggtgcagc tgcaggagag cggccccggc 420

ctggtgaagc ccagcgagac cctgagcctg acctgcaccg tgagcggcgg cagcgtgagc 480

agcggcgact actactggac ctggatccgc cagagccccg gcaagggcct ggagtggatc 540

ggccacatct actacagcgg caacaccaac tacaacccca gcctgaagag ccgcctgacc 600

atcagcatcg acaccagcaa gacccagttc agcctgaagc tgagcagcgt gaccgccgcc 660

gacaccgcca tctactactg cgtgcgcgac cgcgtgaccg gcgccttcga catctggggc 720

cagggcacca tggtgactgt gtctagcgcc tccaccaagg gcccatcggt cttccccctg 780

gcaccctcct ccaagagcac ctctgggggc acagcggccc tgggctgcct ggtcaaggac 840

tacttccccg aaccggtgac ggtgtcgtgg aactcaggcg ccctgaccag cggcgtgcac 900

accttcccgg ctgtcctaca gtcctcagga ctctactccc tcagcagcgt ggtgactgtg 960

ccctctagca gcttgggcac ccagacctac atctgcaacg tgaatcacaa gcccagcaac 1020

accaaggtgg acaagaaagt tgaacccaaa tcttgcgaca aaactcacac atgcccaccg 1080

tgcccagcac ctgaactcct ggggggaccg tcagtcttcc tcttcccccc aaaacccaag 1140

gacaccctca tgatctcccg gacccctgag gtcacatgcg tggtggtgga cgtgagccac 1200

gaagaccctg aggtcaagtt caactggtac gtggacggcg tggaggtgca taatgccaag 1260

acaaagccgc gggaggagca gtacaacagc acgtaccgtg tggtcagcgt cctcaccgtc 1320

ctgcaccagg actggctgaa tggcaaggag tacaagtgca aggtctccaa caaagccctc 1380

ccagccccca tagagaaaac catctccaaa gccaaagggc agccccgaga accacaggtg 1440

tacaccctgc ccccatcccg ggaggagatg accaagaacc aggtcagcct gacctgcctg 1500

gtcaaaggct tctatcccag cgacatcgcc gtggagtggg agagcaatgg gcagccggag 1560

aacaactaca agaccacgcc tcccgtgctg gactccgacg gctccttctt cctctacagc 1620

aagctcaccg tggacaagag caggtggcag caggggaacg tcttctcatg ctccgtgatg 1680

catgaggctc tgcacaacca ctacacgcag aagagcctct ccctgtctcc gggtaaa 1737

<210> 22
<211> 579
<212> PRT
<213> artificial sequence

<400> 22

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Gln Val Gln Leu Gln Glu Ser Gly Pro Gly Leu Val Lys Pro
    130                 135                 140

Ser Glu Thr Leu Ser Leu Thr Cys Thr Val Ser Gly Gly Ser Val Ser
145                 150                 155                 160

Ser Gly Asp Tyr Tyr Trp Thr Trp Ile Arg Gln Ser Pro Gly Lys Gly
                165                 170                 175

Leu Glu Trp Ile Gly His Ile Tyr Tyr Ser Gly Asn Thr Asn Tyr Asn
            180                 185                 190

Pro Ser Leu Lys Ser Arg Leu Thr Ile Ser Ile Asp Thr Ser Lys Thr
        195                 200                 205

Gln Phe Ser Leu Lys Leu Ser Ser Val Thr Ala Ala Asp Thr Ala Ile
    210                 215                 220

Tyr Tyr Cys Val Arg Asp Arg Val Thr Gly Ala Phe Asp Ile Trp Gly
225                 230                 235                 240

Gln Gly Thr Met Val Thr Val Ser Ser Ala Ser Thr Lys Gly Pro Ser
                245                 250                 255

Val Phe Pro Leu Ala Pro Ser Ser Lys Ser Thr Ser Gly Gly Thr Ala
            260                 265                 270

Ala Leu Gly Cys Leu Val Lys Asp Tyr Phe Pro Glu Pro Val Thr Val
        275                 280                 285

Ser Trp Asn Ser Gly Ala Leu Thr Ser Gly Val His Thr Phe Pro Ala
    290                 295                 300

Val Leu Gln Ser Ser Gly Leu Tyr Ser Leu Ser Ser Val Val Thr Val
305                 310                 315                 320

Pro Ser Ser Ser Leu Gly Thr Gln Thr Tyr Ile Cys Asn Val Asn His
                325                 330                 335

Lys Pro Ser Asn Thr Lys Val Asp Lys Lys Val Glu Pro Lys Ser Cys
            340                 345                 350

Asp Lys Thr His Thr Cys Pro Pro Cys Pro Ala Pro Glu Leu Leu Gly
        355                 360                 365

Gly Pro Ser Val Phe Leu Phe Pro Pro Lys Pro Lys Asp Thr Leu Met
    370                 375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val
                405                 410                 415

His Asn Ala Lys Thr Lys Pro Arg Glu Glu Gln Tyr Asn Ser Thr Tyr
            420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Ala Leu Pro Ala Pro Ile
    450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Glu Glu Met Thr Lys Asn Gln Val Ser
                485                 490                 495

Leu Thr Cys Leu Val Lys Gly Phe Tyr Pro Ser Asp Ile Ala Val Glu
            500                 505                 510

Trp Glu Ser Asn Gly Gln Pro Glu Asn Asn Tyr Lys Thr Thr Pro Pro
        515                 520                 525

Val Leu Asp Ser Asp Gly Ser Phe Phe Leu Tyr Ser Lys Leu Thr Val
    530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys

<210>  23
<211>  885
<212>  DNA
<213>  artificial sequence

<400>  23
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

ggggacatcc agatgaccca gagccccagc agcctgagcg ccagcgtggg cgaccgcgtg     300

accatcacct gccaggccag ccaggacatc agcaactacc tgaactggta ccagcagaag     360

cccggcaagg cccccaagct gctgatctac gacgccagca acctggagac cggcgtgccc     420

agccgcttca gcggcagcgg cagcggcacc gacttcacct tcaccatcag cagcctgcag     480

cccgaggaca tcgccaccta cttctgccag cacttcgacc acctgcccct ggccttcggc     540

ggcggcacca aggtggagat caagcgcaca gtggcagccc ccagcgtctt catttttccc     600

ccttccgatg aacagctgaa gtccggcact gcttctgtgg tctgtctgct gaacaatttc     660

tatcccagag aggccaaggt gcagtggaaa gtggacaacg ctctgcagtc cggcaacagc     720

caggagagtg tgaccgaaca ggatagtaag gacagcacat attctctgtc tagtaccctg     780

acactgagta aggcagatta cgagaagcac aaagtgtatg cctgcgaagt cactcatcag     840

ggactgtcaa gccccgtgac caagagcttc aaccggggcg agtgt                     885

<210>  24
<211>  295
<212>  PRT
<213>  artificial sequence

<400>  24

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly

20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Asp Ile Gln Met Thr Gln Ser Pro Ser Ser Leu Ser Ala Ser Val
                85                  90                  95

Gly Asp Arg Val Thr Ile Thr Cys Gln Ala Ser Gln Asp Ile Ser Asn
            100                 105                 110

Tyr Leu Asn Trp Tyr Gln Gln Lys Pro Gly Lys Ala Pro Lys Leu Leu
        115                 120                 125

Ile Tyr Asp Ala Ser Asn Leu Glu Thr Gly Val Pro Ser Arg Phe Ser
    130                 135                 140

Gly Ser Gly Ser Gly Thr Asp Phe Thr Phe Thr Ile Ser Ser Leu Gln
145                 150                 155                 160

Pro Glu Asp Ile Ala Thr Tyr Phe Cys Gln His Phe Asp His Leu Pro
                165                 170                 175

Leu Ala Phe Gly Gly Gly Thr Lys Val Glu Ile Lys Arg Thr Val Ala
            180                 185                 190

Ala Pro Ser Val Phe Ile Phe Pro Pro Ser Asp Glu Gln Leu Lys Ser
        195                 200                 205

Gly Thr Ala Ser Val Val Cys Leu Leu Asn Asn Phe Tyr Pro Arg Glu
    210                 215                 220

Ala Lys Val Gln Trp Lys Val Asp Asn Ala Leu Gln Ser Gly Asn Ser
225                 230                 235                 240

Gln Glu Ser Val Thr Glu Gln Asp Ser Lys Asp Ser Thr Tyr Ser Leu
                245                 250                 255

Ser Ser Thr Leu Thr Leu Ser Lys Ala Asp Tyr Glu Lys His Lys Val
            260                 265                 270

Tyr Ala Cys Glu Val Thr His Gln Gly Leu Ser Ser Pro Val Thr Lys
        275                 280                 285

Ser Phe Asn Arg Gly Glu Cys
    290                 295

<210>  25
<211>  1590
<212>  DNA
<213>  artificial sequence

<400>  25
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

gggcaggtgc agctgcagga gagcggcccc ggcctggtga agcccagcga gaccctgagc     300

ctgacctgca ccgtgagcgg cggcagcgtg agcagcggcg actactactg gacctggatc     360

cgccagagcc ccggcaaggg cctggagtgg atcggccaca tctactacag cggcaacacc     420

aactacaacc ccagcctgaa gagccgcctg accatcagca tcgacaccag caagacccag     480

ttcagcctga agctgagcag cgtgaccgcc gccgacaccg ccatctacta ctgcgtgcgc     540

gaccgcgtga ccggcgcctt cgacatctgg ggccagggca ccatggtgac tgtgtctagc     600

gcctccacca agggcccatc ggtcttcccc ctggcaccct cctccaagag cacctctggg     660

ggcacagcgg ccctgggctg cctggtcaag gactacttcc ccgaaccggt gacggtgtcg     720

tggaactcag gcgccctgac cagcggcgtg cacaccttcc cggctgtcct acagtcctca     780

ggactctact ccctcagcag cgtggtgact gtgccctcta gcagcttggg cacccagacc     840

tacatctgca acgtgaatca caagcccagc aacaccaagg tggacaagaa agttgaaccc     900

aaatcttgcg acaaaactca cacatgccca ccgtgcccag cacctgaact cctgggggga     960

ccgtcagtct tcctcttccc cccaaaaccc aaggacaccc tcatgatctc ccggacccct    1020

gaggtcacat gcgtggtggt ggacgtgagc cacgaagacc ctgaggtcaa gttcaactgg    1080

tacgtggacg gcgtggaggt gcataatgcc aagacaaagc cgcgggagga gcagtacaac    1140

agcacgtacc gtgtggtcag cgtcctcacc gtcctgcacc aggactggct gaatggcaag    1200

gagtacaagt gcaaggtctc caacaaagcc ctcccagccc ccatagagaa aaccatctcc    1260

aaagccaaag ggcagccccg agaaccacag gtgtacaccc tgcccccatc ccgggaggag    1320

atgaccaaga accaggtcag cctgacctgc ctggtcaaag gcttctatcc cagcgacatc    1380

gccgtggagt gggagagcaa tgggcagccg gagaacaact acaagaccac gcctcccgtg    1440

ctggactccg acggctcctt cttcctctac agcaagctca ccgtggacaa gagcaggtgg    1500

cagcagggga acgtcttctc atgctccgtg atgcatgagg ctctgcacaa ccactacacg 1560

cagaagagcc tctccctgtc tccgggtaaa 1590

<210> 26
<211> 530
<212> PRT
<213> artificial sequence

<400> 26

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Gln Val Gln Leu Gln Glu Ser Gly Pro Gly Leu Val Lys Pro Ser
                85                  90                  95

Glu Thr Leu Ser Leu Thr Cys Thr Val Ser Gly Gly Ser Val Ser Ser
            100                 105                 110

Gly Asp Tyr Tyr Trp Thr Trp Ile Arg Gln Ser Pro Gly Lys Gly Leu
        115                 120                 125

Glu Trp Ile Gly His Ile Tyr Tyr Ser Gly Asn Thr Asn Tyr Asn Pro
    130                 135                 140

Ser Leu Lys Ser Arg Leu Thr Ile Ser Ile Asp Thr Ser Lys Thr Gln
145                 150                 155                 160

Phe Ser Leu Lys Leu Ser Ser Val Thr Ala Ala Asp Thr Ala Ile Tyr
                165                 170                 175

Tyr Cys Val Arg Asp Arg Val Thr Gly Ala Phe Asp Ile Trp Gly Gln
            180                 185                 190

Gly Thr Met Val Thr Val Ser Ser Ala Ser Thr Lys Gly Pro Ser Val
        195                 200                 205

Phe Pro Leu Ala Pro Ser Ser Lys Ser Thr Ser Gly Gly Thr Ala Ala
    210                 215                 220

Leu Gly Cys Leu Val Lys Asp Tyr Phe Pro Glu Pro Val Thr Val Ser
225                 230                 235                 240

Trp Asn Ser Gly Ala Leu Thr Ser Gly Val His Thr Phe Pro Ala Val
                245                 250                 255

Leu Gln Ser Ser Gly Leu Tyr Ser Leu Ser Ser Val Val Thr Val Pro
            260                 265                 270

Ser Ser Ser Leu Gly Thr Gln Thr Tyr Ile Cys Asn Val Asn His Lys
        275                 280                 285

Pro Ser Asn Thr Lys Val Asp Lys Lys Val Glu Pro Lys Ser Cys Asp
    290                 295                 300

Lys Thr His Thr Cys Pro Pro Cys Pro Ala Pro Glu Leu Leu Gly Gly
305                 310                 315                 320

Pro Ser Val Phe Leu Phe Pro Pro Lys Pro Lys Asp Thr Leu Met Ile
                325                 330                 335

Ser Arg Thr Pro Glu Val Thr Cys Val Val Val Asp Val Ser His Glu
            340                 345                 350

Asp Pro Glu Val Lys Phe Asn Trp Tyr Val Asp Gly Val Glu Val His
        355                 360                 365

Asn Ala Lys Thr Lys Pro Arg Glu Glu Gln Tyr Asn Ser Thr Tyr Arg
    370                 375                 380

Val Val Ser Val Leu Thr Val Leu His Gln Asp Trp Leu Asn Gly Lys
385                 390                 395                 400

Glu Tyr Lys Cys Lys Val Ser Asn Lys Ala Leu Pro Ala Pro Ile Glu
                405                 410                 415

Lys Thr Ile Ser Lys Ala Lys Gly Gln Pro Arg Glu Pro Gln Val Tyr
            420                 425                 430

Thr Leu Pro Pro Ser Arg Glu Glu Met Thr Lys Asn Gln Val Ser Leu
        435                 440                 445

Thr Cys Leu Val Lys Gly Phe Tyr Pro Ser Asp Ile Ala Val Glu Trp

450                 455                 460

Glu Ser Asn Gly Gln Pro Glu Asn Asn Tyr Lys Thr Thr Pro Pro Val
465                 470                 475                 480

Leu Asp Ser Asp Gly Ser Phe Phe Leu Tyr Ser Lys Leu Thr Val Asp
                485                 490                 495

Lys Ser Arg Trp Gln Gln Gly Asn Val Phe Ser Cys Ser Val Met His
            500                 505                 510

Glu Ala Leu His Asn His Tyr Thr Gln Lys Ser Leu Ser Leu Ser Pro
        515                 520                 525

Gly Lys
    530

<210>  27
<211>  1032
<212>  DNA
<213>  artificial sequence

<400>  27
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg gacatccaga tgacccagag ccccagcagc     420

ctgagcgcca gcgtgggcga ccgcgtgacc atcacctgcc aggccagcca ggacatcagc     480

aactacctga actggtacca gcagaagccc ggcaaggccc ccaagctgct gatctacgac     540

gccagcaacc tggagaccgg cgtgcccagc cgcttcagcg gcagcggcag cggcaccgac     600

ttcaccttca ccatcagcag cctgcagccc gaggacatcg ccacctactt ctgccagcac     660

ttcgaccacc tgcccctggc cttcggcggc ggcaccaagg tggagatcaa gcgcacagtg     720

gcagccccca gcgtcttcat ttttccccct tccgatgaac agctgaagtc cggcactgct     780

tctgtggtct gtctgctgaa caatttctat cccagagagg ccaaggtgca gtggaaagtg     840

gacaacgctc tgcagtccgg caacagccag gagagtgtga ccgaacagga tagtaaggac     900

agcacatatt ctctgtctag taccctgaca ctgagtaagg cagattacga gaagcacaaa     960

gtgtatgcct gcgaagtcac tcatcaggga ctgtcaagcc ccgtgaccaa gagcttcaac    1020

cggggcgagt gt                                                          1032

<210>  28
<211>  344
<212>  PRT
<213>  artificial sequence

<400>  28

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Asp Ile Gln Met Thr Gln Ser Pro Ser Ser Leu Ser Ala Ser
    130                 135                 140

Val Gly Asp Arg Val Thr Ile Thr Cys Gln Ala Ser Gln Asp Ile Ser
145                 150                 155                 160

Asn Tyr Leu Asn Trp Tyr Gln Gln Lys Pro Gly Lys Ala Pro Lys Leu
                165                 170                 175

Leu Ile Tyr Asp Ala Ser Asn Leu Glu Thr Gly Val Pro Ser Arg Phe
            180                 185                 190

Ser Gly Ser Gly Ser Gly Thr Asp Phe Thr Phe Thr Ile Ser Ser Leu
        195                 200                 205

Gln Pro Glu Asp Ile Ala Thr Tyr Phe Cys Gln His Phe Asp His Leu

210                 215                 220

Pro Leu Ala Phe Gly Gly Gly Thr Lys Val Glu Ile Lys Arg Thr Val
225                 230                 235                 240

Ala Ala Pro Ser Val Phe Ile Phe Pro Pro Ser Asp Glu Gln Leu Lys
                245                 250                 255

Ser Gly Thr Ala Ser Val Val Cys Leu Leu Asn Asn Phe Tyr Pro Arg
            260                 265                 270

Glu Ala Lys Val Gln Trp Lys Val Asp Asn Ala Leu Gln Ser Gly Asn
        275                 280                 285

Ser Gln Glu Ser Val Thr Glu Gln Asp Ser Lys Asp Ser Thr Tyr Ser
    290                 295                 300

Leu Ser Ser Thr Leu Thr Leu Ser Lys Ala Asp Tyr Glu Lys His Lys
305                 310                 315                 320

Val Tyr Ala Cys Glu Val Thr His Gln Gly Leu Ser Ser Pro Val Thr
                325                 330                 335

Lys Ser Phe Asn Arg Gly Glu Cys
            340

<210>  29
<211>  1347
<212>  DNA
<213>  artificial sequence

<400>  29
caggtgcagc tgcaggagag cggccccggc ctggtgaagc ccagcgagac cctgagcctg      60

acctgcaccg tgagcggcgg cagcgtgagc agcggcgact actactggac ctggatccgc     120

cagagccccg gcaagggcct ggagtggatc ggccacatct actacagcgg caacaccaac     180

tacaacccca gcctgaagag ccgcctgacc atcagcatcg acaccagcaa gacccagttc     240

agcctgaagc tgagcagcgt gaccgccgcc gacaccgcca tctactactg cgtgcgcgac     300

cgcgtgaccg gcgccttcga catctggggc cagggcacca tggtgactgt gtctagcgcc     360

tccaccaagg gcccatcggt cttccccctg gcaccctcct ccaagagcac ctctgggggc     420

acagcggccc tgggctgcct ggtcaaggac tacttccccg aaccggtgac ggtgtcgtgg     480

aactcaggcg ccctgaccag cggcgtgcac accttcccgg ctgtcctaca gtcctcagga     540

ctctactccc tcagcagcgt ggtgactgtg ccctctagca gcttgggcac ccagacctac     600

atctgcaacg tgaatcacaa gcccagcaac accaaggtgg acaagaaagt tgaacccaaa     660

tcttgcgaca aaactcacac atgcccaccg tgcccagcac ctgaactcct ggggggaccg 720

tcagtcttcc tcttcccccc aaaacccaag gacaccctca tgatctcccg gacccctgag 780

gtcacatgcg tggtggtgga cgtgagccac gaagaccctg aggtcaagtt caactggtac 840

gtggacggcg tggaggtgca taatgccaag acaaagccgc gggaggagca gtacaacagc 900

acgtaccgtg tggtcagcgt cctcaccgtc ctgcaccagg actggctgaa tggcaaggag 960

tacaagtgca aggtctccaa caaagccctc ccagccccca tagagaaaac catctccaaa 1020

gccaaagggc agccccgaga accacaggtg tacaccctgc ccccatcccg ggaggagatg 1080

accaagaacc aggtcagcct gacctgcctg gtcaaaggct tctatcccag cgacatcgcc 1140

gtggagtggg agagcaatgg gcagccggag aacaactaca agaccacgcc tcccgtgctg 1200

gactccgacg gctccttctt cctctacagc aagctcaccg tggacaagag caggtggcag 1260

caggggaacg tcttctcatg ctccgtgatg catgaggctc tgcacaacca ctacacgcag 1320

aagagcctct ccctgtctcc gggtaaa 1347

<210> 30
<211> 449
<212> PRT
<213> artificial sequence

<400> 30

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Asp Tyr Tyr Trp Thr Trp Ile Arg Gln Ser Pro Gly Lys Gly Leu Glu
        35                  40                  45

Trp Ile Gly His Ile Tyr Tyr Ser Gly Asn Thr Asn Tyr Asn Pro Ser
    50                  55                  60

Leu Lys Ser Arg Leu Thr Ile Ser Ile Asp Thr Ser Lys Thr Gln Phe
65                  70                  75                  80

Ser Leu Lys Leu Ser Ser Val Thr Ala Ala Asp Thr Ala Ile Tyr Tyr
                85                  90                  95

Cys Val Arg Asp Arg Val Thr Gly Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  31
<211>  1344
<212>  DNA
<213>  artificial sequence

<400>  31
caggtgcagc tgcaggagag cggccccggc ctggtgaagc ccagcgagac cctgagcctg      60

acctgcaccg tgagcggcgg cagcgtgagc agcggcgact actactggac ctggatccgc     120

cagagccccg gcaagggcct ggagtggatc ggccacatct actacagcgg caacaccaac     180

tacaacccca gcctgaagag ccgcctgacc atcagcatcg acaccagcaa gacccagttc     240

agcctgaagc tgagcagcgt gaccgccgcc gacaccgcca tctactactg cgtgcgcgac     300

cgcgtgaccg gcgccttcga catctggggc cagggcacca tggtgactgt gtctagcgcc     360

tccaccaagg gcccatcggt cttccccctg gcaccctcct ccaagagcac ctctgggggc     420

acagcggccc tgggctgcct ggtcaaggac tacttccccg aaccggtgac ggtgtcgtgg     480

aactcaggcg ccctgaccag cggcgtgcac accttcccgg ctgtcctaca gtcctcagga     540

ctctactccc tcagcagcgt ggtgactgtg ccctctagca gcttgggcac ccagacctac     600

atctgcaacg tgaatcacaa gcccagcaac accaaggtgg acaagaaagt tgaacccaaa     660

tcttgcgaca aaactcacac atgcccaccg tgcccagcac ctccagtcgc cggaccgtca     720

gtcttcctct tccctccaaa acccaaggac accctcatga tctcccggac ccctgaggtc     780

acatgcgtgg tggtggacgt gagccacgaa gaccctgagg tcaagttcaa ctggtacgtg     840

gacggcgtgg aggtgcataa tgccaagaca aagccgcggg aggagcagta caacagcacg     900

taccgtgtgg tcagcgtcct caccgtcctg caccaggact ggctgaatgg caaggagtac     960

aagtgcaagg tctccaacaa aggcctccca agctccatcg agaaaaccat ctccaaagcc    1020

aaagggcagc cccgagaacc acaggtgtac accctgcctc catcccggga tgagctgacc    1080

aagaaccagg tcagcctgac ctgcctggtc aaaggcttct atcccagcga catcgccgtg 1140

gagtgggaga gcaatgggca gccggagaac aactacaaga ccacgcctcc cgtgctggac 1200

tccgacggct ccttcttcct ctacagcaag ctcaccgtgg acaagagcag gtggcagcag 1260

gggaacgtct tctcatgctc cgtgatgcat gaggctctgc acaaccacta cacgcagaag 1320

agcctctccc tgtctccggg taaa 1344

<210> 32
<211> 448
<212> PRT
<213> artificial sequence

<400> 32

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Asp Tyr Tyr Trp Thr Trp Ile Arg Gln Ser Pro Gly Lys Gly Leu Glu
        35                  40                  45

Trp Ile Gly His Ile Tyr Tyr Ser Gly Asn Thr Asn Tyr Asn Pro Ser
    50                  55                  60

Leu Lys Ser Arg Leu Thr Ile Ser Ile Asp Thr Ser Lys Thr Gln Phe
65                  70                  75                  80

Ser Leu Lys Leu Ser Ser Val Thr Ala Ala Asp Thr Ala Ile Tyr Tyr
                85                  90                  95

Cys Val Arg Asp Arg Val Thr Gly Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
        180                 185                 190

Ser Ser Leu Gly Thr Gln Thr Tyr Ile Cys Asn Val Asn His Lys Pro
    195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
        260                 265                 270

Glu Val Lys Phe Asn Trp Tyr Val Asp Gly Val Glu Val His Asn Ala
    275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
        340                 345                 350

Pro Pro Ser Arg Asp Glu Leu Thr Lys Asn Gln Val Ser Leu Thr Cys
    355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
        420                 425                 430

Leu His Asn His Tyr Thr Gln Lys Ser Leu Ser Leu Ser Pro Gly Lys
        435                 440                 445

<210>  33
<211>  642
<212>  DNA
<213>  artificial sequence

<400>  33
gacatccaga tgacccagag ccccagcagc ctgagcgcca gcgtgggcga ccgcgtgacc      60

atcacctgcc aggccagcca ggacatcagc aactacctga actggtacca gcagaagccc     120

ggcaaggccc ccaagctgct gatctacgac gccagcaacc tggagaccgg cgtgcccagc     180

cgcttcagcg gcagcggcag cggcaccgac ttcaccttca ccatcagcag cctgcagccc     240

gaggacatcg ccacctactt ctgccagcac ttcgaccacc tgcccctggc cttcggcggc     300

ggcaccaagg tggagatcaa gcgcacagtg gcagccccca gcgtcttcat ttttccccct      360

tccgatgaac agctgaagtc cggcactgct tctgtggtct gtctgctgaa caatttctat     420

cccagagagg ccaaggtgca gtggaaagtg gacaacgctc tgcagtccgg caacagccag     480

gagagtgtga ccgaacagga tagtaaggac agcacatatt ctctgtctag taccctgaca     540

ctgagtaagg cagattacga gaagcacaaa gtgtatgcct gcgaagtcac tcatcaggga     600

ctgtcaagcc ccgtgaccaa gagcttcaac cggggcgagt gt                        642

<210>  34
<211>  214
<212>  PRT
<213>  artificial sequence

<400>  34

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Phe Cys Gln His Phe Asp His Leu Pro Leu
                85                  90                  95

Ala Phe Gly Gly Gly Thr Lys Val Glu Ile Lys Arg Thr Val Ala Ala
                100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
                180                 185                 190

Ala Cys Glu Val Thr His Gln Gly Leu Ser Ser Pro Val Thr Lys Ser
        195                 200                 205

Phe Asn Arg Gly Glu Cys
    210

<210>  35
<211>  1341
<212>  DNA
<213>  artificial sequence

<400>  35
gaggtgcagc tggtggagag cggaggtgga ctagtacagc ctggtggcag cctacgactg      60

agttgcgccg ccagcggctt caccttcagc gacagctgga tccactgggt gcgccaggcc     120

cccggcaagg gcctggagtg ggtggcctgg atcagcccct acggcggcag cacctactac     180

gccgacagcg tgaagggccg cttcaccatc agcgccgaca ccagcaagaa caccgcctac     240

ctgcagatga acagcctgcg cgccgaggac accgccgtgt actactgcgc ccgccgccac     300

tggcccggcg gcttcgacta ctggggccag ggcaccctgg tgaccgtgag cagcgcctcc     360

accaagggcc catcggtctt ccccctggca ccctcctcca agagcacctc tgggggcaca     420

gcggccctgg gctgcctggt caaggactac ttccccgaac cggtgacggt gtcgtggaac     480

tcaggcgccc tgaccagcgg cgtgcacacc ttcccggctg tcctacagtc ctcaggactc     540

tactccctca gcagcgtggt gactgtgccc tctagcagct tgggcaccca gacctacatc     600

tgcaacgtga atcacaagcc cagcaacacc aaggtggaca agaaagttga acccaaatct     660

tgcgacaaaa ctcacacatg cccaccgtgc ccagcacctc cagtcgccgg accgtcagtc     720

ttcctcttcc ctccaaaacc caaggacacc ctcatgatct cccggacccc tgaggtcaca 780

tgcgtggtgg tggacgtgag ccacgaagac cctgaggtca agttcaactg gtacgtggac 840

ggcgtggagg tgcataatgc caagacaaag ccgcgggagg agcagtacaa cagcacgtac 900

cgtgtggtca gcgtcctcac cgtcctgcac caggactggc tgaatggcaa ggagtacaag 960

tgcaaggtct ccaacaaagg cctcccaagc tccatcgaga aaaccatctc caaagccaaa 1020

gggcagcccc gagaaccaca ggtgtacacc ctgcctccat cccgggatga gctgaccaag 1080

aaccaggtca gcctgacctg cctggtcaaa ggcttctatc ccagcgacat cgccgtggag 1140

tgggagagca atgggcagcc ggagaacaac tacaagacca cgcctcccgt gctggactcc 1200

gacggctcct tcttcctcta cagcaagctc accgtggaca agagcaggtg gcagcagggg 1260

aacgtcttct catgctccgt gatgcatgag gctctgcaca accactacac gcagaagagc 1320

ctctccctgt ctccgggtaa a 1341

<210> 36
<211> 447
<212> PRT
<213> artificial sequence

<400> 36

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Ser
            20                  25                  30

Trp Ile His Trp Val Arg Gln Ala Pro Gly Lys Gly Leu Glu Trp Val
        35                  40                  45

Ala Trp Ile Ser Pro Tyr Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210>  37
<211>  642
<212>  DNA
<213>  artificial sequence

<400>  37
gacatccaga tgacccagag ccccagcagc ctgagcgcca gcgtgggcga ccgcgtgacc      60

atcacctgcc gcgccagcca ggacgtgagc accgccgtgg cctggtacca gcagaagccc     120

ggcaaggccc ccaagctgct gatctacagc gccagcttcc tgtacagcgg cgtgcccagc     180

cgcttcagcg gcagcggcag cggcaccgac ttcaccctga ccatcagcag cctgcagccc     240

gaggacttcg ccacctacta ctgccagcag tacctgtacc accccgccac cttcggccag     300

ggcaccaagg tggagatcaa gcgcacagtg gcagccccca gcgtcttcat tttcccccct      360

tccgatgaac agctgaagtc cggcactgct tctgtggtct gtctgctgaa caatttctat     420

cccagagagg ccaaggtgca gtggaaagtg gacaacgctc tgcagtccgg caacagccag     480

gagagtgtga ccgaacagga tagtaaggac agcacatatt ctctgtctag taccctgaca     540

ctgagtaagg cagattacga gaagcacaaa gtgtatgcct gcgaagtcac tcatcaggga     600

ctgtcaagcc ccgtgaccaa gagcttcaac cggggcgagt gt                        642

<210>  38
<211>  214
<212>  PRT
<213>  artificial sequence

<400>  38

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Leu Tyr His Pro Ala
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  39
<211>  1734
<212>  DNA
<213>  artificial sequence

<400>  39
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg caggtgcagc tgcaggagag cggccccggc 420

ctggtgaagc ccagcgagac cctgagcctg acctgcaccg tgagcggcgg cagcgtgagc 480

agcggcgact actactggac ctggatccgc cagagccccg gcaagggcct ggagtggatc 540

ggccacatct actacagcgg caacaccaac tacaacccca gcctgaagag ccgcctgacc 600

atcagcatcg acaccagcaa gacccagttc agcctgaagc tgagcagcgt gaccgccgcc 660

gacaccgcca tctactactg cgtgcgcgac cgcgtgaccg gcgccttcga catctggggc 720

cagggcacca tggtgactgt gtctagcgcc tccaccaagg gcccatcggt cttccccctg 780

gcaccctcct ccaagagcac ctctgggggc acagcggccc tgggctgcct ggtcaaggac 840

tacttccccg aaccggtgac ggtgtcgtgg aactcaggcg ccctgaccag cggcgtgcac 900

accttcccgg ctgtcctaca gtcctcagga ctctactccc tcagcagcgt ggtgactgtg 960

ccctctagca gcttgggcac ccagacctac atctgcaacg tgaatcacaa gcccagcaac 1020

accaaggtgg acaagaaagt tgaacccaaa tcttgcgaca aaactcacac atgcccaccg 1080

tgcccagcac ctccagtcgc cggaccgtca gtcttcctct tccctccaaa acccaaggac 1140

accctcatga tctcccggac ccctgaggtc acatgcgtgg tggtggacgt gagccacgaa 1200

gaccctgagg tcaagttcaa ctggtacgtg gacggcgtgg aggtgcataa tgccaagaca 1260

aagccgcggg aggagcagta caacagcacg taccgtgtgg tcagcgtcct caccgtcctg 1320

caccaggact ggctgaatgg caaggagtac aagtgcaagg tctccaacaa aggcctccca 1380

agctccatcg agaaaaccat ctccaaagcc aaagggcagc cccgagaacc acaggtgtac 1440

accctgcctc catcccggga tgagctgacc aagaaccagg tcagcctgac ctgcctggtc 1500

aaaggcttct atcccagcga catcgccgtg gagtgggaga gcaatgggca gccggagaac 1560

aactacaaga ccacgcctcc cgtgctggac tccgacggct ccttcttcct ctacagcaag 1620

ctcaccgtgg acaagagcag gtggcagcag gggaacgtct tctcatgctc cgtgatgcat 1680

gaggctctgc acaaccacta cacgcagaag agcctctccc tgtctccggg taaa 1734

<210> 40
<211> 578
<212> PRT
<213> artificial sequence

<400> 40

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Gln Val Gln Leu Gln Glu Ser Gly Pro Gly Leu Val Lys Pro
    130                 135                 140

Ser Glu Thr Leu Ser Leu Thr Cys Thr Val Ser Gly Gly Ser Val Ser
145                 150                 155                 160

Ser Gly Asp Tyr Tyr Trp Thr Trp Ile Arg Gln Ser Pro Gly Lys Gly
                165                 170                 175

Leu Glu Trp Ile Gly His Ile Tyr Tyr Ser Gly Asn Thr Asn Tyr Asn
            180                 185                 190

Pro Ser Leu Lys Ser Arg Leu Thr Ile Ser Ile Asp Thr Ser Lys Thr
        195                 200                 205

Gln Phe Ser Leu Lys Leu Ser Ser Val Thr Ala Ala Asp Thr Ala Ile
    210                 215                 220

Tyr Tyr Cys Val Arg Asp Arg Val Thr Gly Ala Phe Asp Ile Trp Gly
225                 230                 235                 240

Gln Gly Thr Met Val Thr Val Ser Ser Ala Ser Thr Lys Gly Pro Ser
                245                 250                 255

Val Phe Pro Leu Ala Pro Ser Ser Lys Ser Thr Ser Gly Gly Thr Ala
            260                 265                 270

Ala Leu Gly Cys Leu Val Lys Asp Tyr Phe Pro Glu Pro Val Thr Val
        275                 280                 285

Ser Trp Asn Ser Gly Ala Leu Thr Ser Gly Val His Thr Phe Pro Ala
    290                 295                 300

Val Leu Gln Ser Ser Gly Leu Tyr Ser Leu Ser Ser Val Val Thr Val
305                 310                 315                 320

Pro Ser Ser Ser Leu Gly Thr Gln Thr Tyr Ile Cys Asn Val Asn His
                325                 330                 335

Lys Pro Ser Asn Thr Lys Val Asp Lys Lys Val Glu Pro Lys Ser Cys
            340                 345                 350

Asp Lys Thr His Thr Cys Pro Pro Cys Pro Ala Pro Pro Val Ala Gly
        355                 360                 365

Pro Ser Val Phe Leu Phe Pro Pro Lys Pro Lys Asp Thr Leu Met Ile
    370                 375                 380

Ser Arg Thr Pro Glu Val Thr Cys Val Val Val Asp Val Ser His Glu
385                 390                 395                 400

Asp Pro Glu Val Lys Phe Asn Trp Tyr Val Asp Gly Val Glu Val His
                405                 410                 415

Asn Ala Lys Thr Lys Pro Arg Glu Glu Gln Tyr Asn Ser Thr Tyr Arg
            420                 425                 430

Val Val Ser Val Leu Thr Val Leu His Gln Asp Trp Leu Asn Gly Lys
        435                 440                 445

Glu Tyr Lys Cys Lys Val Ser Asn Lys Gly Leu Pro Ser Ser Ile Glu
    450                 455                 460

Lys Thr Ile Ser Lys Ala Lys Gly Gln Pro Arg Glu Pro Gln Val Tyr
465                 470                 475                 480

Thr Leu Pro Pro Ser Arg Asp Glu Leu Thr Lys Asn Gln Val Ser Leu
                485                 490                 495

Thr Cys Leu Val Lys Gly Phe Tyr Pro Ser Asp Ile Ala Val Glu Trp
            500                 505                 510

Glu Ser Asn Gly Gln Pro Glu Asn Asn Tyr Lys Thr Thr Pro Pro Val
        515                 520                 525

Leu Asp Ser Asp Gly Ser Phe Phe Leu Tyr Ser Lys Leu Thr Val Asp
    530                 535                 540

Lys Ser Arg Trp Gln Gln Gly Asn Val Phe Ser Cys Ser Val Met His
545                 550                 555                 560

Glu Ala Leu His Asn His Tyr Thr Gln Lys Ser Leu Ser Leu Ser Pro
                565                 570                 575

Gly Lys

<210>  41
<211>  1569
<212>  DNA
<213>  artificial sequence

<400>  41
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

gggcaggtgc agctggtgga gagtggaggt ggcgtggtac agcccggccg cagcctgcgc     300

ctggactgca aggccagcgg catcaccttc agcaacagcg gcatgcactg ggtgcgccag     360

gcccccggca agggcctgga gtgggtggcc gtgatctggt acgacggcag caagcgctac     420

tacgccgaca gcgtgaaggg ccgcttcacc atcagccgcg acaacagcaa gaacaccctg     480

ttcctgcaga tgaacagcct gcgcgccgag gacaccgccg tgtactactg cgccaccaac     540

gacgactact ggggccaggg caccctggtg accgtgagca gcgcctccac caagggccca     600

tcggtcttcc ccctggcacc ctcctccaag agcacctctg ggggcacagc ggccctgggc     660

tgcctggtca aggactactt ccccgaaccg gtgacggtgt cgtggaactc aggcgccctg     720

accagcggcg tgcacacctt cccggctgtc ctacagtcct caggactcta ctccctcagc     780

agcgtggtga ctgtgccctc tagcagcttg ggcacccaga cctacatctg caacgtgaat     840

cacaagccca gcaacaccaa ggtggacaag aaagttgaac ccaaatcttg cgacaaaact     900

cacacatgcc caccgtgccc agcacctcca gtcgccggac cgtcagtctt cctcttccct     960

ccaaaaccca aggacaccct catgatctcc cggacccctg aggtcacatg cgtggtggtg    1020

gacgtgagcc acgaagaccc tgaggtcaag ttcaactggt acgtggacgg cgtggaggtg    1080

cataatgcca agacaaagcc gcgggaggag cagtacaaca gcacgtaccg tgtggtcagc    1140

gtcctcaccg tcctgcacca ggactggctg aatggcaagg agtacaagtg caaggtctcc    1200

aacaaaggcc tcccaagctc catcgagaaa accatctcca aagccaaagg gcagccccga    1260

gaaccacagg tgtacaccct gcctccatcc cgggatgagc tgaccaagaa ccaggtcagc    1320

ctgacctgcc tggtcaaagg cttctatccc agcgacatcg ccgtggagtg ggagagcaat 1380

gggcagccgg agaacaacta caagaccacg cctcccgtgc tggactccga cggctccttc 1440

ttcctctaca gcaagctcac cgtggacaag agcaggtggc agcaggggaa cgtcttctca 1500

tgctccgtga tgcatgaggc tctgcacaac cactacacgc agaagagcct ctccctgtct 1560

ccgggtaaa 1569

<210> 42
<211> 523
<212> PRT
<213> artificial sequence

<400> 42

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Gln Val Gln Leu Val Glu Ser Gly Gly Gly Val Val Gln Pro Gly
                85                  90                  95

Arg Ser Leu Arg Leu Asp Cys Lys Ala Ser Gly Ile Thr Phe Ser Asn
            100                 105                 110

Ser Gly Met His Trp Val Arg Gln Ala Pro Gly Lys Gly Leu Glu Trp
        115                 120                 125

Val Ala Val Ile Trp Tyr Asp Gly Ser Lys Arg Tyr Tyr Ala Asp Ser
    130                 135                 140

Val Lys Gly Arg Phe Thr Ile Ser Arg Asp Asn Ser Lys Asn Thr Leu
145                 150                 155                 160

Phe Leu Gln Met Asn Ser Leu Arg Ala Glu Asp Thr Ala Val Tyr Tyr
                165                 170                 175

Cys Ala Thr Asn Asp Asp Tyr Trp Gly Gln Gly Thr Leu Val Thr Val
            180                 185                 190

Ser Ser Ala Ser Thr Lys Gly Pro Ser Val Phe Pro Leu Ala Pro Ser
        195                 200                 205

Ser Lys Ser Thr Ser Gly Gly Thr Ala Ala Leu Gly Cys Leu Val Lys
    210                 215                 220

Asp Tyr Phe Pro Glu Pro Val Thr Val Ser Trp Asn Ser Gly Ala Leu
225                 230                 235                 240

Thr Ser Gly Val His Thr Phe Pro Ala Val Leu Gln Ser Ser Gly Leu
                245                 250                 255

Tyr Ser Leu Ser Ser Val Val Thr Val Pro Ser Ser Ser Leu Gly Thr
            260                 265                 270

Gln Thr Tyr Ile Cys Asn Val Asn His Lys Pro Ser Asn Thr Lys Val
        275                 280                 285

Asp Lys Lys Val Glu Pro Lys Ser Cys Asp Lys Thr His Thr Cys Pro
    290                 295                 300

Pro Cys Pro Ala Pro Pro Val Ala Gly Pro Ser Val Phe Leu Phe Pro
305                 310                 315                 320

Pro Lys Pro Lys Asp Thr Leu Met Ile Ser Arg Thr Pro Glu Val Thr
                325                 330                 335

Cys Val Val Val Asp Val Ser His Glu Asp Pro Glu Val Lys Phe Asn
            340                 345                 350

Trp Tyr Val Asp Gly Val Glu Val His Asn Ala Lys Thr Lys Pro Arg
        355                 360                 365

Glu Glu Gln Tyr Asn Ser Thr Tyr Arg Val Val Ser Val Leu Thr Val
    370                 375                 380

Leu His Gln Asp Trp Leu Asn Gly Lys Glu Tyr Lys Cys Lys Val Ser
385                 390                 395                 400

Asn Lys Gly Leu Pro Ser Ser Ile Glu Lys Thr Ile Ser Lys Ala Lys
                405                 410                 415

Gly Gln Pro Arg Glu Pro Gln Val Tyr Thr Leu Pro Pro Ser Arg Asp
            420                 425                 430

Glu Leu Thr Lys Asn Gln Val Ser Leu Thr Cys Leu Val Lys Gly Phe
        435                 440                 445

Tyr Pro Ser Asp Ile Ala Val Glu Trp Glu Ser Asn Gly Gln Pro Glu
    450                 455                 460

Asn Asn Tyr Lys Thr Thr Pro Pro Val Leu Asp Ser Asp Gly Ser Phe
465                 470                 475                 480

Phe Leu Tyr Ser Lys Leu Thr Val Asp Lys Ser Arg Trp Gln Gln Gly
                485                 490                 495

Asn Val Phe Ser Cys Ser Val Met His Glu Ala Leu His Asn His Tyr
            500                 505                 510

Thr Gln Lys Ser Leu Ser Leu Ser Pro Gly Lys
        515                 520

<210>  43
<211>  1587
<212>  DNA
<213>  artificial sequence

<400>  43
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

gggcaggtgc agctgcagga gagcggcccc ggcctggtga agcccagcga gaccctgagc     300

ctgacctgca ccgtgagcgg cggcagcgtg agcagcggcg actactactg gacctggatc     360

cgccagagcc ccggcaaggg cctggagtgg atcggccaca tctactacag cggcaacacc     420

aactacaacc ccagcctgaa gagccgcctg accatcagca tcgacaccag caagacccag     480

ttcagcctga agctgagcag cgtgaccgcc gccgacaccg ccatctacta ctgcgtgcgc     540

gaccgcgtga ccggcgcctt cgacatctgg ggccagggca ccatggtgac tgtgtctagc     600

gcctccacca agggcccatc ggtcttcccc ctggcaccct cctccaagag cacctctggg     660

ggcacagcgg ccctgggctg cctggtcaag gactacttcc ccgaaccggt gacggtgtcg     720

tggaactcag gcgccctgac cagcggcgtg cacaccttcc cggctgtcct acagtcctca     780

ggactctact ccctcagcag cgtggtgact gtgccctcta gcagcttggg cacccagacc     840

tacatctgca acgtgaatca caagcccagc aacaccaagg tggacaagaa agttgaaccc     900

aaatcttgcg acaaaactca cacatgccca ccgtgcccag cacctccagt cgccggaccg     960

tcagtcttcc tcttccctcc aaaacccaag gacaccctca tgatctcccg gacccctgag 1020

gtcacatgcg tggtggtgga cgtgagccac gaagaccctg aggtcaagtt caactggtac 1080

gtggacggcg tggaggtgca taatgccaag acaaagccgc gggaggagca gtacaacagc 1140

acgtaccgtg tggtcagcgt cctcaccgtc ctgcaccagg actggctgaa tggcaaggag 1200

tacaagtgca aggtctccaa caaaggcctc ccaagctcca tcgagaaaac catctccaaa 1260

gccaaagggc agccccgaga accacaggtg tacaccctgc ctccatcccg ggatgagctg 1320

accaagaacc aggtcagcct gacctgcctg gtcaaaggct tctatcccag cgacatcgcc 1380

gtggagtggg agagcaatgg gcagccggag aacaactaca agaccacgcc tcccgtgctg 1440

gactccgacg gctccttctt cctctacagc aagctcaccg tggacaagag caggtggcag 1500

caggggaacg tcttctcatg ctccgtgatg catgaggctc tgcacaacca ctacacgcag 1560

aagagcctct ccctgtctcc gggtaaa 1587

<210> 44
<211> 529
<212> PRT
<213> artificial sequence

<400> 44

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Gln Val Gln Leu Gln Glu Ser Gly Pro Gly Leu Val Lys Pro Ser
                85                  90                  95

Glu Thr Leu Ser Leu Thr Cys Thr Val Ser Gly Gly Ser Val Ser Ser
            100                 105                 110

Gly Asp Tyr Tyr Trp Thr Trp Ile Arg Gln Ser Pro Gly Lys Gly Leu
        115                 120                 125

Glu Trp Ile Gly His Ile Tyr Tyr Ser Gly Asn Thr Asn Tyr Asn Pro

130                 135                 140

Ser Leu Lys Ser Arg Leu Thr Ile Ser Ile Asp Thr Ser Lys Thr Gln
145                 150                 155                 160

Phe Ser Leu Lys Leu Ser Ser Val Thr Ala Ala Asp Thr Ala Ile Tyr
                165                 170                 175

Tyr Cys Val Arg Asp Arg Val Thr Gly Ala Phe Asp Ile Trp Gly Gln
            180                 185                 190

Gly Thr Met Val Thr Val Ser Ser Ala Ser Thr Lys Gly Pro Ser Val
        195                 200                 205

Phe Pro Leu Ala Pro Ser Ser Lys Ser Thr Ser Gly Gly Thr Ala Ala
    210                 215                 220

Leu Gly Cys Leu Val Lys Asp Tyr Phe Pro Glu Pro Val Thr Val Ser
225                 230                 235                 240

Trp Asn Ser Gly Ala Leu Thr Ser Gly Val His Thr Phe Pro Ala Val
                245                 250                 255

Leu Gln Ser Ser Gly Leu Tyr Ser Leu Ser Ser Val Val Thr Val Pro
            260                 265                 270

Ser Ser Ser Leu Gly Thr Gln Thr Tyr Ile Cys Asn Val Asn His Lys
        275                 280                 285

Pro Ser Asn Thr Lys Val Asp Lys Lys Val Glu Pro Lys Ser Cys Asp
    290                 295                 300

Lys Thr His Thr Cys Pro Pro Cys Pro Ala Pro Pro Val Ala Gly Pro
305                 310                 315                 320

Ser Val Phe Leu Phe Pro Pro Lys Pro Lys Asp Thr Leu Met Ile Ser
                325                 330                 335

Arg Thr Pro Glu Val Thr Cys Val Val Val Asp Val Ser His Glu Asp
            340                 345                 350

Pro Glu Val Lys Phe Asn Trp Tyr Val Asp Gly Val Glu Val His Asn
        355                 360                 365

Ala Lys Thr Lys Pro Arg Glu Glu Gln Tyr Asn Ser Thr Tyr Arg Val
    370                 375                 380

Val Ser Val Leu Thr Val Leu His Gln Asp Trp Leu Asn Gly Lys Glu
385                 390                 395                 400

Tyr Lys Cys Lys Val Ser Asn Lys Gly Leu Pro Ser Ser Ile Glu Lys
                405                 410                 415

Thr Ile Ser Lys Ala Lys Gly Gln Pro Arg Glu Pro Gln Val Tyr Thr
            420                 425                 430

Leu Pro Pro Ser Arg Asp Glu Leu Thr Lys Asn Gln Val Ser Leu Thr
        435                 440                 445

Cys Leu Val Lys Gly Phe Tyr Pro Ser Asp Ile Ala Val Glu Trp Glu
    450                 455                 460

Ser Asn Gly Gln Pro Glu Asn Asn Tyr Lys Thr Thr Pro Pro Val Leu
465                 470                 475                 480

Asp Ser Asp Gly Ser Phe Phe Leu Tyr Ser Lys Leu Thr Val Asp Lys
                485                 490                 495

Ser Arg Trp Gln Gln Gly Asn Val Phe Ser Cys Ser Val Met His Glu
            500                 505                 510

Ala Leu His Asn His Tyr Thr Gln Lys Ser Leu Ser Leu Ser Pro Gly
        515                 520                 525

Lys

<210>  45
<211>  1731
<212>  DNA
<213>  artificial sequence

<400>  45
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg gaggtgcagc tggtggagag cggaggtgga     420

ctagtacagc ctggtggcag cctacgactg agttgcgccg ccagcggctt caccttcagc     480

gacagctgga tccactgggt gcgccaggcc cccggcaagg gcctggagtg ggtggcctgg     540

atcagcccct acggcggcag cacctactac gccgacagcg tgaagggccg cttcaccatc 600

agcgccgaca ccagcaagaa caccgcctac ctgcagatga acagcctgcg cgccgaggac 660

accgccgtgt actactgcgc ccgccgccac tggcccggcg gcttcgacta ctggggccag 720

ggcaccctgg tgaccgtgag cagcgcctcc accaagggcc catcggtctt ccccctggca 780

ccctcctcca agagcacctc tgggggcaca gcggccctgg gctgcctggt caaggactac 840

ttccccgaac cggtgacggt gtcgtggaac tcaggcgccc tgaccagcgg cgtgcacacc 900

ttcccggctg tcctacagtc ctcaggactc tactccctca gcagcgtggt gactgtgccc 960

tctagcagct tgggcaccca gacctacatc tgcaacgtga atcacaagcc cagcaacacc 1020

aaggtggaca agaaagttga acccaaatct tgcgacaaaa ctcacacatg cccaccgtgc 1080

ccagcacctc cagtcgccgg accgtcagtc ttcctcttcc ctccaaaacc caaggacacc 1140

ctcatgatct cccggacccc tgaggtcaca tgcgtggtgg tggacgtgag ccacgaagac 1200

cctgaggtca agttcaactg gtacgtggac ggcgtggagg tgcataatgc caagacaaag 1260

ccgcgggagg agcagtacaa cagcacgtac cgtgtggtca gcgtcctcac cgtcctgcac 1320

caggactggc tgaatggcaa ggagtacaag tgcaaggtct ccaacaaagg cctcccaagc 1380

tccatcgaga aaaccatctc caaagccaaa gggcagcccc gagaaccaca ggtgtacacc 1440

ctgcctccat cccgggatga gctgaccaag aaccaggtca gcctgacctg cctggtcaaa 1500

ggcttctatc ccagcgacat cgccgtggag tgggagagca atgggcagcc ggagaacaac 1560

tacaagacca cgcctcccgt gctggactcc gacggctcct tcttcctcta cagcaagctc 1620

accgtggaca agagcaggtg gcagcagggg aacgtcttct catgctccgt gatgcatgag 1680

gctctgcaca accactacac gcagaagagc ctctccctgt ctccgggtaa a 1731

<210> 46
<211> 577
<212> PRT
<213> artificial sequence

<400> 46
   Asn Trp Val Asn Val Ile Ser Asp Leu Lys Lys Ile Glu Asp Leu Ile
   1 5 10 15
   Gln Ser Met His Ile Asp Ala Thr Leu Tyr Thr Glu Ser Asp Val His
   20 25 30
   Pro Ser Cys Lys Val Thr Ala Met Lys Cys Phe Leu Leu Glu Leu Gln
   35 40 45
   Val Ile Ser Leu Glu Ser Gly Asp Ala Ser Ile His Asp Thr Val Glu
   50 55 60
   Asn Leu Ile Ile Leu Ala Asn Asp Ser Leu Ser Ser Asn Gly Asn Val
   65                  70                  75                  80
   Thr Glu Ser Gly Cys Lys Glu Cys Glu Glu Leu Glu Glu Lys Asn Ile
                   85                  90                  95
   Lys Glu Phe Leu Gln Ser Phe Val His Ile Val Gln Met Phe Ile Asn
               100                 105                 110
   Thr Ser Gly Gly Gly Gly Ser Gly Gly Gly Gly Ser Gly Gly Gly Gly
           115                 120                 125
   Ser Gly Glu Val Gln Leu Val Glu Ser Gly Gly Gly Leu Val Gln Pro
       130                 135                 140
   Gly Gly Ser Leu Arg Leu Ser Cys Ala Ala Ser Gly Phe Thr Phe Ser
   145                 150                 155                 160
   Asp Ser Trp Ile His Trp Val Arg Gln Ala Pro Gly Lys Gly Leu Glu
                   165                 170                 175
   Trp Val Ala Trp Ile Ser Pro Tyr Gly Gly Ser Thr Tyr Tyr Ala Asp
               180                 185                 190
   Ser Val Lys Gly Arg Phe Thr Ile Ser Ala Asp Thr Ser Lys Asn Thr
           195                 200                 205
   Ala Tyr Leu Gln Met Asn Ser Leu Arg Ala Glu Asp Thr Ala Val Tyr
       210                 215                 220
   Tyr Cys Ala Arg Arg His Trp Pro Gly Gly Phe Asp Tyr Trp Gly Gln
   225                 230                 235                 240
   Gly Thr Leu Val Thr Val Ser Ser Ala Ser Thr Lys Gly Pro Ser Val
                   245                 250                 255
   Phe Pro Leu Ala Pro Ser Ser Lys Ser Thr Ser Gly Gly Thr Ala Ala
               260                 265                 270
   Leu Gly Cys Leu Val Lys Asp Tyr Phe Pro Glu Pro Val Thr Val Ser
           275                 280                 285
   Trp Asn Ser Gly Ala Leu Thr Ser Gly Val His Thr Phe Pro Ala Val
       290                 295                 300
   Leu Gln Ser Ser Gly Leu Tyr Ser Leu Ser Ser Val Val Thr Val Pro
   305                 310                 315                 320
   Ser Ser Ser Leu Gly Thr Gln Thr Tyr Ile Cys Asn Val Asn His Lys
                   325                 330                 335
   Pro Ser Asn Thr Lys Val Asp Lys Lys Val Glu Pro Lys Ser Cys Asp
               340                 345                 350
   Lys Thr His Thr Cys Pro Pro Cys Pro Ala Pro Pro Val Ala Gly Pro
           355                 360                 365
   Ser Val Phe Leu Phe Pro Pro Lys Pro Lys Asp Thr Leu Met Ile Ser
       370                 375                 380
   Arg Thr Pro Glu Val Thr Cys Val Val Val Asp Val Ser His Glu Asp
   385                 390                 395                 400
   Pro Glu Val Lys Phe Asn Trp Tyr Val Asp Gly Val Glu Val His Asn
                   405                 410                 415
   Ala Lys Thr Lys Pro Arg Glu Glu Gln Tyr Asn Ser Thr Tyr Arg Val
               420                 425                 430
   Val Ser Val Leu Thr Val Leu His Gln Asp Trp Leu Asn Gly Lys Glu
           435                 440                 445
   Tyr Lys Cys Lys Val Ser Asn Lys Gly Leu Pro Ser Ser Ile Glu Lys
       450                 455                 460
   Thr Ile Ser Lys Ala Lys Gly Gln Pro Arg Glu Pro Gln Val Tyr Thr
   465                 470                 475                 480
   Leu Pro Pro Ser Arg Asp Glu Leu Thr Lys Asn Gln Val Ser Leu Thr
                   485                 490                 495
   Cys Leu Val Lys Gly Phe Tyr Pro Ser Asp Ile Ala Val Glu Trp Glu
               500                 505                 510
   Ser Asn Gly Gln Pro Glu Asn Asn Tyr Lys Thr Thr Pro Pro Val Leu
           515                 520                 525
   Asp Ser Asp Gly Ser Phe Phe Leu Tyr Ser Lys Leu Thr Val Asp Lys
       530                 535                 540
   Ser Arg Trp Gln Gln Gly Asn Val Phe Ser Cys Ser Val Met His Glu
   545                 550                 555                 560
   Ala Leu His Asn His Tyr Thr Gln Lys Ser Leu Ser Leu Ser Pro Gly
                   565                 570                 575
   Lys

<210>  47
<211>  885
<212>  DNA
<213>  artificial sequence

<400>  47
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

ggggacatcc agatgaccca gagccccagc agcctgagcg ccagcgtggg cgaccgcgtg     300

accatcacct gccgcgccag ccaggacgtg agcaccgccg tggcctggta ccagcagaag     360

cccggcaagg cccccaagct gctgatctac agcgccagct tcctgtacag cggcgtgccc     420

agccgcttca gcggcagcgg cagcggcacc gacttcaccc tgaccatcag cagcctgcag     480

cccgaggact tcgccaccta ctactgccag cagtacctgt accaccccgc caccttcggc     540

cagggcacca aggtggagat caagcgcaca gtggcagccc ccagcgtctt catttttccc     600

ccttccgatg aacagctgaa gtccggcact gcttctgtgg tctgtctgct gaacaatttc     660

tatcccagag aggccaaggt gcagtggaaa gtggacaacg ctctgcagtc cggcaacagc     720

caggagagtg tgaccgaaca ggatagtaag gacagcacat attctctgtc tagtaccctg     780

acactgagta aggcagatta cgagaagcac aaagtgtatg cctgcgaagt cactcatcag     840

ggactgtcaa gccccgtgac caagagcttc aaccggggcg agtgt                     885

<210>  48
<211>  295
<212>  PRT
<213>  artificial sequence

<400>  48

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Asp Ile Gln Met Thr Gln Ser Pro Ser Ser Leu Ser Ala Ser Val
                85                  90                  95

Gly Asp Arg Val Thr Ile Thr Cys Arg Ala Ser Gln Asp Val Ser Thr
            100                 105                 110

Ala Val Ala Trp Tyr Gln Gln Lys Pro Gly Lys Ala Pro Lys Leu Leu
        115                 120                 125

Ile Tyr Ser Ala Ser Phe Leu Tyr Ser Gly Val Pro Ser Arg Phe Ser
    130                 135                 140

Gly Ser Gly Ser Gly Thr Asp Phe Thr Leu Thr Ile Ser Ser Leu Gln
145                 150                 155                 160

Pro Glu Asp Phe Ala Thr Tyr Tyr Cys Gln Gln Tyr Leu Tyr His Pro
                165                 170                 175

Ala Thr Phe Gly Gln Gly Thr Lys Val Glu Ile Lys Arg Thr Val Ala
            180                 185                 190

Ala Pro Ser Val Phe Ile Phe Pro Pro Ser Asp Glu Gln Leu Lys Ser
        195                 200                 205

Gly Thr Ala Ser Val Val Cys Leu Leu Asn Asn Phe Tyr Pro Arg Glu
    210                 215                 220

Ala Lys Val Gln Trp Lys Val Asp Asn Ala Leu Gln Ser Gly Asn Ser
225                 230                 235                 240

Gln Glu Ser Val Thr Glu Gln Asp Ser Lys Asp Ser Thr Tyr Ser Leu
                245                 250                 255

Ser Ser Thr Leu Thr Leu Ser Lys Ala Asp Tyr Glu Lys His Lys Val
            260                 265                 270

Tyr Ala Cys Glu Val Thr His Gln Gly Leu Ser Ser Pro Val Thr Lys
        275                 280                 285

Ser Phe Asn Arg Gly Glu Cys
    290                 295

<210>  49
<211>  1584
<212>  DNA
<213>  artificial sequence

<400>  49
atcacctgcc cacctcccat gagcgtggag cacgccgaca tctgggtgaa gagctacagc        60

ctgtacagcc gcgagcgcta catctgcaac agcggcttca agcgcaaggc cggcaccagc       120

agcctgaccg agtgcgtgct gaacaaggcc accaacgtgg cccactggac cacccccagc       180

ctgaagtgca tccgcggcgg aggcggatcc ggaggcggag gttccggcgg gggtgggagc       240

ggggaggtgc agctggtgga gagcggaggt ggactagtac agcctggtgg cagcctacga       300

ctgagttgcg ccgccagcgg cttcaccttc agcgacagct ggatccactg ggtgcgccag       360

gcccccggca agggcctgga gtgggtggcc tggatcagcc cctacggcgg cagcacctac       420

tacgccgaca gcgtgaaggg ccgcttcacc atcagcgccg acaccagcaa gaacaccgcc       480

tacctgcaga tgaacagcct gcgcgccgag gacaccgccg tgtactactg cgcccgccgc       540

cactggcccg gcggcttcga ctactggggc cagggcaccc tggtgaccgt gagcagcgcc       600

tccaccaagg gcccatcggt cttccccctg gcaccctcct ccaagagcac ctctgggggc       660

acagcggccc tgggctgcct ggtcaaggac tacttccccg aaccggtgac ggtgtcgtgg       720

aactcaggcg ccctgaccag cggcgtgcac accttcccgg ctgtcctaca gtcctcagga       780

ctctactccc tcagcagcgt ggtgactgtg ccctctagca gcttgggcac ccagacctac       840

atctgcaacg tgaatcacaa gcccagcaac accaaggtgg acaagaaagt tgaacccaaa       900

tcttgcgaca aaactcacac atgcccaccg tgcccagcac ctccagtcgc cggaccgtca       960

gtcttcctct tccctccaaa acccaaggac accctcatga tctcccggac ccctgaggtc      1020

acatgcgtgg tggtggacgt gagccacgaa gaccctgagg tcaagttcaa ctggtacgtg      1080

gacggcgtgg aggtgcataa tgccaagaca aagccgcggg aggagcagta caacagcacg      1140

taccgtgtgg tcagcgtcct caccgtcctg caccaggact ggctgaatgg caaggagtac      1200

aagtgcaagg tctccaacaa aggcctccca agctccatcg agaaaaccat ctccaaagcc      1260

aaagggcagc cccgagaacc acaggtgtac accctgcctc catcccggga tgagctgacc      1320

aagaaccagg tcagcctgac ctgcctggtc aaaggcttct atcccagcga catcgccgtg      1380

gagtgggaga gcaatgggca gccggagaac aactacaaga ccacgcctcc cgtgctggac      1440

tccgacggct ccttcttcct ctacagcaag ctcaccgtgg acaagagcag gtggcagcag      1500

gggaacgtct tctcatgctc cgtgatgcat gaggctctgc acaaccacta cacgcagaag      1560

agcctctccc tgtctccggg taaa                                             1584

<210>  50
<211>  528
<212>  PRT
<213>  artificial sequence

<400>  50

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Glu Val Gln Leu Val Glu Ser Gly Gly Gly Leu Val Gln Pro Gly
                85                  90                  95

Gly Ser Leu Arg Leu Ser Cys Ala Ala Ser Gly Phe Thr Phe Ser Asp
            100                 105                 110

Ser Trp Ile His Trp Val Arg Gln Ala Pro Gly Lys Gly Leu Glu Trp
        115                 120                 125

Val Ala Trp Ile Ser Pro Tyr Gly Gly Ser Thr Tyr Tyr Ala Asp Ser
    130                 135                 140

Val Lys Gly Arg Phe Thr Ile Ser Ala Asp Thr Ser Lys Asn Thr Ala
145                 150                 155                 160

Tyr Leu Gln Met Asn Ser Leu Arg Ala Glu Asp Thr Ala Val Tyr Tyr
                165                 170                 175

Cys Ala Arg Arg His Trp Pro Gly Gly Phe Asp Tyr Trp Gly Gln Gly
            180                 185                 190

Thr Leu Val Thr Val Ser Ser Ala Ser Thr Lys Gly Pro Ser Val Phe
        195                 200                 205

Pro Leu Ala Pro Ser Ser Lys Ser Thr Ser Gly Gly Thr Ala Ala Leu
    210                 215                 220

Gly Cys Leu Val Lys Asp Tyr Phe Pro Glu Pro Val Thr Val Ser Trp
225                 230                 235                 240

Asn Ser Gly Ala Leu Thr Ser Gly Val His Thr Phe Pro Ala Val Leu
                245                 250                 255

Gln Ser Ser Gly Leu Tyr Ser Leu Ser Ser Val Val Thr Val Pro Ser
            260                 265                 270

Ser Ser Leu Gly Thr Gln Thr Tyr Ile Cys Asn Val Asn His Lys Pro
        275                 280                 285

Ser Asn Thr Lys Val Asp Lys Lys Val Glu Pro Lys Ser Cys Asp Lys
    290                 295                 300

Thr His Thr Cys Pro Pro Cys Pro Ala Pro Pro Val Ala Gly Pro Ser
305                 310                 315                 320

Val Phe Leu Phe Pro Pro Lys Pro Lys Asp Thr Leu Met Ile Ser Arg
                325                 330                 335

Thr Pro Glu Val Thr Cys Val Val Val Asp Val Ser His Glu Asp Pro
            340                 345                 350

Glu Val Lys Phe Asn Trp Tyr Val Asp Gly Val Glu Val His Asn Ala
        355                 360                 365

Lys Thr Lys Pro Arg Glu Glu Gln Tyr Asn Ser Thr Tyr Arg Val Val
    370                 375                 380

Ser Val Leu Thr Val Leu His Gln Asp Trp Leu Asn Gly Lys Glu Tyr
385                 390                 395                 400

Lys Cys Lys Val Ser Asn Lys Gly Leu Pro Ser Ser Ile Glu Lys Thr
                405                 410                 415

Ile Ser Lys Ala Lys Gly Gln Pro Arg Glu Pro Gln Val Tyr Thr Leu
            420                 425                 430

Pro Pro Ser Arg Asp Glu Leu Thr Lys Asn Gln Val Ser Leu Thr Cys
        435                 440                 445

Leu Val Lys Gly Phe Tyr Pro Ser Asp Ile Ala Val Glu Trp Glu Ser
    450                 455                 460

Asn Gly Gln Pro Glu Asn Asn Tyr Lys Thr Thr Pro Pro Val Leu Asp
465                 470                 475                 480

Ser Asp Gly Ser Phe Phe Leu Tyr Ser Lys Leu Thr Val Asp Lys Ser
                485                 490                 495

Arg Trp Gln Gln Gly Asn Val Phe Ser Cys Ser Val Met His Glu Ala
            500                 505                 510

Leu His Asn His Tyr Thr Gln Lys Ser Leu Ser Leu Ser Pro Gly Lys
        515                 520                 525

<210>  51
<211>  1032
<212>  DNA
<213>  artificial sequence

<400>  51
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg gacatccaga tgacccagag ccccagcagc     420

ctgagcgcca gcgtgggcga ccgcgtgacc atcacctgcc gcgccagcca ggacgtgagc     480

accgccgtgg cctggtacca gcagaagccc ggcaaggccc ccaagctgct gatctacagc     540

gccagcttcc tgtacagcgg cgtgcccagc cgcttcagcg gcagcggcag cggcaccgac     600

ttcaccctga ccatcagcag cctgcagccc gaggacttcg ccacctacta ctgccagcag     660

tacctgtacc accccgccac cttcggccag ggcaccaagg tggagatcaa gcgcacagtg     720

gcagccccca gcgtcttcat ttttccccct tccgatgaac agctgaagtc cggcactgct     780

tctgtggtct gtctgctgaa caatttctat cccagagagg ccaaggtgca gtggaaagtg     840

gacaacgctc tgcagtccgg caacagccag gagagtgtga ccgaacagga tagtaaggac     900

agcacatatt ctctgtctag taccctgaca ctgagtaagg cagattacga gaagcacaaa     960

gtgtatgcct gcgaagtcac tcatcaggga ctgtcaagcc ccgtgaccaa gagcttcaac    1020

cggggcgagt gt                                                        1032

<210>  52
<211>  344
<212>  PRT
<213>  artificial sequence

<400>  52

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Asp Ile Gln Met Thr Gln Ser Pro Ser Ser Leu Ser Ala Ser
    130                 135                 140

Val Gly Asp Arg Val Thr Ile Thr Cys Arg Ala Ser Gln Asp Val Ser
145                 150                 155                 160

Thr Ala Val Ala Trp Tyr Gln Gln Lys Pro Gly Lys Ala Pro Lys Leu
                165                 170                 175

Leu Ile Tyr Ser Ala Ser Phe Leu Tyr Ser Gly Val Pro Ser Arg Phe
            180                 185                 190

Ser Gly Ser Gly Ser Gly Thr Asp Phe Thr Leu Thr Ile Ser Ser Leu
        195                 200                 205

Gln Pro Glu Asp Phe Ala Thr Tyr Tyr Cys Gln Gln Tyr Leu Tyr His
    210                 215                 220

Pro Ala Thr Phe Gly Gln Gly Thr Lys Val Glu Ile Lys Arg Thr Val
225                 230                 235                 240

Ala Ala Pro Ser Val Phe Ile Phe Pro Pro Ser Asp Glu Gln Leu Lys
                245                 250                 255

Ser Gly Thr Ala Ser Val Val Cys Leu Leu Asn Asn Phe Tyr Pro Arg
            260                 265                 270

Glu Ala Lys Val Gln Trp Lys Val Asp Asn Ala Leu Gln Ser Gly Asn
        275                 280                 285

Ser Gln Glu Ser Val Thr Glu Gln Asp Ser Lys Asp Ser Thr Tyr Ser
    290                 295                 300

Leu Ser Ser Thr Leu Thr Leu Ser Lys Ala Asp Tyr Glu Lys His Lys
305                 310                 315                 320

Val Tyr Ala Cys Glu Val Thr His Gln Gly Leu Ser Ser Pro Val Thr
                325                 330                 335

Lys Ser Phe Asn Arg Gly Glu Cys
            340

<210>  53
<211>  1350
<212>  DNA
<213>  artificial sequence

<400>  53
caggtgaccc tgcgcgagtc cggccctgca ctggtgaagc ccacccagac cctgaccctg      60

acctgcacct tctccggctt ctccctgtcc acctccggca tgtccgtggg ctggatccgg     120

cagcctcccg gcaaggccct ggagtggctg gctgacatct ggtgggacga caagaaggac     180

tacaacccct ccctgaagtc ccgcctgacc atctccaagg acacctccaa gaaccaggtg     240

gtgctgaagg tgaccaacat ggaccccgcc gacaccgcca cctactactg cgcccgctca     300

atgattacca actggtactt cgacgtgtgg ggagccggta ccaccgtgac cgtgtcttcc     360

gcctccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgact gtgccctcta gcagcttggg cacccagacc     600

tacatctgca acgtgaatca caagcccagc aacaccaagg tggacaagaa agttgaaccc     660

aaatcttgcg acaaaactca cacatgccca ccgtgcccag cacctgaact cctgggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgggagga gcagtacaac     900

agcacgtacc gtgtggtcag cgtcctcacc gtcctgcacc aggactggct gaatggcaag     960

gagtacaagt gcaaggtctc caacaaagcc ctcccagccc ccatcgagaa aaccatctcc 1020

aaagccaaag ggcagccccg agaaccacag gtgtacaccc tgcccccatc ccgggatgag 1080

ctgaccaaga accaggtcag cctgacctgc ctggtcaaag gcttctatcc cagcgacatc 1140

gccgtggagt gggagagcaa tgggcagccg gagaacaact acaagaccac gcctcccgtg 1200

ctggactccg acggctcctt cttcctctac agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtaaa 1350

<210> 54
<211> 450
<212> PRT
<213> artificial sequence

<400> 54

Gln Val Thr Leu Arg Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Ser Val Gly Trp Ile Arg Gln Pro Pro Gly Lys Ala Leu Glu
        35                  40                  45

Trp Leu Ala Asp Ile Trp Trp Asp Asp Lys Lys Asp Tyr Asn Pro Ser
    50                  55                  60

Leu Lys Ser Arg Leu Thr Ile Ser Lys Asp Thr Ser Lys Asn Gln Val
65                  70                  75                  80

Val Leu Lys Val Thr Asn Met Asp Pro Ala Asp Thr Ala Thr Tyr Tyr
                85                  90                  95

Cys Ala Arg Ser Met Ile Thr Asn Trp Tyr Phe Asp Val Trp Gly Ala
            100                 105                 110

Gly Thr Thr Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val

                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  55
<211>  639
<212>  DNA
<213>  artificial sequence

<400>  55
gacatccaga tgacccagtc cccctccacc ctgtccgcct ccgtgggcga ccgcgtgacc      60

atcacctgca agtgccagct gtccgtgggc tacatgcact ggtaccagca gaagcccggc     120

aaggccccca agctgctgat ctacgacacc tccaagctgg cctccggcgt gccctcccgc     180

ttctccggct ccggctccgg caccgagttc accctgacca tctcctccct gcagcccgac     240

gacttcgcca cctactactg cttccagggc tccggctacc ccttcacctt cggcggcggc     300

accaagctgg agatcaaacg aactgtggct gcaccatctg tcttcatctt cccgccatct     360

gatgagcagt tgaaatctgg aactgcctct gtcgtgtgcc tgctgaataa cttctatccc     420

agagaggcca aagtacagtg gaaggtggat aacgccctcc aatcgggtaa ctcccaggag     480

agtgtcacag agcaggacag caaggacagc acctacagcc tcagcagcac cctgacgctg     540

agcaaagcag actacgagaa acacaaagtc tacgcctgcg aagtcaccca tcagggcctg     600

tcctcgcccg tcacaaagag cttcaacagg ggagagtgt                            639

<210>  56
<211>  213
<212>  PRT
<213>  artificial sequence

<400>  56

Asp Ile Gln Met Thr Gln Ser Pro Ser Thr Leu Ser Ala Ser Val Gly
1               5                   10                  15

Asp Arg Val Thr Ile Thr Cys Lys Cys Gln Leu Ser Val Gly Tyr Met
            20                  25                  30

His Trp Tyr Gln Gln Lys Pro Gly Lys Ala Pro Lys Leu Leu Ile Tyr
        35                  40                  45

Asp Thr Ser Lys Leu Ala Ser Gly Val Pro Ser Arg Phe Ser Gly Ser
    50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Phe Gln Gly Ser Gly Tyr Pro Phe Thr
                85                  90                  95

Phe Gly Gly Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  57
<211>  1032
<212>  DNA
<213>  artificial sequence

<400>  57
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg gagatcgtgc tgacccagag ccccgccacc     420

ctgagcctga gccccggcga gcgcgccacc ctgagctgcc gcgccagcca gagcgtgagc     480

agctacctgg cctggtacca gcagaagcca ggacaggctc cacgactgct aatctatgac 540

gccagcaacc gcgccaccgg catccccgcc cgcttcagcg gcagcggcag cggcaccgac 600

ttcaccctga ccatcagcag cctggagccc gaggacttcg ccgtgtacta ctgccagcag 660

agcagcaact ggccccgcac cttcggccag ggcaccaagg tggagatcaa gcgcacagtg 720

gcagccccca gcgtcttcat ttttccccct tccgatgaac agctgaagtc cggcactgct 780

tctgtggtct gtctgctgaa caatttctat cccagagagg ccaaggtgca gtggaaagtg 840

gacaacgctc tgcagtccgg caacagccag gagagtgtga ccgaacagga tagtaaggac 900

agcacatatt ctctgtctag taccctgaca ctgagtaagg cagattacga gaagcacaaa 960

gtgtatgcct gcgaagtcac tcatcaggga ctgtcaagcc ccgtgaccaa gagcttcaac 1020

cggggcgagt gt 1032

<210> 58
<211> 344
<212> PRT
<213> artificial sequence

<400> 58

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Glu Ile Val Leu Thr Gln Ser Pro Ala Thr Leu Ser Leu Ser
    130                 135                 140

Pro Gly Glu Arg Ala Thr Leu Ser Cys Arg Ala Ser Gln Ser Val Ser
145                 150                 155                 160

Ser Tyr Leu Ala Trp Tyr Gln Gln Lys Pro Gly Gln Ala Pro Arg Leu
                165                 170                 175

Leu Ile Tyr Asp Ala Ser Asn Arg Ala Thr Gly Ile Pro Ala Arg Phe
            180                 185                 190

Ser Gly Ser Gly Ser Gly Thr Asp Phe Thr Leu Thr Ile Ser Ser Leu
        195                 200                 205

Glu Pro Glu Asp Phe Ala Val Tyr Tyr Cys Gln Gln Ser Ser Asn Trp
    210                 215                 220

Pro Arg Thr Phe Gly Gln Gly Thr Lys Val Glu Ile Lys Arg Thr Val
225                 230                 235                 240

Ala Ala Pro Ser Val Phe Ile Phe Pro Pro Ser Asp Glu Gln Leu Lys
                245                 250                 255

Ser Gly Thr Ala Ser Val Val Cys Leu Leu Asn Asn Phe Tyr Pro Arg
            260                 265                 270

Glu Ala Lys Val Gln Trp Lys Val Asp Asn Ala Leu Gln Ser Gly Asn
        275                 280                 285

Ser Gln Glu Ser Val Thr Glu Gln Asp Ser Lys Asp Ser Thr Tyr Ser
    290                 295                 300

Leu Ser Ser Thr Leu Thr Leu Ser Lys Ala Asp Tyr Glu Lys His Lys
305                 310                 315                 320

Val Tyr Ala Cys Glu Val Thr His Gln Gly Leu Ser Ser Pro Val Thr
                325                 330                 335

Lys Ser Phe Asn Arg Gly Glu Cys
            340

<210>  59
<211>  1740
<212>  DNA
<213>  artificial sequence

<400>  59
aactgggtga acgtgatcag cgacctgaag aagatcgagg acctgatcca gagcatgcac        60

atcgacgcca ccctgtacac cgagagcgac gtgcacccca gctgcaaggt gaccgccatg       120

aagtgcttcc tgctggagct gcaggtgatc agcctggaga gcggcgacgc cagcatccac 180

gacaccgtgg agaacctgat catcctggcc aacgacagcc tgagcagcaa cggcaacgtg 240

accgagagcg gctgcaagga gtgcgaggag ctggaggaga agaacatcaa ggagttcctg 300

cagagcttcg tgcacatcgt gcagatgttc atcaacacca gcggcggagg cggatccgga 360

ggcggaggtt ccggcggggg tgggagcggg gaagtacagc tgcttgagag tggaggaggt 420

ttggtacagc ccggcggatc cctccgcctg tcctgtgcgg ctagtggctt tacattctca 480

tcctatatca tgatgtgggt aagacaggcc ccaggaaagg gcctggagtg ggttagttct 540

atctacccct caggcgggat taccttctac gcagatactg tgaagggcag gtttaccata 600

tcccgagaca acagtaagaa taccctttac cttcaaatga actcccttcg ggccgaggac 660

actgcggtgt actattgcgc tcgcattaag cttggcaccg tgacaaccgt gaactattgg 720

ggtcaaggca cgctggtgac tgtctcttcc gcctccacca agggcccatc ggtcttcccc 780

ctggcaccct cctccaagag cacctctggg ggcacagcgg ccctgggctg cctggtcaag 840

gactacttcc ccgaaccggt gacggtgtcg tggaactcag gcgccctgac cagcggcgtg 900

cacaccttcc cggctgtcct acagtcctca ggactctact ccctcagcag cgtggtgact 960

gtgccctcta gcagcttggg cacccagacc tacatctgca acgtgaatca caagcccagc 1020

aacaccaagg tggacaagaa agttgaaccc aaatcttgcg acaaaactca cacatgccca 1080

ccgtgcccag cacctgaact cctgggggga ccgtcagtct tcctcttccc cccaaaaccc 1140

aaggacaccc tcatgatctc ccggacccct gaggtcacat gcgtggtggt ggacgtgagc 1200

cacgaagacc ctgaggtcaa gttcaactgg tacgtggacg gcgtggaggt gcataatgcc 1260

aagacaaagc cgcgggagga gcagtacaac agcacgtacc gtgtggtcag cgtcctcacc 1320

gtcctgcacc aggactggct gaatggcaag gagtacaagt gcaaggtctc caacaaagcc 1380

ctcccagccc ccatagagaa aaccatctcc aaagccaaag ggcagccccg agaaccacag 1440

gtgtacaccc tgcccccatc ccgggatgag ctgaccaaga accaggtcag cctgacctgc 1500

ctggtcaaag gcttctatcc cagcgacatc gccgtggagt gggagagcaa tgggcagccg 1560

gagaacaact acaagaccac gcctcccgtg ctggactccg acggctcctt cttcctctac 1620

agcaagctca ccgtggacaa gagcaggtgg cagcagggga acgtcttctc atgctccgtg 1680

atgcatgagg ctctgcacaa ccactacacg cagaagagcc tctccctgtc tccgggtaaa 1740

<210> 60
<211> 580
<212> PRT
<213> artificial sequence

<400> 60

Asn Trp Val Asn Val Ile Ser Asp Leu Lys Lys Ile Glu Asp Leu Ile

1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Glu Val Gln Leu Leu Glu Ser Gly Gly Gly Leu Val Gln Pro
    130                 135                 140

Gly Gly Ser Leu Arg Leu Ser Cys Ala Ala Ser Gly Phe Thr Phe Ser
145                 150                 155                 160

Ser Tyr Ile Met Met Trp Val Arg Gln Ala Pro Gly Lys Gly Leu Glu
                165                 170                 175

Trp Val Ser Ser Ile Tyr Pro Ser Gly Gly Ile Thr Phe Tyr Ala Asp
            180                 185                 190

Thr Val Lys Gly Arg Phe Thr Ile Ser Arg Asp Asn Ser Lys Asn Thr
        195                 200                 205

Leu Tyr Leu Gln Met Asn Ser Leu Arg Ala Glu Asp Thr Ala Val Tyr
    210                 215                 220

Tyr Cys Ala Arg Ile Lys Leu Gly Thr Val Thr Thr Val Asn Tyr Trp
225                 230                 235                 240

Gly Gln Gly Thr Leu Val Thr Val Ser Ser Ala Ser Thr Lys Gly Pro
                245                 250                 255

Ser Val Phe Pro Leu Ala Pro Ser Ser Lys Ser Thr Ser Gly Gly Thr
            260                 265                 270

Ala Ala Leu Gly Cys Leu Val Lys Asp Tyr Phe Pro Glu Pro Val Thr
        275                 280                 285

Val Ser Trp Asn Ser Gly Ala Leu Thr Ser Gly Val His Thr Phe Pro
    290                 295                 300

Ala Val Leu Gln Ser Ser Gly Leu Tyr Ser Leu Ser Ser Val Val Thr
305                 310                 315                 320

Val Pro Ser Ser Ser Leu Gly Thr Gln Thr Tyr Ile Cys Asn Val Asn
                325                 330                 335

His Lys Pro Ser Asn Thr Lys Val Asp Lys Lys Val Glu Pro Lys Ser
            340                 345                 350

Cys Asp Lys Thr His Thr Cys Pro Pro Cys Pro Ala Pro Glu Leu Leu
        355                 360                 365

Gly Gly Pro Ser Val Phe Leu Phe Pro Pro Lys Pro Lys Asp Thr Leu
    370                 375                 380

Met Ile Ser Arg Thr Pro Glu Val Thr Cys Val Val Val Asp Val Ser
385                 390                 395                 400

His Glu Asp Pro Glu Val Lys Phe Asn Trp Tyr Val Asp Gly Val Glu
                405                 410                 415

Val His Asn Ala Lys Thr Lys Pro Arg Glu Glu Gln Tyr Asn Ser Thr
            420                 425                 430

Tyr Arg Val Val Ser Val Leu Thr Val Leu His Gln Asp Trp Leu Asn
        435                 440                 445

Gly Lys Glu Tyr Lys Cys Lys Val Ser Asn Lys Ala Leu Pro Ala Pro
    450                 455                 460

Ile Glu Lys Thr Ile Ser Lys Ala Lys Gly Gln Pro Arg Glu Pro Gln
465                 470                 475                 480

Val Tyr Thr Leu Pro Pro Ser Arg Asp Glu Leu Thr Lys Asn Gln Val
                485                 490                 495

Ser Leu Thr Cys Leu Val Lys Gly Phe Tyr Pro Ser Asp Ile Ala Val
            500                 505                 510

Glu Trp Glu Ser Asn Gly Gln Pro Glu Asn Asn Tyr Lys Thr Thr Pro
        515                 520                 525

Pro Val Leu Asp Ser Asp Gly Ser Phe Phe Leu Tyr Ser Lys Leu Thr
    530                 535                 540

Val Asp Lys Ser Arg Trp Gln Gln Gly Asn Val Phe Ser Cys Ser Val
545                 550                 555                 560

Met His Glu Ala Leu His Asn His Tyr Thr Gln Lys Ser Leu Ser Leu
                565                 570                 575

Ser Pro Gly Lys
            580

<210> 61
<211> 891
<212> DNA
<213> artificial sequence

<400> 61
atcacctgcc cacctcccat gagcgtggag cacgccgaca tctgggtgaa gagctacagc 60

ctgtacagcc gcgagcgcta catctgcaac agcggcttca agcgcaaggc cggcaccagc 120

agcctgaccg agtgcgtgct gaacaaggcc accaacgtgg cccactggac cacccccagc 180

ctgaagtgca tccgcggcgg aggcggatcc ggaggcggag gttccggcgg gggtgggagc 240

gggcaatccg cactgactca accagccagc gttagcggct cccctggtca atctatcacc 300

atcagctgta ccgggaccag ctcagacgtt ggcggttaca actacgtcag ctggtaccag 360

cagcacccgg gtaaagctcc aaagctgatg atttatgatg tgtctaatcg accttctggt 420

gtatctaacc gattttcagg ctctaaaagt ggaaatactg cttccctcac gatctcaggg 480

ctgcaagccg aagacgaagc cgattattat tgttctagct atacatccag cagcacccgc 540

gtgtttggaa cgggaaccaa ggtcacggtt ctgggacagc ccaaagccaa tcctaccgtc 600

actctgttcc cacccagtag tgaggagctg caggcaaata aggctaccct ggtctgtctt 660

atatccgatt tctatcccgg ggcagtcaca gtcgcttgga aggcagatgg ctctccagtg 720

aaggccggcg tcgaaacaac taaaccttcc aagcagtcta ataacaagta cgctgcttct 780

tcttaccttt cacttactcc tgaacaatgg aagagccaca ggagttactc ttgtcaggta 840

acccacgagg ggtccactgt ggagaaaacc gtcgctccca cagagtgttc t 891

<210> 62
<211> 297
<212> PRT
<213> artificial sequence

<400>  62

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Gln Ser Ala Leu Thr Gln Pro Ala Ser Val Ser Gly Ser Pro Gly
                85                  90                  95

Gln Ser Ile Thr Ile Ser Cys Thr Gly Thr Ser Ser Asp Val Gly Gly
            100                 105                 110

Tyr Asn Tyr Val Ser Trp Tyr Gln Gln His Pro Gly Lys Ala Pro Lys
        115                 120                 125

Leu Met Ile Tyr Asp Val Ser Asn Arg Pro Ser Gly Val Ser Asn Arg
    130                 135                 140

Phe Ser Gly Ser Lys Ser Gly Asn Thr Ala Ser Leu Thr Ile Ser Gly
145                 150                 155                 160

Leu Gln Ala Glu Asp Glu Ala Asp Tyr Tyr Cys Ser Ser Tyr Thr Ser
                165                 170                 175

Ser Ser Thr Arg Val Phe Gly Thr Gly Thr Lys Val Thr Val Leu Gly
            180                 185                 190

Gln Pro Lys Ala Asn Pro Thr Val Thr Leu Phe Pro Pro Ser Ser Glu
        195                 200                 205

Glu Leu Gln Ala Asn Lys Ala Thr Leu Val Cys Leu Ile Ser Asp Phe
    210                 215                 220

Tyr Pro Gly Ala Val Thr Val Ala Trp Lys Ala Asp Gly Ser Pro Val
225                 230                 235                 240

Lys Ala Gly Val Glu Thr Thr Lys Pro Ser Lys Gln Ser Asn Asn Lys

245                 250                 255

Tyr Ala Ala Ser Ser Tyr Leu Ser Leu Thr Pro Glu Gln Trp Lys Ser
            260                 265                 270

His Arg Ser Tyr Ser Cys Gln Val Thr His Glu Gly Ser Thr Val Glu
        275                 280                 285

Lys Thr Val Ala Pro Thr Glu Cys Ser
    290                 295

<210>  63
<211>  1593
<212>  DNA
<213>  artificial sequence

<400>  63
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

ggggaagtac agctgcttga gagtggagga ggtttggtac agcccggcgg atccctccgc     300

ctgtcctgtg cggctagtgg ctttacattc tcatcctata tcatgatgtg ggtaagacag     360

gccccaggaa agggcctgga gtgggttagt tctatctacc cctcaggcgg gattaccttc     420

tacgcagata ctgtgaaggg caggtttacc atatcccgag acaacagtaa gaataccctt     480

taccttcaaa tgaactccct tcgggccgag gacactgcgg tgtactattg cgctcgcatt     540

aagcttggca ccgtgacaac cgtgaactat tggggtcaag gcacgctggt gactgtctct     600

tccgcctcca ccaagggccc atcggtcttc cccctggcac cctcctccaa gagcacctct     660

gggggcacag cggccctggg ctgcctggtc aaggactact tccccgaacc ggtgacggtg     720

tcgtggaact caggcgccct gaccagcggc gtgcacacct tcccggctgt cctacagtcc     780

tcaggactct actccctcag cagcgtggtg actgtgccct ctagcagctt gggcacccag     840

acctacatct gcaacgtgaa tcacaagccc agcaacacca aggtggacaa gaaagttgaa     900

cccaaatctt gcgacaaaac tcacacatgc ccaccgtgcc cagcacctga actcctgggg     960

ggaccgtcag tcttcctctt ccccccaaaa cccaaggaca ccctcatgat ctcccggacc    1020

cctgaggtca catgcgtggt ggtggacgtg agccacgaag accctgaggt caagttcaac    1080

tggtacgtgg acggcgtgga ggtgcataat gccaagacaa agccgcggga ggagcagtac    1140

aacagcacgt accgtgtggt cagcgtcctc accgtcctgc accaggactg gctgaatggc    1200

aaggagtaca agtgcaaggt ctccaacaaa gccctcccag cccccataga gaaaaccatc    1260

tccaaagcca aagggcagcc ccgagaacca caggtgtaca ccctgccccc atcccgggat 1320

gagctgacca agaaccaggt cagcctgacc tgcctggtca aaggcttcta tcccagcgac 1380

atcgccgtgg agtgggagag caatgggcag ccggagaaca actacaagac cacgcctccc 1440

gtgctggact ccgacggctc cttcttcctc tacagcaagc tcaccgtgga caagagcagg 1500

tggcagcagg ggaacgtctt ctcatgctcc gtgatgcatg aggctctgca caaccactac 1560

acgcagaaga gcctctccct gtctccgggt aaa 1593

<210> 64
<211> 531
<212> PRT
<213> artificial sequence

<400> 64

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Glu Val Gln Leu Leu Glu Ser Gly Gly Gly Leu Val Gln Pro Gly
                85                  90                  95

Gly Ser Leu Arg Leu Ser Cys Ala Ala Ser Gly Phe Thr Phe Ser Ser
            100                 105                 110

Tyr Ile Met Met Trp Val Arg Gln Ala Pro Gly Lys Gly Leu Glu Trp
        115                 120                 125

Val Ser Ser Ile Tyr Pro Ser Gly Gly Ile Thr Phe Tyr Ala Asp Thr
    130                 135                 140

Val Lys Gly Arg Phe Thr Ile Ser Arg Asp Asn Ser Lys Asn Thr Leu
145                 150                 155                 160

Tyr Leu Gln Met Asn Ser Leu Arg Ala Glu Asp Thr Ala Val Tyr Tyr
                165                 170                 175

Cys Ala Arg Ile Lys Leu Gly Thr Val Thr Thr Val Asn Tyr Trp Gly
            180                 185                 190

Gln Gly Thr Leu Val Thr Val Ser Ser Ala Ser Thr Lys Gly Pro Ser
        195                 200                 205

Val Phe Pro Leu Ala Pro Ser Ser Lys Ser Thr Ser Gly Gly Thr Ala
    210                 215                 220

Ala Leu Gly Cys Leu Val Lys Asp Tyr Phe Pro Glu Pro Val Thr Val
225                 230                 235                 240

Ser Trp Asn Ser Gly Ala Leu Thr Ser Gly Val His Thr Phe Pro Ala
                245                 250                 255

Val Leu Gln Ser Ser Gly Leu Tyr Ser Leu Ser Ser Val Val Thr Val
            260                 265                 270

Pro Ser Ser Ser Leu Gly Thr Gln Thr Tyr Ile Cys Asn Val Asn His
        275                 280                 285

Lys Pro Ser Asn Thr Lys Val Asp Lys Lys Val Glu Pro Lys Ser Cys
    290                 295                 300

Asp Lys Thr His Thr Cys Pro Pro Cys Pro Ala Pro Glu Leu Leu Gly
305                 310                 315                 320

Gly Pro Ser Val Phe Leu Phe Pro Pro Lys Pro Lys Asp Thr Leu Met
                325                 330                 335

Ile Ser Arg Thr Pro Glu Val Thr Cys Val Val Val Asp Val Ser His
            340                 345                 350

Glu Asp Pro Glu Val Lys Phe Asn Trp Tyr Val Asp Gly Val Glu Val
        355                 360                 365

His Asn Ala Lys Thr Lys Pro Arg Glu Glu Gln Tyr Asn Ser Thr Tyr
    370                 375                 380

Arg Val Val Ser Val Leu Thr Val Leu His Gln Asp Trp Leu Asn Gly
385                 390                 395                 400

Lys Glu Tyr Lys Cys Lys Val Ser Asn Lys Ala Leu Pro Ala Pro Ile
                405                 410                 415

Glu Lys Thr Ile Ser Lys Ala Lys Gly Gln Pro Arg Glu Pro Gln Val
            420                 425                 430

Tyr Thr Leu Pro Pro Ser Arg Asp Glu Leu Thr Lys Asn Gln Val Ser
        435                 440                 445

Leu Thr Cys Leu Val Lys Gly Phe Tyr Pro Ser Asp Ile Ala Val Glu
    450                 455                 460

Trp Glu Ser Asn Gly Gln Pro Glu Asn Asn Tyr Lys Thr Thr Pro Pro
465                 470                 475                 480

Val Leu Asp Ser Asp Gly Ser Phe Phe Leu Tyr Ser Lys Leu Thr Val
                485                 490                 495

Asp Lys Ser Arg Trp Gln Gln Gly Asn Val Phe Ser Cys Ser Val Met
            500                 505                 510

His Glu Ala Leu His Asn His Tyr Thr Gln Lys Ser Leu Ser Leu Ser
        515                 520                 525

Pro Gly Lys
    530

<210>  65
<211>  1038
<212>  DNA
<213>  artificial sequence

<400>  65
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg caatccgcac tgactcaacc agccagcgtt     420

agcggctccc ctggtcaatc tatcaccatc agctgtaccg ggaccagctc agacgttggc     480

ggttacaact acgtcagctg gtaccagcag cacccgggta aagctccaaa gctgatgatt     540

tatgatgtgt ctaatcgacc ttctggtgta tctaaccgat tttcaggctc taaaagtgga     600

aatactgctt ccctcacgat ctcagggctg caagccgaag acgaagccga ttattattgt     660

tctagctata catccagcag cacccgcgtg tttggaacgg gaaccaaggt cacggttctg     720

ggacagccca aagccaatcc taccgtcact ctgttcccac ccagtagtga ggagctgcag     780

gcaaataagg ctaccctggt ctgtcttata tccgatttct atcccggggc agtcacagtc     840

gcttggaagg cagatggctc tccagtgaag gccggcgtcg aaacaactaa accttccaag 900

cagtctaata acaagtacgc tgcttcttct tacctttcac ttactcctga acaatggaag 960

agccacagga gttactcttg tcaggtaacc cacgaggggt ccactgtgga gaaaaccgtc 1020

gctcccacag agtgttct 1038

<210> 66
<211> 346
<212> PRT
<213> artificial sequence

<400> 66

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Gln Ser Ala Leu Thr Gln Pro Ala Ser Val Ser Gly Ser Pro
    130                 135                 140

Gly Gln Ser Ile Thr Ile Ser Cys Thr Gly Thr Ser Ser Asp Val Gly
145                 150                 155                 160

Gly Tyr Asn Tyr Val Ser Trp Tyr Gln Gln His Pro Gly Lys Ala Pro
                165                 170                 175

Lys Leu Met Ile Tyr Asp Val Ser Asn Arg Pro Ser Gly Val Ser Asn
            180                 185                 190

Arg Phe Ser Gly Ser Lys Ser Gly Asn Thr Ala Ser Leu Thr Ile Ser
        195                 200                 205

Gly Leu Gln Ala Glu Asp Glu Ala Asp Tyr Tyr Cys Ser Ser Tyr Thr
    210                 215                 220

Ser Ser Ser Thr Arg Val Phe Gly Thr Gly Thr Lys Val Thr Val Leu
225                 230                 235                 240

Gly Gln Pro Lys Ala Asn Pro Thr Val Thr Leu Phe Pro Pro Ser Ser
                245                 250                 255

Glu Glu Leu Gln Ala Asn Lys Ala Thr Leu Val Cys Leu Ile Ser Asp
            260                 265                 270

Phe Tyr Pro Gly Ala Val Thr Val Ala Trp Lys Ala Asp Gly Ser Pro
        275                 280                 285

Val Lys Ala Gly Val Glu Thr Thr Lys Pro Ser Lys Gln Ser Asn Asn
    290                 295                 300

Lys Tyr Ala Ala Ser Ser Tyr Leu Ser Leu Thr Pro Glu Gln Trp Lys
305                 310                 315                 320

Ser His Arg Ser Tyr Ser Cys Gln Val Thr His Glu Gly Ser Thr Val
                325                 330                 335

Glu Lys Thr Val Ala Pro Thr Glu Cys Ser
            340                 345

<210>  67
<211>  1740
<212>  DNA
<213>  artificial sequence

<400>  67
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg caggtgaccc tgcgcgagtc cggccctgca     420

ctggtgaagc ccacccagac cctgaccctg acctgcacct tctccggctt ctccctgtcc     480

acctccggca tgtccgtggg ctggatccgg cagcctcccg gcaaggccct ggagtggctg 540

gctgacatct ggtgggacga caagaaggac tacaacccct ccctgaagtc ccgcctgacc 600

atctccaagg acacctccaa gaaccaggtg gtgctgaagg tgaccaacat ggaccccgcc 660

gacaccgcca cctactactg cgcccgctca atgattacca actggtactt cgacgtgtgg 720

ggagccggta ccaccgtgac cgtgtcttcc gcctccacca agggcccatc ggtcttcccc 780

ctggcaccct cctccaagag cacctctggg ggcacagcgg ccctgggctg cctggtcaag 840

gactacttcc ccgaaccggt gacggtgtcg tggaactcag gcgccctgac cagcggcgtg 900

cacaccttcc cggctgtcct acagtcctca ggactctact ccctcagcag cgtggtgact 960

gtgccctcta gcagcttggg cacccagacc tacatctgca acgtgaatca caagcccagc 1020

aacaccaagg tggacaagaa agttgaaccc aaatcttgcg acaaaactca cacatgccca 1080

ccgtgcccag cacctgaact cctgggggga ccgtcagtct tcctcttccc cccaaaaccc 1140

aaggacaccc tcatgatctc ccggacccct gaggtcacat gcgtggtggt ggacgtgagc 1200

cacgaagacc ctgaggtcaa gttcaactgg tacgtggacg gcgtggaggt gcataatgcc 1260

aagacaaagc cgcgggagga gcagtacaac agcacgtacc gtgtggtcag cgtcctcacc 1320

gtcctgcacc aggactggct gaatggcaag gagtacaagt gcaaggtctc caacaaagcc 1380

ctcccagccc ccatagagaa aaccatctcc aaagccaaag ggcagccccg agaaccacag 1440

gtgtacaccc tgcccccatc ccgggaggag atgaccaaga accaggtcag cctgacctgc 1500

ctggtcaaag gcttctatcc cagcgacatc gccgtggagt gggagagcaa tgggcagccg 1560

gagaacaact acaagaccac gcctcccgtg ctggactccg acggctcctt cttcctctac 1620

agcaagctca ccgtggacaa gagcaggtgg cagcagggga acgtcttctc atgctccgtg 1680

atgcatgagg ctctgcacaa ccactacacg cagaagagcc tctccctgtc tccgggtaaa 1740

<210> 68
<211> 563
<212> PRT
<213> artificial sequence

<400> 68

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Gln Val Thr Leu Arg Glu Ser Gly Pro Ala Leu Val Lys Pro
    130                 135                 140

Thr Gln Thr Leu Thr Leu Thr Cys Thr Phe Ser Gly Phe Ser Leu Ser
145                 150                 155                 160

Thr Ser Gly Met Ser Val Gly Trp Ile Arg Gln Pro Pro Gly Lys Ala
                165                 170                 175

Leu Glu Trp Leu Ala Asp Ile Trp Trp Asp Asp Lys Lys Asp Tyr Asn
            180                 185                 190

Pro Ser Leu Lys Ser Arg Leu Thr Ile Ser Lys Asp Thr Ser Lys Asn
        195                 200                 205

Gln Val Val Leu Lys Val Thr Asn Met Asp Pro Ala Asp Thr Ala Thr
    210                 215                 220

Tyr Tyr Cys Ala Arg Ser Met Ile Thr Asn Trp Tyr Phe Asp Val Trp
225                 230                 235                 240

Gly Ala Gly Thr Thr Val Thr Val Ser Ser Ala Ser Thr Lys Gly Pro
                245                 250                 255

Ser Val Phe Pro Leu Ala Pro Ser Ser Lys Ser Thr Ser Gly Gly Thr
            260                 265                 270

Ala Ala Leu Gly Cys Leu Val Lys Asp Tyr Phe Pro Glu Pro Val Thr
        275                 280                 285

Val Ser Trp Asn Ser Gly Ala Leu Thr Ser Gly Val His Thr Phe Pro
    290                 295                 300

Ala Val Leu Gln Ser Ser Gly Leu Tyr Ser Leu Ser Ser Val Val Thr
305                 310                 315                 320

Val Pro Ser Ser Ser Leu Gly Thr Gln Thr Tyr Ile Cys Asn Val Asn
                325                 330                 335

His Lys Pro Ser Asn Thr Lys Val Asp Lys Lys Val Glu Pro Lys Ser
            340                 345                 350

Cys Asp Lys Thr His Thr Cys Pro Pro Cys Pro Ala Pro Glu Leu Leu
        355                 360                 365

Gly Gly Pro Ser Val Phe Leu Phe Pro Pro Lys Pro Lys Asp Thr Leu
    370                 375                 380

Met Ile Ser Arg Thr Pro Glu Val Thr Cys Val Val Val Asp Val Ser
385                 390                 395                 400

His Glu Asp Pro Glu Val Lys Phe Asn Trp Tyr Val Asp Gly Val Glu
                405                 410                 415

Val His Asn Ala Lys Thr Lys Pro Arg Glu Glu Gln Tyr Asn Ser Thr
            420                 425                 430

Tyr Arg Val Val Ser Val Leu Thr Val Leu His Gln Asp Trp Leu Asn
        435                 440                 445

Gly Lys Glu Tyr Lys Cys Lys Val Ser Asn Lys Ala Leu Pro Ala Pro
    450                 455                 460

Ile Glu Lys Thr Ile Ser Lys Ala Lys Gly Gln Pro Arg Glu Pro Gln
465                 470                 475                 480

Val Tyr Thr Leu Pro Pro Ser Arg Glu Glu Met Thr Lys Asn Gln Val
                485                 490                 495

Ser Leu Thr Cys Leu Val Lys Gly Phe Tyr Pro Ser Asp Ile Ala Val
            500                 505                 510

Glu Trp Glu Ser Asn Gly Gln Pro Glu Asn Asn Tyr Lys Thr Thr Pro
        515                 520                 525

Pro Val Leu Asp Ser Asp Gly Ser Phe Phe Leu Tyr Ser Lys Leu Thr
    530                 535                 540

Val Asp Lys Ser Arg Trp Gln Gln Gly Asn Val Phe Ser Cys Ser Val
545                 550                 555                 560

Met His Glu

<210> 69
<211> 1593
<212> DNA
<213> artificial sequence

<400> 69
atcacctgcc cacctcccat gagcgtggag cacgccgaca tctgggtgaa gagctacagc 60

ctgtacagcc gcgagcgcta catctgcaac agcggcttca agcgcaaggc cggcaccagc 120

agcctgaccg agtgcgtgct gaacaaggcc accaacgtgg cccactggac cacccccagc 180

ctgaagtgca tccgcggcgg aggcggatcc ggaggcggag gttccggcgg gggtgggagc 240

gggcaggtga ccctgcgcga gtccggccct gcactggtga agcccaccca gaccctgacc 300

ctgacctgca ccttctccgg cttctccctg tccacctccg gcatgtccgt gggctggatc 360

cggcagcctc ccggcaaggc cctggagtgg ctggctgaca tctggtggga cgacaagaag 420

gactacaacc cctccctgaa gtcccgcctg accatctcca aggacacctc caagaaccag 480

gtggtgctga aggtgaccaa catggacccc gccgacaccg ccacctacta ctgcgcccgc 540

tcaatgatta ccaactggta cttcgacgtg tggggagccg gtaccaccgt gaccgtgtct 600

tccgcctcca ccaagggccc atcggtcttc cccctggcac cctcctccaa gagcacctct 660

gggggcacag cggccctggg ctgcctggtc aaggactact tccccgaacc ggtgacggtg 720

tcgtggaact caggcgccct gaccagcggc gtgcacacct tcccggctgt cctacagtcc 780

tcaggactct actccctcag cagcgtggtg actgtgccct ctagcagctt gggcacccag 840

acctacatct gcaacgtgaa tcacaagccc agcaacacca aggtggacaa gaaagttgaa 900

cccaaatctt gcgacaaaac tcacacatgc ccaccgtgcc cagcacctga actcctgggg 960

ggaccgtcag tcttcctctt ccccccaaaa cccaaggaca ccctcatgat ctcccggacc 1020

cctgaggtca catgcgtggt ggtggacgtg agccacgaag accctgaggt caagttcaac 1080

tggtacgtgg acggcgtgga ggtgcataat gccaagacaa agccgcggga ggagcagtac 1140

aacagcacgt accgtgtggt cagcgtcctc accgtcctgc accaggactg gctgaatggc 1200

aaggagtaca agtgcaaggt ctccaacaaa gccctcccag cccccataga gaaaaccatc 1260

tccaaagcca aagggcagcc ccgagaacca caggtgtaca ccctgccccc atcccgggag 1320

gagatgacca agaaccaggt cagcctgacc tgcctggtca aaggcttcta tcccagcgac 1380

atcgccgtgg agtgggagag caatgggcag ccggagaaca actacaagac cacgcctccc 1440

gtgctggact ccgacggctc cttcttcctc tacagcaagc tcaccgtgga caagagcagg 1500

tggcagcagg ggaacgtctt ctcatgctcc gtgatgcatg aggctctgca caaccactac 1560

acgcagaaga gcctctccct gtctccgggt aaa                                    1593

<210>  70
<211>  531
<212>  PRT
<213>  artificial sequence

<400>  70

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Gln Val Thr Leu Arg Glu Ser Gly Pro Ala Leu Val Lys Pro Thr
                85                  90                  95

Gln Thr Leu Thr Leu Thr Cys Thr Phe Ser Gly Phe Ser Leu Ser Thr
            100                 105                 110

Ser Gly Met Ser Val Gly Trp Ile Arg Gln Pro Pro Gly Lys Ala Leu
        115                 120                 125

Glu Trp Leu Ala Asp Ile Trp Trp Asp Asp Lys Lys Asp Tyr Asn Pro
    130                 135                 140

Ser Leu Lys Ser Arg Leu Thr Ile Ser Lys Asp Thr Ser Lys Asn Gln
145                 150                 155                 160

Val Val Leu Lys Val Thr Asn Met Asp Pro Ala Asp Thr Ala Thr Tyr
                165                 170                 175

Tyr Cys Ala Arg Ser Met Ile Thr Asn Trp Tyr Phe Asp Val Trp Gly
            180                 185                 190

Ala Gly Thr Thr Val Thr Val Ser Ser Ala Ser Thr Lys Gly Pro Ser
        195                 200                 205

Val Phe Pro Leu Ala Pro Ser Ser Lys Ser Thr Ser Gly Gly Thr Ala
    210                 215                 220

Ala Leu Gly Cys Leu Val Lys Asp Tyr Phe Pro Glu Pro Val Thr Val
225                 230                 235                 240

Ser Trp Asn Ser Gly Ala Leu Thr Ser Gly Val His Thr Phe Pro Ala
                245                 250                 255

Val Leu Gln Ser Ser Gly Leu Tyr Ser Leu Ser Ser Val Val Thr Val
            260                 265                 270

Pro Ser Ser Ser Leu Gly Thr Gln Thr Tyr Ile Cys Asn Val Asn His
        275                 280                 285

Lys Pro Ser Asn Thr Lys Val Asp Lys Lys Val Glu Pro Lys Ser Cys
    290                 295                 300

Asp Lys Thr His Thr Cys Pro Pro Cys Pro Ala Pro Glu Leu Leu Gly
305                 310                 315                 320

Gly Pro Ser Val Phe Leu Phe Pro Pro Lys Pro Lys Asp Thr Leu Met
                325                 330                 335

Ile Ser Arg Thr Pro Glu Val Thr Cys Val Val Val Asp Val Ser His
            340                 345                 350

Glu Asp Pro Glu Val Lys Phe Asn Trp Tyr Val Asp Gly Val Glu Val
        355                 360                 365

His Asn Ala Lys Thr Lys Pro Arg Glu Glu Gln Tyr Asn Ser Thr Tyr
    370                 375                 380

Arg Val Val Ser Val Leu Thr Val Leu His Gln Asp Trp Leu Asn Gly
385                 390                 395                 400

Lys Glu Tyr Lys Cys Lys Val Ser Asn Lys Ala Leu Pro Ala Pro Ile
                405                 410                 415

Glu Lys Thr Ile Ser Lys Ala Lys Gly Gln Pro Arg Glu Pro Gln Val
            420                 425                 430

Tyr Thr Leu Pro Pro Ser Arg Glu Glu Met Thr Lys Asn Gln Val Ser
        435                 440                 445

Leu Thr Cys Leu Val Lys Gly Phe Tyr Pro Ser Asp Ile Ala Val Glu
    450                 455                 460

Trp Glu Ser Asn Gly Gln Pro Glu Asn Asn Tyr Lys Thr Thr Pro Pro
465                 470                 475                 480

Val Leu Asp Ser Asp Gly Ser Phe Phe Leu Tyr Ser Lys Leu Thr Val
                485                 490                 495

Asp Lys Ser Arg Trp Gln Gln Gly Asn Val Phe Ser Cys Ser Val Met
            500                 505                 510

His Glu Ala Leu His Asn His Tyr Thr Gln Lys Ser Leu Ser Leu Ser
        515                 520                 525

Pro Gly Lys
    530

<210>  71
<211>  342
<212>  DNA
<213>  artificial sequence

<400>  71
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gc                        342

<210>  72
<211>  114
<212>  PRT
<213>  artificial sequence

<400>  72

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser

<210>  73
<211>  195
<212>  DNA
<213>  artificial sequence

<400>  73
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgc                                                      195

<210>  74
<211>  65
<212>  PRT
<213>  artificial sequence

<400>  74

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg
65

<210>  75
<211>  1326
<212>  DNA
<213>  artificial sequence

<400>  75
caggtgcagc tggtggagag tggaggtggc gtggtacagc ccggccgcag cctgcgcctg      60

gactgcaagg ccagcggcat caccttcagc aacagcggca tgcactgggt gcgccaggcc     120

cccggcaagg gcctggagtg ggtggccgtg atctggtacg acggcagcaa gcgctactac     180

gccgacagcg tgaagggccg cttcaccatc agccgcgaca acagcaagaa caccctgttc     240

ctgcagatga acagcctgcg cgccgaggac accgccgtgt actactgcgc caccaacgac     300

gactactggg gccagggcac cctggtgacc gtgagcagcg cctccaccaa gggcccatcg     360

gtcttccccc tggcaccctc ctccaagagc acctctgggg gcacagcggc cctgggctgc     420

ctggtcaagg actacttccc cgaaccggtg acggtgtcgt ggaactcagg cgccctgacc     480

agcggcgtgc acaccttccc ggctgtccta cagtcctcag gactctactc cctcagcagc     540

gtggtgactg tgccctctag cagcttgggc acccagacct acatctgcaa cgtgaatcac     600

aagcccagca acaccaaggt ggacaagaaa gttgaaccca aatcttgcga caaaactcac     660

acatgcccac cgtgcccagc acctccagtc gccggaccgt cagtcttcct cttccctcca     720

aaacccaagg acaccctcat gatctcccgg acccctgagg tcacatgcgt ggtggtggac     780

gtgagccacg aagaccctga ggtcaagttc aactggtacg tggacggcgt ggaggtgcat     840

aatgccaaga caaagccgcg ggaggagcag tacaacagca cgtaccgtgt ggtcagcgtc     900

ctcaccgtcc tgcaccagga ctggctgaat ggcaaggagt acaagtgcaa ggtctccaac     960

aaaggcctcc caagctccat cgagaaaacc atctccaaag ccaaagggca gccccgagaa    1020

ccacaggtgt acaccctgcc tccatcccgg gatgagctga ccaagaacca ggtcagcctg    1080

acctgcctgg tcaaaggctt ctatcccagc gacatcgccg tggagtggga gagcaatggg    1140

cagccggaga acaactacaa gaccacgcct cccgtgctgg actccgacgg ctccttcttc    1200

ctctacagca agctcaccgt ggacaagagc aggtggcagc aggggaacgt cttctcatgc    1260

tccgtgatgc atgaggctct gcacaaccac tacacgcaga agagcctctc cctgtctccg    1320

ggtaaa                                                                1326

<210>  76
<211>  442
<212>  PRT
<213>  artificial sequence

<400>  76

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Ser Ser
        115                 120                 125

Lys Ser Thr Ser Gly Gly Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Gln
            180                 185                 190

Thr Tyr Ile Cys Asn Val Asn His Lys Pro Ser Asn Thr Lys Val Asp
        195                 200                 205

Lys Lys Val Glu Pro Lys Ser Cys Asp Lys Thr His Thr Cys Pro Pro
    210                 215                 220

Cys Pro Ala Pro Pro Val Ala Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Gly Leu Pro Ser Ser Ile Glu Lys Thr Ile Ser Lys Ala Lys Gly
                325                 330                 335

Gln Pro Arg Glu Pro Gln Val Tyr Thr Leu Pro Pro Ser Arg Asp Glu
            340                 345                 350

Leu Thr Lys Asn Gln Val Ser Leu Thr Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Tyr Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  77
<211>  642
<212>  DNA
<213>  artificial sequence

<400>  77
gagatcgtgc tgacccagag ccccgccacc ctgagcctga gccccggcga gcgcgccacc      60

ctgagctgcc gcgccagcca gagcgtgagc agctacctgg cctggtacca gcagaagcca     120

ggacaggctc cacgactgct aatctatgac gccagcaacc gcgccaccgg catccccgcc     180

cgcttcagcg gcagcggcag cggcaccgac ttcaccctga ccatcagcag cctggagccc     240

gaggacttcg ccgtgtacta ctgccagcag agcagcaact ggccccgcac cttcggccag     300

ggcaccaagg tggagatcaa gcgcacagtg gcagccccca gcgtcttcat tttcccccct      360

tccgatgaac agctgaagtc cggcactgct tctgtggtct gtctgctgaa caatttctat     420

cccagagagg ccaaggtgca gtggaaagtg gacaacgctc tgcagtccgg caacagccag     480

gagagtgtga ccgaacagga tagtaaggac agcacatatt ctctgtctag taccctgaca 540

ctgagtaagg cagattacga gaagcacaaa gtgtatgcct gcgaagtcac tcatcaggga 600

ctgtcaagcc ccgtgaccaa gagcttcaac cggggcgagt gt 642

<210> 78
<211> 214
<212> PRT
<213> artificial sequence

<400> 78

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser

195                 200                 205

Phe Asn Arg Gly Glu Cys
    210

<210>  79
<211>  1350
<212>  DNA
<213>  artificial sequence

<400>  79
gaagtacagc tgcttgagag tggaggaggt ttggtacagc ccggcggatc cctccgcctg      60

tcctgtgcgg ctagtggctt tacattctca tcctatatca tgatgtgggt aagacaggcc     120

ccaggaaagg gcctggagtg ggttagttct atctacccct caggcgggat taccttctac     180

gcagatactg tgaagggcag gtttaccata tcccgagaca acagtaagaa taccctttac     240

cttcaaatga actcccttcg ggccgaggac actgcggtgt actattgcgc tcgcattaag     300

cttggcaccg tgacaaccgt gaactattgg ggtcaaggca cgctggtgac tgtctcttcc     360

gcctccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgact gtgccctcta gcagcttggg cacccagacc     600

tacatctgca acgtgaatca caagcccagc aacaccaagg tggacaagaa agttgaaccc     660

aaatcttgcg acaaaactca cacatgccca ccgtgcccag cacctgaact cctgggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgggagga gcagtacaac     900

agcacgtacc gtgtggtcag cgtcctcacc gtcctgcacc aggactggct gaatggcaag     960

gagtacaagt gcaaggtctc caacaaagcc ctcccagccc ccatagagaa aaccatctcc    1020

aaagccaaag ggcagccccg agaaccacag gtgtacaccc tgcccccatc ccgggatgag    1080

ctgaccaaga accaggtcag cctgacctgc ctggtcaaag gcttctatcc cagcgacatc    1140

gccgtggagt gggagagcaa tgggcagccg gagaacaact acaagaccac gcctcccgtg    1200

ctggactccg acggctcctt cttcctctac agcaagctca ccgtggacaa gagcaggtgg    1260

cagcagggga acgtcttctc atgctccgtg atgcatgagg ctctgcacaa ccactacacg    1320

cagaagagcc tctccctgtc tccgggtaaa                                     1350

<210>  80
<211>  450
<212>  PRT

<213>  artificial sequence

<400>  80

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ile Met Met Trp Val Arg Gln Ala Pro Gly Lys Gly Leu Glu Trp Val
        35                  40                  45

Ser Ser Ile Tyr Pro Ser Gly Gly Ile Thr Phe Tyr Ala Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ile Lys Leu Gly Thr Val Thr Thr Val Asn Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  81
<211>  648
<212>  DNA
<213>  artificial sequence

<400>  81

caatccgcac tgactcaacc agccagcgtt agcggctccc ctggtcaatc tatcaccatc 60

agctgtaccg ggaccagctc agacgttggc ggttacaact acgtcagctg gtaccagcag 120

cacccgggta aagctccaaa gctgatgatt tatgatgtgt ctaatcgacc ttctggtgta 180

tctaaccgat tttcaggctc taaaagtgga aatactgctt ccctcacgat ctcagggctg 240

caagccgaag acgaagccga ttattattgt tctagctata catccagcag cacccgcgtg 300

tttggaacgg gaaccaaggt cacggttctg ggacagccca aagccaatcc taccgtcact 360

ctgttcccac ccagtagtga ggagctgcag gcaaataagg ctaccctggt ctgtcttata 420

tccgatttct atcccggggc agtcacagtc gcttggaagg cagatggctc tccagtgaag 480

gccggcgtcg aaacaactaa accttccaag cagtctaata acaagtacgc tgcttcttct 540

tacctttcac ttactcctga acaatggaag agccacagga gttactcttg tcaggtaacc 600

cacgaggggt ccactgtgga gaaaaccgtc gctcccacag agtgttct 648

<210> 82
<211> 216
<212> PRT
<213> artificial sequence

<400> 82

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Arg Val Phe Gly Thr Gly Thr Lys Val Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr

130                 135                 140

Pro Gly Ala Val Thr Val Ala Trp Lys Ala Asp Gly Ser Pro Val Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  83
<211>  1716
<212>  DNA
<213>  artificial sequence

<400>  83
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacgacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg caggtgcagc tggtggagag tggaggtggc     420

gtggtacagc ccggccgcag cctgcgcctg gactgcaagg ccagcggcat caccttcagc     480

aacagcggca tgcactgggt gcgccaggcc cccggcaagg gcctggagtg ggtggccgtg     540

atctggtacg acggcagcaa gcgctactac gccgacagcg tgaagggccg cttcaccatc     600

agccgcgaca acagcaagaa caccctgttc ctgcagatga acagcctgcg cgccgaggac     660

accgccgtgt actactgcgc caccaacgac gactactggg gccagggcac cctggtgacc     720

gtgagcagcg cctccaccaa gggcccatcg gtcttccccc tggcaccctc ctccaagagc     780

acctctgggg gcacagcggc cctgggctgc ctggtcaagg actacttccc cgaaccggtg     840

acggtgtcgt ggaactcagg cgccctgacc agcggcgtgc acaccttccc ggctgtccta     900

cagtcctcag gactctactc cctcagcagc gtggtgactg tgccctctag cagcttgggc     960

acccagacct acatctgcaa cgtgaatcac aagcccagca acaccaaggt ggacaagaaa    1020

gttgaaccca aatcttgcga caaaactcac acatgcccac cgtgcccagc acctccagtc 1080

gccggaccgt cagtcttcct cttccctcca aaacccaagg acaccctcat gatctcccgg 1140

acccctgagg tcacatgcgt ggtggtggac gtgagccacg aagaccctga ggtcaagttc 1200

aactggtacg tggacggcgt ggaggtgcat aatgccaaga caaagccgcg ggaggagcag 1260

tacaacagca cgtaccgtgt ggtcagcgtc ctcaccgtcc tgcaccagga ctggctgaat 1320

ggcaaggagt acaagtgcaa ggtctccaac aaaggcctcc caagctccat cgagaaaacc 1380

atctccaaag ccaaagggca gccccgagaa ccacaggtgt acaccctgcc tccatcccgg 1440

gatgagctga ccaagaacca ggtcagcctg acctgcctgg tcaaaggctt ctatcccagc 1500

gacatcgccg tggagtggga gagcaatggg cagccggaga acaactacaa gaccacgcct 1560

cccgtgctgg actccgacgg ctccttcttc ctctacagca agctcaccgt ggacaagagc 1620

aggtggcagc aggggaacgt cttctcatgc tccgtgatgc atgaggctct gcacaaccac 1680

tacacgcaga agagcctctc cctgtctccg ggtaaa 1716

<210> 84
<211> 572
<212> PRT
<213> artificial sequence

<400> 84

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Gln Val Gln Leu Val Glu Ser Gly Gly Gly Val Val Gln Pro
    130                 135                 140

Gly Arg Ser Leu Arg Leu Asp Cys Lys Ala Ser Gly Ile Thr Phe Ser
145                 150                 155                 160

Asn Ser Gly Met His Trp Val Arg Gln Ala Pro Gly Lys Gly Leu Glu
                165                 170                 175

Trp Val Ala Val Ile Trp Tyr Asp Gly Ser Lys Arg Tyr Tyr Ala Asp
            180                 185                 190

Ser Val Lys Gly Arg Phe Thr Ile Ser Arg Asp Asn Ser Lys Asn Thr
        195                 200                 205

Leu Phe Leu Gln Met Asn Ser Leu Arg Ala Glu Asp Thr Ala Val Tyr
    210                 215                 220

Tyr Cys Ala Thr Asn Asp Asp Tyr Trp Gly Gln Gly Thr Leu Val Thr
225                 230                 235                 240

Val Ser Ser Ala Ser Thr Lys Gly Pro Ser Val Phe Pro Leu Ala Pro
                245                 250                 255

Ser Ser Lys Ser Thr Ser Gly Gly Thr Ala Ala Leu Gly Cys Leu Val
            260                 265                 270

Lys Asp Tyr Phe Pro Glu Pro Val Thr Val Ser Trp Asn Ser Gly Ala
        275                 280                 285

Leu Thr Ser Gly Val His Thr Phe Pro Ala Val Leu Gln Ser Ser Gly
    290                 295                 300

Leu Tyr Ser Leu Ser Ser Val Val Thr Val Pro Ser Ser Ser Leu Gly
305                 310                 315                 320

Thr Gln Thr Tyr Ile Cys Asn Val Asn His Lys Pro Ser Asn Thr Lys
                325                 330                 335

Val Asp Lys Lys Val Glu Pro Lys Ser Cys Asp Lys Thr His Thr Cys
            340                 345                 350

Pro Pro Cys Pro Ala Pro Pro Val Ala Gly Pro Ser Val Phe Leu Phe
        355                 360                 365

Pro Pro Lys Pro Lys Asp Thr Leu Met Ile Ser Arg Thr Pro Glu Val
    370                 375                 380

Thr Cys Val Val Val Asp Val Ser His Glu Asp Pro Glu Val Lys Phe
385                 390                 395                 400

Asn Trp Tyr Val Asp Gly Val Glu Val His Asn Ala Lys Thr Lys Pro
                405                 410                 415

Arg Glu Glu Gln Tyr Asn Ser Thr Tyr Arg Val Val Ser Val Leu Thr
            420                 425                 430

Val Leu His Gln Asp Trp Leu Asn Gly Lys Glu Tyr Lys Cys Lys Val
        435                 440                 445

Ser Asn Lys Gly Leu Pro Ser Ser Ile Glu Lys Thr Ile Ser Lys Ala
    450                 455                 460

Lys Gly Gln Pro Arg Glu Pro Gln Val Tyr Thr Leu Pro Pro Ser Arg
465                 470                 475                 480

Asp Glu Leu Thr Lys Asn Gln Val Ser Leu Thr Cys Leu Val Lys Gly
                485                 490                 495

Phe Tyr Pro Ser Asp Ile Ala Val Glu Trp Glu Ser Asn Gly Gln Pro
            500                 505                 510

Glu Asn Asn Tyr Lys Thr Thr Pro Pro Val Leu Asp Ser Asp Gly Ser
        515                 520                 525

Phe Phe Leu Tyr Ser Lys Leu Thr Val Asp Lys Ser Arg Trp Gln Gln
    530                 535                 540

Gly Asn Val Phe Ser Cys Ser Val Met His Glu Ala Leu His Asn His
545                 550                 555                 560

Tyr Thr Gln Lys Ser Leu Ser Leu Ser Pro Gly Lys
                565                 570

<210>  85
<211>  885
<212>  DNA
<213>  artificial sequence

<400>  85
atcacctgcc cacctcccat gagcgtggag cacgccgaca tctgggtgaa gagctacagc      60

ctgtacagcc gcgagcgcta catctgcaac agcggcttca agcgcaaggc cggcaccagc     120

agcctgaccg agtgcgtgct gaacaaggcc accaacgtgg cccactggac cacccccagc     180

ctgaagtgca tccgcggcgg aggcggatcc ggaggcggag gttccggcgg gggtgggagc     240

ggggagatcg tgctgaccca gagccccgcc accctgagcc tgagccccgg cgagcgcgcc 300

accctgagct gccgcgccag ccagagcgtg agcagctacc tggcctggta ccagcagaag 360

ccaggacagg ctccacgact gctaatctat gacgccagca accgcgccac cggcatcccc 420

gcccgcttca gcggcagcgg cagcggcacc gacttcaccc tgaccatcag cagcctggag 480

cccgaggact tcgccgtgta ctactgccag cagagcagca actggccccg caccttcggc 540

cagggcacca aggtggagat caagcgcaca gtggcagccc ccagcgtctt catttttccc 600

ccttccgatg aacagctgaa gtccggcact gcttctgtgg tctgtctgct gaacaatttc 660

tatcccagag aggccaaggt gcagtggaaa gtggacaacg ctctgcagtc cggcaacagc 720

caggagagtg tgaccgaaca ggatagtaag gacagcacat attctctgtc tagtaccctg 780

acactgagta aggcagatta cgagaagcac aaagtgtatg cctgcgaagt cactcatcag 840

ggactgtcaa gccccgtgac caagagcttc aaccggggcg agtgt 885

<210> 86
<211> 295
<212> PRT
<213> artificial sequence

<400> 86

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Glu Ile Val Leu Thr Gln Ser Pro Ala Thr Leu Ser Leu Ser Pro
                85                  90                  95

Gly Glu Arg Ala Thr Leu Ser Cys Arg Ala Ser Gln Ser Val Ser Ser
            100                 105                 110

Tyr Leu Ala Trp Tyr Gln Gln Lys Pro Gly Gln Ala Pro Arg Leu Leu
        115                 120                 125

Ile Tyr Asp Ala Ser Asn Arg Ala Thr Gly Ile Pro Ala Arg Phe Ser
    130                 135                 140

Gly Ser Gly Ser Gly Thr Asp Phe Thr Leu Thr Ile Ser Ser Leu Glu
145                 150                 155                 160

Pro Glu Asp Phe Ala Val Tyr Tyr Cys Gln Gln Ser Ser Asn Trp Pro
                165                 170                 175

Arg Thr Phe Gly Gln Gly Thr Lys Val Glu Ile Lys Arg Thr Val Ala
            180                 185                 190

Ala Pro Ser Val Phe Ile Phe Pro Pro Ser Asp Glu Gln Leu Lys Ser
        195                 200                 205

Gly Thr Ala Ser Val Val Cys Leu Leu Asn Asn Phe Tyr Pro Arg Glu
    210                 215                 220

Ala Lys Val Gln Trp Lys Val Asp Asn Ala Leu Gln Ser Gly Asn Ser
225                 230                 235                 240

Gln Glu Ser Val Thr Glu Gln Asp Ser Lys Asp Ser Thr Tyr Ser Leu
                245                 250                 255

Ser Ser Thr Leu Thr Leu Ser Lys Ala Asp Tyr Glu Lys His Lys Val
            260                 265                 270

Tyr Ala Cys Glu Val Thr His Gln Gly Leu Ser Ser Pro Val Thr Lys
        275                 280                 285

Ser Phe Asn Arg Gly Glu Cys
    290                 295

<210>  87
<211>  711
<212>  DNA
<213>  artificial sequence

<400>  87
atcacgtgcc ctccccccat gtccgtggaa cacgcagaca tctgggtcaa gagctacagc      60

ttgtactcca gggagcggta catttgtaac tctggtttca agcgtaaagc cggcacgtcc     120

agcctgacgg agtgcgtgtt gaacaaggcc acgaatgtcg cccactggac aacccccagt     180

ctcaaatgca ttagagaccc tgccctggtt caccaaaggc cagcgccacc ctccacagta     240

acgacggcag gggtgacccc acagccagag agcctctccc cttctggaaa agagcccgca     300

gcttcatctc ccagctcaaa caacacagcg gccacaacag cagctattgt cccgggctcc     360

cagctgatgc cttcaaaatc accttccaca ggaaccacag agataagcag tcatgagtcc     420

tcccacggca ccccctctca gacaacagcc aagaactggg aactcacagc atccgcctcc 480

caccagccgc caggtgtgta tccacagggc cacagcgaca ccactgtggc tatctccacg 540

tccactgtcc tgctgtgtgg gctgagcgct gtgtctctcc tggcatgcta cctcaagtca 600

aggcaaactc ccccgctggc cagcgttgaa atggaagcca tggaggctct gccggtgact 660

tgggggacca gcagcagaga tgaagacttg gaaaactgct ctcaccacct a 711

<210> 88
<211> 237
<212> PRT
<213> artificial sequence

<400> 88

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Asp Pro Ala Leu Val His Gln Arg Pro Ala Pro Pro Ser Thr Val
65                  70                  75                  80

Thr Thr Ala Gly Val Thr Pro Gln Pro Glu Ser Leu Ser Pro Ser Gly
                85                  90                  95

Lys Glu Pro Ala Ala Ser Ser Pro Ser Ser Asn Asn Thr Ala Ala Thr
            100                 105                 110

Thr Ala Ala Ile Val Pro Gly Ser Gln Leu Met Pro Ser Lys Ser Pro
        115                 120                 125

Ser Thr Gly Thr Thr Glu Ile Ser Ser His Glu Ser Ser His Gly Thr
    130                 135                 140

Pro Ser Gln Thr Thr Ala Lys Asn Trp Glu Leu Thr Ala Ser Ala Ser
145                 150                 155                 160

His Gln Pro Pro Gly Val Tyr Pro Gln Gly His Ser Asp Thr Thr Val
                165                 170                 175

Ala Ile Ser Thr Ser Thr Val Leu Leu Cys Gly Leu Ser Ala Val Ser
            180                 185                 190

Leu Leu Ala Cys Tyr Leu Lys Ser Arg Gln Thr Pro Pro Leu Ala Ser
        195                 200                 205

Val Glu Met Glu Ala Met Glu Ala Leu Pro Val Thr Trp Gly Thr Ser
    210                 215                 220

Ser Arg Asp Glu Asp Leu Glu Asn Cys Ser His His Leu
225                 230                 235

<210>  89
<211>  1737
<212>  DNA
<213>  artificial sequence

<400>  89
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacaacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccggt     360

cctctgggag tacgaggcgg gggtgggagc gggcaggtgc agctgcagga gagcggcccc     420

ggcctggtga agcccagcga gaccctgagc ctgacctgca ccgtgagcgg cggcagcgtg     480

agcagcggcg actactactg gacctggatt cgccagagcc ccggcaaggg cctggagtgg     540

atcggccaca tctactacag cggcaacacc aactacaacc ccagcctgaa gagccgcctg     600

accatcagca tcgacaccag caagacccag ttcagcctga agctgagcag cgtgaccgcc     660

gccgacaccg ccatctacta ctgcgtgcgc gaccgcgtga ccggcgcctt cgacatctgg     720

ggccagggca ccatggtgac tgtgtctagc gcctccacca agggcccatc ggtcttcccc     780

ctggcaccct cctccaagag cacctctggg ggcacagcgg ccctgggctg cctggtcaag     840

gactacttcc ccgaaccggt gacggtgtcg tggaactcag gcgccctgac cagcggcgtg     900

cacaccttcc cggctgtcct acagtcctca ggactctact ccctcagcag cgtggtgact     960

gtgccctcta gcagcttggg cacccagacc tacatctgca acgtgaatca caagcccagc    1020

aacaccaagg tggacaagaa agttgaaccc aaatcttgcg acaaaactca cacatgccca    1080

ccgtgcccag cacctccagt cgccggaccg tcagtcttcc tcttccctcc aaaacccaag    1140

gacaccctca tgatctcccg gacccctgag gtcacatgcg tggtggtgga cgtgagccac    1200

gaagaccctg aggtcaagtt caactggtac gtggacggcg tggaggtgca taatgccaag    1260

acaaagccgc gggaggagca gtacaacagc acgtaccgtg tggtcagcgt cctcaccgtc 1320

ctgcaccagg actggctgaa tggcaaggag tacaagtgca aggtctccaa caaaggcctc 1380

ccaagctcca tcgagaaaac catctccaaa gccaaagggc agccccgaga accacaggtg 1440

tacaccctgc ctccatcccg ggatgagctg accaagaacc aggtcagcct gacctgcctg 1500

gtcaaaggct tctatcccag cgacatcgcc gtggagtggg agagcaatgg gcagccggag 1560

aacaactaca agaccacgcc tcccgtgctg gactccgacg gctccttctt cctctacagc 1620

aagctcaccg tggacaagag caggtggcag caggggaacg tcttctcatg ctccgtgatg 1680

catgaggctc tgcacaacca ctacacgcag aagagcctct ccctgtctcc gggtaaa 1737

<210> 90
<211> 579
<212> PRT
<213> artificial sequence

<400> 90

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Pro Leu Gly Val Arg Gly Gly Gly
        115                 120                 125

Gly Ser Gly Gln Val Gln Leu Gln Glu Ser Gly Pro Gly Leu Val Lys
    130                 135                 140

Pro Ser Glu Thr Leu Ser Leu Thr Cys Thr Val Ser Gly Gly Ser Val
145                 150                 155                 160

Ser Ser Gly Asp Tyr Tyr Trp Thr Trp Ile Arg Gln Ser Pro Gly Lys
                165                 170                 175

Gly Leu Glu Trp Ile Gly His Ile Tyr Tyr Ser Gly Asn Thr Asn Tyr
            180                 185                 190

Asn Pro Ser Leu Lys Ser Arg Leu Thr Ile Ser Ile Asp Thr Ser Lys
        195                 200                 205

Thr Gln Phe Ser Leu Lys Leu Ser Ser Val Thr Ala Ala Asp Thr Ala
    210                 215                 220

Ile Tyr Tyr Cys Val Arg Asp Arg Val Thr Gly Ala Phe Asp Ile Trp
225                 230                 235                 240

Gly Gln Gly Thr Met Val Thr Val Ser Ser Ala Ser Thr Lys Gly Pro
                245                 250                 255

Ser Val Phe Pro Leu Ala Pro Ser Ser Lys Ser Thr Ser Gly Gly Thr
            260                 265                 270

Ala Ala Leu Gly Cys Leu Val Lys Asp Tyr Phe Pro Glu Pro Val Thr
        275                 280                 285

Val Ser Trp Asn Ser Gly Ala Leu Thr Ser Gly Val His Thr Phe Pro
    290                 295                 300

Ala Val Leu Gln Ser Ser Gly Leu Tyr Ser Leu Ser Ser Val Val Thr
305                 310                 315                 320

Val Pro Ser Ser Ser Leu Gly Thr Gln Thr Tyr Ile Cys Asn Val Asn
                325                 330                 335

His Lys Pro Ser Asn Thr Lys Val Asp Lys Lys Val Glu Pro Lys Ser
            340                 345                 350

Cys Asp Lys Thr His Thr Cys Pro Pro Cys Pro Ala Pro Pro Val Ala
        355                 360                 365

Gly Pro Ser Val Phe Leu Phe Pro Pro Lys Pro Lys Asp Thr Leu Met
    370                 375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val

405                 410                 415

His Asn Ala Lys Thr Lys Pro Arg Glu Glu Gln Tyr Asn Ser Thr Tyr
            420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Gly Leu Pro Ser Ser Ile
    450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Asp Glu Leu Thr Lys Asn Gln Val Ser
                485                 490                 495

Leu Thr Cys Leu Val Lys Gly Phe Tyr Pro Ser Asp Ile Ala Val Glu
            500                 505                 510

Trp Glu Ser Asn Gly Gln Pro Glu Asn Asn Tyr Lys Thr Thr Pro Pro
        515                 520                 525

Val Leu Asp Ser Asp Gly Ser Phe Phe Leu Tyr Ser Lys Leu Thr Val
    530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys

<210>  91
<211>  1719
<212>  DNA
<213>  artificial sequence

<400>  91
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacaacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccggt 360

cctctgggag tacgacaggt gcagctgcag gagagcggcc ccggcctggt gaagcccagc 420

gagaccctga gcctgacctg caccgtgagc ggcggcagcg tgagcagcgg cgactactac 480

tggacctgga ttcgccagag ccccggcaag ggcctggagt ggatcggcca catctactac 540

agcggcaaca ccaactacaa ccccagcctg aagagccgcc tgaccatcag catcgacacc 600

agcaagaccc agttcagcct gaagctgagc agcgtgaccg ccgccgacac cgccatctac 660

tactgcgtgc gcgaccgcgt gaccggcgcc ttcgacatct ggggccaggg caccatggtg 720

actgtgtcta gcgcctccac caagggccca tcggtcttcc ccctggcacc ctcctccaag 780

agcacctctg ggggcacagc ggccctgggc tgcctggtca aggactactt ccccgaaccg 840

gtgacggtgt cgtggaactc aggcgccctg accagcggcg tgcacacctt cccggctgtc 900

ctacagtcct caggactcta ctccctcagc agcgtggtga ctgtgccctc tagcagcttg 960

ggcacccaga cctacatctg caacgtgaat cacaagccca gcaacaccaa ggtggacaag 1020

aaagttgaac ccaaatcttg cgacaaaact cacacatgcc caccgtgccc agcacctcca 1080

gtcgccggac cgtcagtctt cctcttccct ccaaaaccca aggacaccct catgatctcc 1140

cggacccctg aggtcacatg cgtggtggtg gacgtgagcc acgaagaccc tgaggtcaag 1200

ttcaactggt acgtggacgg cgtggaggtg cataatgcca agacaaagcc gcgggaggag 1260

cagtacaaca gcacgtaccg tgtggtcagc gtcctcaccg tcctgcacca ggactggctg 1320

aatggcaagg agtacaagtg caaggtctcc aacaaaggcc tcccaagctc catcgagaaa 1380

accatctcca aagccaaagg gcagccccga gaaccacagg tgtacaccct gcctccatcc 1440

cgggatgagc tgaccaagaa ccaggtcagc ctgacctgcc tggtcaaagg cttctatccc 1500

agcgacatcg ccgtggagtg ggagagcaat gggcagccgg agaacaacta caagaccacg 1560

cctcccgtgc tggactccga cggctccttc ttcctctaca gcaagctcac cgtggacaag 1620

agcaggtggc agcaggggaa cgtcttctca tgctccgtga tgcatgaggc tctgcacaac 1680

cactacacgc agaagagcct ctccctgtct ccgggtaaa 1719

<210> 92
<211> 573
<212> PRT
<213> artificial sequence

<400> 92

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Pro Leu Gly Val Arg Gln Val Gln
        115                 120                 125

Leu Gln Glu Ser Gly Pro Gly Leu Val Lys Pro Ser Glu Thr Leu Ser
    130                 135                 140

Leu Thr Cys Thr Val Ser Gly Gly Ser Val Ser Ser Gly Asp Tyr Tyr
145                 150                 155                 160

Trp Thr Trp Ile Arg Gln Ser Pro Gly Lys Gly Leu Glu Trp Ile Gly
                165                 170                 175

His Ile Tyr Tyr Ser Gly Asn Thr Asn Tyr Asn Pro Ser Leu Lys Ser
            180                 185                 190

Arg Leu Thr Ile Ser Ile Asp Thr Ser Lys Thr Gln Phe Ser Leu Lys
        195                 200                 205

Leu Ser Ser Val Thr Ala Ala Asp Thr Ala Ile Tyr Tyr Cys Val Arg
    210                 215                 220

Asp Arg Val Thr Gly Ala Phe Asp Ile Trp Gly Gln Gly Thr Met Val
225                 230                 235                 240

Thr Val Ser Ser Ala Ser Thr Lys Gly Pro Ser Val Phe Pro Leu Ala
                245                 250                 255

Pro Ser Ser Lys Ser Thr Ser Gly Gly Thr Ala Ala Leu Gly Cys Leu
            260                 265                 270

Val Lys Asp Tyr Phe Pro Glu Pro Val Thr Val Ser Trp Asn Ser Gly
        275                 280                 285

Ala Leu Thr Ser Gly Val His Thr Phe Pro Ala Val Leu Gln Ser Ser
    290                 295                 300

Gly Leu Tyr Ser Leu Ser Ser Val Val Thr Val Pro Ser Ser Ser Leu
305                 310                 315                 320

Gly Thr Gln Thr Tyr Ile Cys Asn Val Asn His Lys Pro Ser Asn Thr
                325                 330                 335

Lys Val Asp Lys Lys Val Glu Pro Lys Ser Cys Asp Lys Thr His Thr
            340                 345                 350

Cys Pro Pro Cys Pro Ala Pro Pro Val Ala Gly Pro Ser Val Phe Leu
        355                 360                 365

Phe Pro Pro Lys Pro Lys Asp Thr Leu Met Ile Ser Arg Thr Pro Glu
    370                 375                 380

Val Thr Cys Val Val Val Asp Val Ser His Glu Asp Pro Glu Val Lys
385                 390                 395                 400

Phe Asn Trp Tyr Val Asp Gly Val Glu Val His Asn Ala Lys Thr Lys
                405                 410                 415

Pro Arg Glu Glu Gln Tyr Asn Ser Thr Tyr Arg Val Val Ser Val Leu
            420                 425                 430

Thr Val Leu His Gln Asp Trp Leu Asn Gly Lys Glu Tyr Lys Cys Lys
        435                 440                 445

Val Ser Asn Lys Gly Leu Pro Ser Ser Ile Glu Lys Thr Ile Ser Lys
    450                 455                 460

Ala Lys Gly Gln Pro Arg Glu Pro Gln Val Tyr Thr Leu Pro Pro Ser
465                 470                 475                 480

Arg Asp Glu Leu Thr Lys Asn Gln Val Ser Leu Thr Cys Leu Val Lys
                485                 490                 495

Gly Phe Tyr Pro Ser Asp Ile Ala Val Glu Trp Glu Ser Asn Gly Gln
            500                 505                 510

Pro Glu Asn Asn Tyr Lys Thr Thr Pro Pro Val Leu Asp Ser Asp Gly
        515                 520                 525

Ser Phe Phe Leu Tyr Ser Lys Leu Thr Val Asp Lys Ser Arg Trp Gln

530                 535                 540

Gln Gly Asn Val Phe Ser Cys Ser Val Met His Glu Ala Leu His Asn
545                 550                 555                 560

His Tyr Thr Gln Lys Ser Leu Ser Leu Ser Pro Gly Lys
                565                 570

<210>  93
<211>  1737
<212>  DNA
<213>  artificial sequence

<400>  93
caggtgcagc tgcaggagag cggccccggc ctggtgaagc ccagcgagac cctgagcctg      60

acctgcaccg tgagcggcgg cagcgtgagc agcggcgact actactggac ctggattcgc     120

cagagccccg gcaagggcct ggagtggatc ggccacatct actacagcgg caacaccaac     180

tacaacccca gcctgaagag ccgcctgacc atcagcatcg acaccagcaa gacccagttc     240

agcctgaagc tgagcagcgt gaccgccgcc gacaccgcca tctactactg cgtgcgcgac     300

cgcgtgaccg gcgccttcga catctggggc cagggcacca tggtgactgt gtctagcgcc     360

tccaccaagg gcccatcggt cttccccctg gcaccctcct ccaagagcac ctctgggggc     420

acagcggccc tgggctgcct ggtcaaggac tacttccccg aaccggtgac ggtgtcgtgg     480

aactcaggcg ccctgaccag cggcgtgcac accttcccgg ctgtcctaca gtcctcagga     540

ctctactccc tcagcagcgt ggtgactgtg ccctctagca gcttgggcac ccagacctac     600

atctgcaacg tgaatcacaa gcccagcaac accaaggtgg acaagaaagt tgaacccaaa     660

tcttgcgaca aaactcacac atgcccaccg tgcccagcac ctccagtcgc cggaccgtca     720

gtcttcctct tccctccaaa acccaaggac accctcatga tctcccggac ccctgaggtc     780

acatgcgtgg tggtggacgt gagccacgaa gaccctgagg tcaagttcaa ctggtacgtg     840

gacggcgtgg aggtgcataa tgccaagaca aagccgcggg aggagcagta caacagcacg     900

taccgtgtgg tcagcgtcct caccgtcctg caccaggact ggctgaatgg caaggagtac     960

aagtgcaagg tctccaacaa aggcctccca agctccatcg agaaaaccat ctccaaagcc    1020

aaagggcagc cccgagaacc acaggtgtac accctgcctc catcccggga tgagctgacc    1080

aagaaccagg tcagcctgac ctgcctggtc aaaggcttct atcccagcga catcgccgtg    1140

gagtgggaga gcaatgggca gccggagaac aactacaaga ccacgcctcc cgtgctggac    1200

tccgacggct ccttcttcct ctacagcaag ctcaccgtgg acaagagcag gtggcagcag    1260

gggaacgtct tctcatgctc cgtgatgcat gaggctctgc acaaccacta cacgcagaag    1320

agcctctccc tgtctccggg taaaggcgga ggcggatccg gtcctctggg agtacgaggc    1380

gggggtggga gcgggaactg ggtgaacgtg atcagcgacc tgaagaagat cgaggacctg 1440

atccagagca tgcacatcga cgccaccctg tacaccgaga gcgacgtgca ccccagctgc 1500

aaggtgaccg ccatgaagtg cttcctgctg gagctgcagg tgatcagcct ggagagcggc 1560

gacgccagca tccacgacac cgtggagaac ctgatcatcc tggccaacaa cagcctgagc 1620

agcaacggca acgtgaccga gagcggctgc aaggagtgcg aggagctgga ggagaagaac 1680

atcaaggagt tcctgcagag cttcgtgcac atcgtgcaga tgttcatcaa caccagc 1737

<210> 94
<211> 579
<212> PRT
<213> artificial sequence

<400> 94

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Asp Tyr Tyr Trp Thr Trp Ile Arg Gln Ser Pro Gly Lys Gly Leu Glu
        35                  40                  45

Trp Ile Gly His Ile Tyr Tyr Ser Gly Asn Thr Asn Tyr Asn Pro Ser
    50                  55                  60

Leu Lys Ser Arg Leu Thr Ile Ser Ile Asp Thr Ser Lys Thr Gln Phe
65                  70                  75                  80

Ser Leu Lys Leu Ser Ser Val Thr Ala Ala Asp Thr Ala Ile Tyr Tyr
                85                  90                  95

Cys Val Arg Asp Arg Val Thr Gly Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
        180                 185                 190

Ser Ser Leu Gly Thr Gln Thr Tyr Ile Cys Asn Val Asn His Lys Pro
    195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
        260                 265                 270

Glu Val Lys Phe Asn Trp Tyr Val Asp Gly Val Glu Val His Asn Ala
    275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
        340                 345                 350

Pro Pro Ser Arg Asp Glu Leu Thr Lys Asn Gln Val Ser Leu Thr Cys
    355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
        420                 425                 430

Leu His Asn His Tyr Thr Gln Lys Ser Leu Ser Leu Ser Pro Gly Lys
        435                 440                 445

Gly Gly Gly Gly Ser Gly Pro Leu Gly Val Arg Gly Gly Gly Gly Ser
    450                 455                 460

Gly Asn Trp Val Asn Val Ile Ser Asp Leu Lys Lys Ile Glu Asp Leu
465                 470                 475                 480

Ile Gln Ser Met His Ile Asp Ala Thr Leu Tyr Thr Glu Ser Asp Val
                485                 490                 495

His Pro Ser Cys Lys Val Thr Ala Met Lys Cys Phe Leu Leu Glu Leu
            500                 505                 510

Gln Val Ile Ser Leu Glu Ser Gly Asp Ala Ser Ile His Asp Thr Val
        515                 520                 525

Glu Asn Leu Ile Ile Leu Ala Asn Asn Ser Leu Ser Ser Asn Gly Asn
    530                 535                 540

Val Thr Glu Ser Gly Cys Lys Glu Cys Glu Glu Leu Glu Glu Lys Asn
545                 550                 555                 560

Ile Lys Glu Phe Leu Gln Ser Phe Val His Ile Val Gln Met Phe Ile
                565                 570                 575

Asn Thr Ser

<210>  95
<211>  1722
<212>  DNA
<213>  artificial sequence

<400>  95
caggtgcagc tgcaggagag cggccccggc ctggtgaagc ccagcgagac cctgagcctg      60

acctgcaccg tgagcggcgg cagcgtgagc agcggcgact actactggac ctggattcgc     120

cagagccccg gcaagggcct ggagtggatc ggccacatct actacagcgg caacaccaac     180

tacaacccca gcctgaagag ccgcctgacc atcagcatcg acaccagcaa gacccagttc     240

agcctgaagc tgagcagcgt gaccgccgcc gacaccgcca tctactactg cgtgcgcgac     300

cgcgtgaccg gcgccttcga catctggggc cagggcacca tggtgactgt gtctagcgcc     360

tccaccaagg gcccatcggt cttccccctg gcaccctcct ccaagagcac ctctgggggc     420

acagcggccc tgggctgcct ggtcaaggac tacttccccg aaccggtgac ggtgtcgtgg     480

aactcaggcg ccctgaccag cggcgtgcac accttcccgg ctgtcctaca gtcctcagga 540

ctctactccc tcagcagcgt ggtgactgtg ccctctagca gcttgggcac ccagacctac 600

atctgcaacg tgaatcacaa gcccagcaac accaaggtgg acaagaaagt tgaacccaaa 660

tcttgcgaca aaactcacac atgcccaccg tgcccagcac ctccagtcgc cggaccgtca 720

gtcttcctct tccctccaaa acccaaggac accctcatga tctcccggac ccctgaggtc 780

acatgcgtgg tggtggacgt gagccacgaa gaccctgagg tcaagttcaa ctggtacgtg 840

gacggcgtgg aggtgcataa tgccaagaca aagccgcggg aggagcagta caacagcacg 900

taccgtgtgg tcagcgtcct caccgtcctg caccaggact ggctgaatgg caaggagtac 960

aagtgcaagg tctccaacaa aggcctccca agctccatcg agaaaaccat ctccaaagcc 1020

aaagggcagc cccgagaacc acaggtgtac accctgcctc catcccggga tgagctgacc 1080

aagaaccagg tcagcctgac ctgcctggtc aaaggcttct atcccagcga catcgccgtg 1140

gagtgggaga gcaatgggca gccggagaac aactacaaga ccacgcctcc cgtgctggac 1200

tccgacggct ccttcttcct ctacagcaag ctcaccgtgg acaagagcag gtggcagcag 1260

gggaacgtct tctcatgctc cgtgatgcat gaggctctgc acaaccacta cacgcagaag 1320

agcctctccc tgtctccggg taaaggtcct ctgggagtac gaggcggggg tgggagcggg 1380

aactgggtga acgtgatcag cgacctgaag aagatcgagg acctgatcca gagcatgcac 1440

atcgacgcca ccctgtacac cgagagcgac gtgcacccca gctgcaaggt gaccgccatg 1500

aagtgcttcc tgctggagct gcaggtgatc agcctggaga gcggcgacgc cagcatccac 1560

gacaccgtgg agaacctgat catcctggcc aacaacagcc tgagcagcaa cggcaacgtg 1620

accgagagcg gctgcaagga gtgcgaggag ctggaggaga agaacatcaa ggagttcctg 1680

cagagcttcg tgcacatcgt gcagatgttc atcaacacca gc 1722

<210> 96
<211> 574
<212> PRT
<213> artificial sequence

<400> 96

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Asp Tyr Tyr Trp Thr Trp Ile Arg Gln Ser Pro Gly Lys Gly Leu Glu
        35                  40                  45

Trp Ile Gly His Ile Tyr Tyr Ser Gly Asn Thr Asn Tyr Asn Pro Ser
    50                  55                  60

Leu Lys Ser Arg Leu Thr Ile Ser Ile Asp Thr Ser Lys Thr Gln Phe
65                  70                  75                  80

Ser Leu Lys Leu Ser Ser Val Thr Ala Ala Asp Thr Ala Ile Tyr Tyr
                85                  90                  95

Cys Val Arg Asp Arg Val Thr Gly Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

Gly Pro Leu Gly Val Arg Gly Gly Gly Gly Ser Gly Asn Trp Val Asn
    450                 455                 460

Val Ile Ser Asp Leu Lys Lys Ile Glu Asp Leu Ile Gln Ser Met His
465                 470                 475                 480

Ile Asp Ala Thr Leu Tyr Thr Glu Ser Asp Val His Pro Ser Cys Lys
                485                 490                 495

Val Thr Ala Met Lys Cys Phe Leu Leu Glu Leu Gln Val Ile Ser Leu
            500                 505                 510

Glu Ser Gly Asp Ala Ser Ile His Asp Thr Val Glu Asn Leu Ile Ile
        515                 520                 525

Leu Ala Asn Asn Ser Leu Ser Ser Asn Gly Asn Val Thr Glu Ser Gly
    530                 535                 540

Cys Lys Glu Cys Glu Glu Leu Glu Glu Lys Asn Ile Lys Glu Phe Leu
545                 550                 555                 560

Gln Ser Phe Val His Ile Val Gln Met Phe Ile Asn Thr Ser
                565                 570

<210>  97
<211>  1740
<212>  DNA
<213>  artificial sequence

<400>  97
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacaacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccggt     360

cctctgggag tacgaggcgg gggtgggagc ggggaggtgc agctggtgga gtctggagga     420

ggcttggtcc agcctggggg gtccctgaga ctctcctgtg cagcctctgg gttcaatatt     480

aaggacactt acatccactg ggtccgccag gctccaggga aggggctgga gtgggtcgca     540

cgtatttatc ctaccaatgg ttacacacgc tacgcagact ccgtgaaggg ccgattcacc     600

atctccgcag acacttccaa gaacacggcg tatcttcaaa tgaacagcct gagagccgag     660

gacacggccg tgtattactg ttcgagatgg ggcggtgacg gcttctatgc catggactac     720

tggggccaag gaaccctggt caccgtctcc tcagcctcca ccaagggccc atcggtcttc     780

cccctggcac cctcctccaa gagcacctct gggggcacag cggccctggg ctgcctggtc     840

aaggactact tccccgaacc ggtgacggtg tcgtggaact caggcgccct gaccagcggc     900

gtgcacacct tcccggctgt cctacagtcc tcaggactct actccctcag cagcgtggtg     960

actgtgccct ctagcagctt gggcacccag acctacatct gcaacgtgaa tcacaagccc    1020

agcaacacca aggtggacaa gaaagttgaa cccaaatctt gcgacaaaac tcacacatgc    1080

ccaccgtgcc cagcacctcc agtcgccgga ccgtcagtct tcctcttccc tccaaaaccc    1140

aaggacaccc tcatgatctc ccggacccct gaggtcacat gcgtggtggt ggacgtgagc    1200

cacgaagacc ctgaggtcaa gttcaactgg tacgtggacg gcgtggaggt gcataatgcc    1260

aagacaaagc cgcgggagga gcagtacaac agcacgtacc gtgtggtcag cgtcctcacc    1320

gtcctgcacc aggactggct gaatggcaag gagtacaagt gcaaggtctc caacaaaggc    1380

ctcccaagct ccatcgagaa aaccatctcc aaagccaaag ggcagccccg agaaccacag    1440

gtgtacaccc tgcctccatc ccgggatgag ctgaccaaga accaggtcag cctgacctgc    1500

ctggtcaaag gcttctatcc cagcgacatc gccgtggagt gggagagcaa tgggcagccg    1560

gagaacaact acaagaccac gcctcccgtg ctggactccg acggctcctt cttcctctac 1620

agcaagctca ccgtggacaa gagcaggtgg cagcagggga acgtcttctc atgctccgtg 1680

atgcatgagg ctctgcacaa ccactacacg cagaagagcc tctccctgtc tccgggtaaa 1740

<210> 98
<211> 580
<212> PRT
<213> artificial sequence

<400> 98

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Pro Leu Gly Val Arg Gly Gly Gly
        115                 120                 125

Gly Ser Gly Glu Val Gln Leu Val Glu Ser Gly Gly Gly Leu Val Gln
    130                 135                 140

Pro Gly Gly Ser Leu Arg Leu Ser Cys Ala Ala Ser Gly Phe Asn Ile
145                 150                 155                 160

Lys Asp Thr Tyr Ile His Trp Val Arg Gln Ala Pro Gly Lys Gly Leu
                165                 170                 175

Glu Trp Val Ala Arg Ile Tyr Pro Thr Asn Gly Tyr Thr Arg Tyr Ala
            180                 185                 190

Asp Ser Val Lys Gly Arg Phe Thr Ile Ser Ala Asp Thr Ser Lys Asn
        195                 200                 205

Thr Ala Tyr Leu Gln Met Asn Ser Leu Arg Ala Glu Asp Thr Ala Val
    210                 215                 220

Tyr Tyr Cys Ser Arg Trp Gly Gly Asp Gly Phe Tyr Ala Met Asp Tyr
225                 230                 235                 240

Trp Gly Gln Gly Thr Leu Val Thr Val Ser Ser Ala Ser Thr Lys Gly
                245                 250                 255

Pro Ser Val Phe Pro Leu Ala Pro Ser Ser Lys Ser Thr Ser Gly Gly
            260                 265                 270

Thr Ala Ala Leu Gly Cys Leu Val Lys Asp Tyr Phe Pro Glu Pro Val
        275                 280                 285

Thr Val Ser Trp Asn Ser Gly Ala Leu Thr Ser Gly Val His Thr Phe
    290                 295                 300

Pro Ala Val Leu Gln Ser Ser Gly Leu Tyr Ser Leu Ser Ser Val Val
305                 310                 315                 320

Thr Val Pro Ser Ser Ser Leu Gly Thr Gln Thr Tyr Ile Cys Asn Val
                325                 330                 335

Asn His Lys Pro Ser Asn Thr Lys Val Asp Lys Lys Val Glu Pro Lys
            340                 345                 350

Ser Cys Asp Lys Thr His Thr Cys Pro Pro Cys Pro Ala Pro Pro Val
        355                 360                 365

Ala Gly Pro Ser Val Phe Leu Phe Pro Pro Lys Pro Lys Asp Thr Leu
    370                 375                 380

Met Ile Ser Arg Thr Pro Glu Val Thr Cys Val Val Val Asp Val Ser
385                 390                 395                 400

His Glu Asp Pro Glu Val Lys Phe Asn Trp Tyr Val Asp Gly Val Glu
                405                 410                 415

Val His Asn Ala Lys Thr Lys Pro Arg Glu Glu Gln Tyr Asn Ser Thr
            420                 425                 430

Tyr Arg Val Val Ser Val Leu Thr Val Leu His Gln Asp Trp Leu Asn
        435                 440                 445

Gly Lys Glu Tyr Lys Cys Lys Val Ser Asn Lys Gly Leu Pro Ser Ser
    450                 455                 460

Ile Glu Lys Thr Ile Ser Lys Ala Lys Gly Gln Pro Arg Glu Pro Gln
465                 470                 475                 480

Val Tyr Thr Leu Pro Pro Ser Arg Asp Glu Leu Thr Lys Asn Gln Val
                485                 490                 495

Ser Leu Thr Cys Leu Val Lys Gly Phe Tyr Pro Ser Asp Ile Ala Val
            500                 505                 510

Glu Trp Glu Ser Asn Gly Gln Pro Glu Asn Asn Tyr Lys Thr Thr Pro
        515                 520                 525

Pro Val Leu Asp Ser Asp Gly Ser Phe Phe Leu Tyr Ser Lys Leu Thr
    530                 535                 540

Val Asp Lys Ser Arg Trp Gln Gln Gly Asn Val Phe Ser Cys Ser Val
545                 550                 555                 560

Met His Glu Ala Leu His Asn His Tyr Thr Gln Lys Ser Leu Ser Leu
                565                 570                 575

Ser Pro Gly Lys
            580

<210>  99
<211>  1722
<212>  DNA
<213>  artificial sequence

<400>  99
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacaacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccggt     360

cctctgggag tacgagaggt gcagctggtg gagtctggag gaggcttggt ccagcctggg     420

gggtccctga gactctcctg tgcagcctct gggttcaata ttaaggacac ttacatccac     480

tgggtccgcc aggctccagg gaaggggctg gagtgggtcg cacgtattta tcctaccaat     540

ggttacacac gctacgcaga ctccgtgaag ggccgattca ccatctccgc agacacttcc     600

aagaacacgg cgtatcttca aatgaacagc ctgagagccg aggacacggc cgtgtattac 660

tgttcgagat ggggcggtga cggcttctat gccatggact actggggcca aggaaccctg 720

gtcaccgtct cctcagcctc caccaagggc ccatcggtct tccccctggc accctcctcc 780

aagagcacct ctgggggcac agcggccctg ggctgcctgg tcaaggacta cttccccgaa 840

ccggtgacgg tgtcgtggaa ctcaggcgcc ctgaccagcg gcgtgcacac cttcccggct 900

gtcctacagt cctcaggact ctactccctc agcagcgtgg tgactgtgcc ctctagcagc 960

ttgggcaccc agacctacat ctgcaacgtg aatcacaagc ccagcaacac caaggtggac 1020

aagaaagttg aacccaaatc ttgcgacaaa actcacacat gcccaccgtg cccagcacct 1080

ccagtcgccg gaccgtcagt cttcctcttc cctccaaaac ccaaggacac cctcatgatc 1140

tcccggaccc ctgaggtcac atgcgtggtg gtggacgtga gccacgaaga ccctgaggtc 1200

aagttcaact ggtacgtgga cggcgtggag gtgcataatg ccaagacaaa gccgcgggag 1260

gagcagtaca acagcacgta ccgtgtggtc agcgtcctca ccgtcctgca ccaggactgg 1320

ctgaatggca aggagtacaa gtgcaaggtc tccaacaaag gcctcccaag ctccatcgag 1380

aaaaccatct ccaaagccaa agggcagccc cgagaaccac aggtgtacac cctgcctcca 1440

tcccgggatg agctgaccaa gaaccaggtc agcctgacct gcctggtcaa aggcttctat 1500

cccagcgaca tcgccgtgga gtgggagagc aatgggcagc cggagaacaa ctacaagacc 1560

acgcctcccg tgctggactc cgacggctcc ttcttcctct acagcaagct caccgtggac 1620

aagagcaggt ggcagcaggg gaacgtcttc tcatgctccg tgatgcatga ggctctgcac 1680

aaccactaca cgcagaagag cctctccctg tctccgggta aa 1722

<210> 100
<211> 574
<212> PRT
<213> artificial sequence

<400> 100

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val

65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Pro Leu Gly Val Arg Glu Val Gln
        115                 120                 125

Leu Val Glu Ser Gly Gly Gly Leu Val Gln Pro Gly Gly Ser Leu Arg
    130                 135                 140

Leu Ser Cys Ala Ala Ser Gly Phe Asn Ile Lys Asp Thr Tyr Ile His
145                 150                 155                 160

Trp Val Arg Gln Ala Pro Gly Lys Gly Leu Glu Trp Val Ala Arg Ile
                165                 170                 175

Tyr Pro Thr Asn Gly Tyr Thr Arg Tyr Ala Asp Ser Val Lys Gly Arg
            180                 185                 190

Phe Thr Ile Ser Ala Asp Thr Ser Lys Asn Thr Ala Tyr Leu Gln Met
        195                 200                 205

Asn Ser Leu Arg Ala Glu Asp Thr Ala Val Tyr Tyr Cys Ser Arg Trp
    210                 215                 220

Gly Gly Asp Gly Phe Tyr Ala Met Asp Tyr Trp Gly Gln Gly Thr Leu
225                 230                 235                 240

Val Thr Val Ser Ser Ala Ser Thr Lys Gly Pro Ser Val Phe Pro Leu
                245                 250                 255

Ala Pro Ser Ser Lys Ser Thr Ser Gly Gly Thr Ala Ala Leu Gly Cys
            260                 265                 270

Leu Val Lys Asp Tyr Phe Pro Glu Pro Val Thr Val Ser Trp Asn Ser
        275                 280                 285

Gly Ala Leu Thr Ser Gly Val His Thr Phe Pro Ala Val Leu Gln Ser
    290                 295                 300

Ser Gly Leu Tyr Ser Leu Ser Ser Val Val Thr Val Pro Ser Ser Ser
305                 310                 315                 320

Leu Gly Thr Gln Thr Tyr Ile Cys Asn Val Asn His Lys Pro Ser Asn
                325                 330                 335

Thr Lys Val Asp Lys Lys Val Glu Pro Lys Ser Cys Asp Lys Thr His
            340                 345                 350

Thr Cys Pro Pro Cys Pro Ala Pro Pro Val Ala Gly Pro Ser Val Phe
        355                 360                 365

Leu Phe Pro Pro Lys Pro Lys Asp Thr Leu Met Ile Ser Arg Thr Pro
    370                 375                 380

Glu Val Thr Cys Val Val Val Asp Val Ser His Glu Asp Pro Glu Val
385                 390                 395                 400

Lys Phe Asn Trp Tyr Val Asp Gly Val Glu Val His Asn Ala Lys Thr
                405                 410                 415

Lys Pro Arg Glu Glu Gln Tyr Asn Ser Thr Tyr Arg Val Val Ser Val
            420                 425                 430

Leu Thr Val Leu His Gln Asp Trp Leu Asn Gly Lys Glu Tyr Lys Cys
        435                 440                 445

Lys Val Ser Asn Lys Gly Leu Pro Ser Ser Ile Glu Lys Thr Ile Ser
    450                 455                 460

Lys Ala Lys Gly Gln Pro Arg Glu Pro Gln Val Tyr Thr Leu Pro Pro
465                 470                 475                 480

Ser Arg Asp Glu Leu Thr Lys Asn Gln Val Ser Leu Thr Cys Leu Val
                485                 490                 495

Lys Gly Phe Tyr Pro Ser Asp Ile Ala Val Glu Trp Glu Ser Asn Gly
            500                 505                 510

Gln Pro Glu Asn Asn Tyr Lys Thr Thr Pro Pro Val Leu Asp Ser Asp
        515                 520                 525

Gly Ser Phe Phe Leu Tyr Ser Lys Leu Thr Val Asp Lys Ser Arg Trp
    530                 535                 540

Gln Gln Gly Asn Val Phe Ser Cys Ser Val Met His Glu Ala Leu His
545                 550                 555                 560

Asn His Tyr Thr Gln Lys Ser Leu Ser Leu Ser Pro Gly Lys
                565                 570

<210>  101
<211>  1035
<212>  DNA
<213>  artificial sequence

<400>  101
aactgggtga acgtgatcag cgacctgaag aagatcgagg acctgatcca gagcatgcac        60

atcgacgcca ccctgtacac cgagagcgac gtgcacccca gctgcaaggt gaccgccatg       120

aagtgcttcc tgctggagct gcaggtgatc agcctggaga gcggcgacgc cagcatccac       180

gacaccgtgg agaacctgat catcctggcc aacaacagcc tgagcagcaa cggcaacgtg       240

accgagagcg gctgcaagga gtgcgaggag ctggaggaga agaacatcaa ggagttcctg       300

cagagcttcg tgcacatcgt gcagatgttc atcaacacca gcggcggagg cggatccggt       360

cctctgggag tacgaggcgg gggtgggagc ggggacatcc agatgaccca gtctccatcc       420

tccctgtctg catctgtagg agacagagtc accatcactt gccgggcaag tcaggatgtg       480

aataccgcgg tcgcatggta tcagcagaaa ccagggaaag cccctaagct cctgatctat       540

tctgcatcct tcttgtatag tggggtccca tcaaggttca gtggcagtag atctgggaca       600

gatttcactc tcaccatcag cagtctgcaa cctgaagatt ttgcaactta ctactgtcaa       660

cagcattaca ctacccctcc gacgttcggc caaggtacca aggttgagat caaacgcaca       720

gtggcagccc ccagcgtctt catttttccc ccttccgatg aacagctgaa gtccggcact       780

gcttctgtgg tctgtctgct gaacaatttc tatcccagag aggccaaggt gcagtggaaa       840

gtggacaacg ctctgcagtc cggcaacagc caggagagtg tgaccgaaca ggatagtaag       900

gacagcacat attctctgtc tagtaccctg acactgagta aggcagatta cgagaagcac       960

aaagtgtatg cctgcgaagt cactcatcag ggactgtcaa gccccgtgac caagagcttc      1020

aaccggggcg agtgt                                                       1035

<210>  102
<211>  345
<212>  PRT
<213>  artificial sequence

<400>  102

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Pro Leu Gly Val Arg Gly Gly Gly
        115                 120                 125

Gly Ser Gly Asp Ile Gln Met Thr Gln Ser Pro Ser Ser Leu Ser Ala
    130                 135                 140

Ser Val Gly Asp Arg Val Thr Ile Thr Cys Arg Ala Ser Gln Asp Val
145                 150                 155                 160

Asn Thr Ala Val Ala Trp Tyr Gln Gln Lys Pro Gly Lys Ala Pro Lys
                165                 170                 175

Leu Leu Ile Tyr Ser Ala Ser Phe Leu Tyr Ser Gly Val Pro Ser Arg
            180                 185                 190

Phe Ser Gly Ser Arg Ser Gly Thr Asp Phe Thr Leu Thr Ile Ser Ser
        195                 200                 205

Leu Gln Pro Glu Asp Phe Ala Thr Tyr Tyr Cys Gln Gln His Tyr Thr
    210                 215                 220

Thr Pro Pro Thr Phe Gly Gln Gly Thr Lys Val Glu Ile Lys Arg Thr
225                 230                 235                 240

Val Ala Ala Pro Ser Val Phe Ile Phe Pro Pro Ser Asp Glu Gln Leu
                245                 250                 255

Lys Ser Gly Thr Ala Ser Val Val Cys Leu Leu Asn Asn Phe Tyr Pro
            260                 265                 270

Arg Glu Ala Lys Val Gln Trp Lys Val Asp Asn Ala Leu Gln Ser Gly
        275                 280                 285

Asn Ser Gln Glu Ser Val Thr Glu Gln Asp Ser Lys Asp Ser Thr Tyr
    290                 295                 300

Ser Leu Ser Ser Thr Leu Thr Leu Ser Lys Ala Asp Tyr Glu Lys His
305                 310                 315                 320

Lys Val Tyr Ala Cys Glu Val Thr His Gln Gly Leu Ser Ser Pro Val
                325                 330                 335

Thr Lys Ser Phe Asn Arg Gly Glu Cys
            340                 345

<210>  103
<211>  1017
<212>  DNA
<213>  artificial sequence

<400>  103
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacaacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccggt     360

cctctgggag tacgagacat ccagatgacc cagtctccat cctccctgtc tgcatctgta     420

ggagacagag tcaccatcac ttgccgggca agtcaggatg tgaataccgc ggtcgcatgg     480

tatcagcaga aaccaggaaa agcccctaag ctcctgatct attctgcatc cttcttgtat     540

agtggggtcc catcaaggtt cagtggcagt agatctggga cagatttcac tctcaccatc     600

agcagtctgc aacctgaaga ttttgcaact tactactgtc aacagcatta cactacccct     660

ccgacgttcg gccaaggtac caaggttgag atcaaacgca cagtggcagc ccccagcgtc      720

ttcatttttc ccccttccga tgaacagctg aagtccggca ctgcttctgt ggtctgtctg     780

ctgaacaatt tctatcccag agaggccaag gtgcagtgga aagtggacaa cgctctgcag     840

tccggcaaca gccaggagag tgtgaccgaa caggatagta aggacagcac atattctctg     900

tctagtaccc tgacactgag taaggcagat tacgagaagc acaaagtgta tgcctgcgaa     960

gtcactcatc agggactgtc aagccccgtg accaagagct tcaaccgggg cgagtgt      1017

<210>  104
<211>  339
<212>  PRT
<213>  artificial sequence

<400>  104

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Pro Leu Gly Val Arg Asp Ile Gln
        115                 120                 125

Met Thr Gln Ser Pro Ser Ser Leu Ser Ala Ser Val Gly Asp Arg Val
    130                 135                 140

Thr Ile Thr Cys Arg Ala Ser Gln Asp Val Asn Thr Ala Val Ala Trp
145                 150                 155                 160

Tyr Gln Gln Lys Pro Gly Lys Ala Pro Lys Leu Leu Ile Tyr Ser Ala
                165                 170                 175

Ser Phe Leu Tyr Ser Gly Val Pro Ser Arg Phe Ser Gly Ser Arg Ser
            180                 185                 190

Gly Thr Asp Phe Thr Leu Thr Ile Ser Ser Leu Gln Pro Glu Asp Phe
        195                 200                 205

Ala Thr Tyr Tyr Cys Gln Gln His Tyr Thr Thr Pro Pro Thr Phe Gly
    210                 215                 220

Gln Gly Thr Lys Val Glu Ile Lys Arg Thr Val Ala Ala Pro Ser Val
225                 230                 235                 240

Phe Ile Phe Pro Pro Ser Asp Glu Gln Leu Lys Ser Gly Thr Ala Ser
                245                 250                 255

Val Val Cys Leu Leu Asn Asn Phe Tyr Pro Arg Glu Ala Lys Val Gln

260                 265                 270

Trp Lys Val Asp Asn Ala Leu Gln Ser Gly Asn Ser Gln Glu Ser Val
        275                 280                 285

Thr Glu Gln Asp Ser Lys Asp Ser Thr Tyr Ser Leu Ser Ser Thr Leu
    290                 295                 300

Thr Leu Ser Lys Ala Asp Tyr Glu Lys His Lys Val Tyr Ala Cys Glu
305                 310                 315                 320

Val Thr His Gln Gly Leu Ser Ser Pro Val Thr Lys Ser Phe Asn Arg
                325                 330                 335

Gly Glu Cys

<210>  105
<211>  1737
<212>  DNA
<213>  artificial sequence

<400>  105
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacaacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gcggcggagg cggatccgga     360

ggcggaggtt ccggcggggg tgggagcggg gaggtgcagc tggtggagtc tggaggaggc     420

ttggtccagc ctggggggtc cctgagactc tcctgtgcag cctctgggtt caatattaag     480

gacacttaca tccactgggt ccgccaggct ccagggaagg ggctggagtg ggtcgcacgt     540

atttatccta ccaatggtta cacacgctac gcagactccg tgaagggccg attcaccatc     600

tccgcagaca cttccaagaa cacggcgtat cttcaaatga acagcctgag agccgaggac     660

acggccgtgt attactgttc gagatggggc ggtgacggct tctatgccat ggactactgg     720

ggccaaggaa ccctggtcac cgtctcctca gcctccacca agggcccatc ggtcttcccc     780

ctggcaccct cctccaagag cacctctggg ggcacagcgg ccctgggctg cctggtcaag     840

gactacttcc ccgaaccggt gacggtgtcg tggaactcag gcgccctgac cagcggcgtg     900

cacaccttcc cggctgtcct acagtcctca ggactctact ccctcagcag cgtggtgact     960

gtgccctcta gcagcttggg cacccagacc tacatctgca acgtgaatca caagcccagc    1020

aacaccaagg tggacaagaa agttgaaccc aaatcttgcg acaaaactca cacatgccca 1080

ccgtgcccag cacctccagt cgccggaccg tcagtcttcc tcttccctcc aaaacccaag 1140

gacaccctca tgatctcccg gacccctgag gtcacatgcg tggtggtgga cgtgagccac 1200

gaagaccctg aggtcaagtt caactggtac gtggacggcg tggaggtgca taatgccaag 1260

acaaagccgc gggaggagca gtacaacagc acgtaccgtg tggtcagcgt cctcaccgtc 1320

ctgcaccagg actggctgaa tggcaaggag tacaagtgca aggtctccaa caaaggcctc 1380

ccaagctcca tcgagaaaac catctccaaa gccaaagggc agccccgaga accacaggtg 1440

tacaccctgc ctccatcccg ggatgagctg accaagaacc aggtcagcct gacctgcctg 1500

gtcaaaggct tctatcccag cgacatcgcc gtggagtggg agagcaatgg gcagccggag 1560

aacaactaca agaccacgcc tcccgtgctg gactccgacg gctccttctt cctctacagc 1620

aagctcaccg tggacaagag caggtggcag caggggaacg tcttctcatg ctccgtgatg 1680

catgaggctc tgcacaacca ctacacgcag aagagcctct ccctgtctcc gggtaaa 1737

<210> 106
<211> 579
<212> PRT
<213> artificial sequence

<400> 106

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Glu Val Gln Leu Val Glu Ser Gly Gly Gly Leu Val Gln Pro
    130                 135                 140

Gly Gly Ser Leu Arg Leu Ser Cys Ala Ala Ser Gly Phe Asn Ile Lys
145                 150                 155                 160

Asp Thr Tyr Ile His Trp Val Arg Gln Ala Pro Gly Lys Gly Leu Glu
                165                 170                 175

Trp Val Ala Arg Ile Tyr Pro Thr Asn Gly Tyr Thr Arg Tyr Ala Asp
            180                 185                 190

Ser Val Lys Gly Arg Phe Thr Ile Ser Ala Asp Thr Ser Lys Asn Thr
        195                 200                 205

Ala Tyr Leu Gln Met Asn Ser Leu Arg Ala Glu Asp Thr Ala Val Tyr
    210                 215                 220

Tyr Cys Ser Arg Trp Gly Gly Asp Gly Phe Tyr Ala Met Asp Tyr Trp
225                 230                 235                 240

Gly Gln Gly Thr Leu Val Thr Val Ser Ser Ala Ser Thr Lys Gly Pro
                245                 250                 255

Ser Val Phe Pro Leu Ala Pro Ser Ser Lys Ser Thr Ser Gly Gly Thr
            260                 265                 270

Ala Ala Leu Gly Cys Leu Val Lys Asp Tyr Phe Pro Glu Pro Val Thr
        275                 280                 285

Val Ser Trp Asn Ser Gly Ala Leu Thr Ser Gly Val His Thr Phe Pro
    290                 295                 300

Ala Val Leu Gln Ser Ser Gly Leu Tyr Ser Leu Ser Ser Val Val Thr
305                 310                 315                 320

Val Pro Ser Ser Ser Leu Gly Thr Gln Thr Tyr Ile Cys Asn Val Asn
                325                 330                 335

His Lys Pro Ser Asn Thr Lys Val Asp Lys Lys Val Glu Pro Lys Ser
            340                 345                 350

Cys Asp Lys Thr His Thr Cys Pro Pro Cys Pro Ala Pro Pro Val Ala
        355                 360                 365

Gly Pro Ser Val Phe Leu Phe Pro Pro Lys Pro Lys Asp Thr Leu Met
    370                 375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val
                405                 410                 415

His Asn Ala Lys Thr Lys Pro Arg Glu Glu Gln Tyr Asn Ser Thr Tyr
            420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Gly Leu Pro Ser Ser Ile
    450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Asp Glu Leu Thr Lys Asn Gln Val Ser
                485                 490                 495

Leu Thr Cys Leu Val Lys Gly Phe Tyr Pro Ser Asp Ile Ala Val Glu
            500                 505                 510

Trp Glu Ser Asn Gly Gln Pro Glu Asn Asn Tyr Lys Thr Thr Pro Pro
        515                 520                 525

Val Leu Asp Ser Asp Gly Ser Phe Phe Leu Tyr Ser Lys Leu Thr Val
    530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys

<210>  107
<211>  1032
<212>  DNA
<213>  artificial sequence

<400>  107
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac 180

gacaccgtgg agaacctgat catcctggcc aacaacagcc tgagcagcaa cggcaacgtg 240

accgagagcg gctgcaagga gtgcgaggag ctggaggaga agaacatcaa ggagttcctg 300

cagagcttcg tgcacatcgt gcagatgttc atcaacacca gcggcggagg cggatccgga 360

ggcggaggtt ccggcggggg tgggagcggg gacatccaga tgacccagtc tccatcctcc 420

ctgtctgcat ctgtaggaga cagagtcacc atcacttgcc gggcaagtca ggatgtgaat 480

accgcggtcg catggtatca gcagaaacca gggaaagccc ctaagctcct gatctattct 540

gcatccttct tgtatagtgg ggtcccatca aggttcagtg gcagtagatc tgggacagat 600

ttcactctca ccatcagcag tctgcaacct gaagattttg caacttacta ctgtcaacag 660

cattacacta cccctccgac gttcggccaa ggtaccaagg ttgagatcaa acgcacagtg 720

gcagccccca gcgtcttcat tttcccccct tccgatgaac agctgaagtc cggcactgct 780

tctgtggtct gtctgctgaa caatttctat cccagagagg ccaaggtgca gtggaaagtg 840

gacaacgctc tgcagtccgg caacagccag gagagtgtga ccgaacagga tagtaaggac 900

agcacatatt ctctgtctag taccctgaca ctgagtaagg cagattacga gaagcacaaa 960

gtgtatgcct gcgaagtcac tcatcaggga ctgtcaagcc ccgtgaccaa gagcttcaac 1020

cggggcgagt gt 1032

<210> 108
<211> 344
<212> PRT
<213> artificial sequence

<400> 108

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Asp Ile Gln Met Thr Gln Ser Pro Ser Ser Leu Ser Ala Ser
    130                 135                 140

Val Gly Asp Arg Val Thr Ile Thr Cys Arg Ala Ser Gln Asp Val Asn
145                 150                 155                 160

Thr Ala Val Ala Trp Tyr Gln Gln Lys Pro Gly Lys Ala Pro Lys Leu
                165                 170                 175

Leu Ile Tyr Ser Ala Ser Phe Leu Tyr Ser Gly Val Pro Ser Arg Phe
            180                 185                 190

Ser Gly Ser Arg Ser Gly Thr Asp Phe Thr Leu Thr Ile Ser Ser Leu
        195                 200                 205

Gln Pro Glu Asp Phe Ala Thr Tyr Tyr Cys Gln Gln His Tyr Thr Thr
    210                 215                 220

Pro Pro Thr Phe Gly Gln Gly Thr Lys Val Glu Ile Lys Arg Thr Val
225                 230                 235                 240

Ala Ala Pro Ser Val Phe Ile Phe Pro Pro Ser Asp Glu Gln Leu Lys
                245                 250                 255

Ser Gly Thr Ala Ser Val Val Cys Leu Leu Asn Asn Phe Tyr Pro Arg
            260                 265                 270

Glu Ala Lys Val Gln Trp Lys Val Asp Asn Ala Leu Gln Ser Gly Asn
        275                 280                 285

Ser Gln Glu Ser Val Thr Glu Gln Asp Ser Lys Asp Ser Thr Tyr Ser
    290                 295                 300

Leu Ser Ser Thr Leu Thr Leu Ser Lys Ala Asp Tyr Glu Lys His Lys
305                 310                 315                 320

Val Tyr Ala Cys Glu Val Thr His Gln Gly Leu Ser Ser Pro Val Thr
                325                 330                 335

Lys Ser Phe Asn Arg Gly Glu Cys

            340

<210>  109
<211>  1347
<212>  DNA
<213>  artificial sequence

<400>  109
gaggtgcagc tggtggagtc tggaggaggc ttggtccagc ctggggggtc cctgagactc      60

tcctgtgcag cctctgggtt caatattaag gacacttaca tccactgggt ccgccaggct     120

ccagggaagg ggctggagtg ggtcgcacgt atttatccta ccaatggtta cacacgctac     180

gcagactccg tgaagggccg attcaccatc tccgcagaca cttccaagaa cacggcgtat     240

cttcaaatga acagcctgag agccgaggac acggccgtgt attactgttc gagatggggc     300

ggtgacggct tctatgccat ggactactgg ggccaaggaa ccctggtcac cgtctcctca     360

gcctccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgact gtgccctcta gcagcttggg cacccagacc     600

tacatctgca acgtgaatca caagcccagc aacaccaagg tggacaagaa agttgaaccc     660

aaatcttgcg acaaaactca cacatgccca ccgtgcccag cacctccagt cgccggaccg     720

tcagtcttcc tcttccctcc aaaacccaag gacaccctca tgatctcccg gacccctgag     780

gtcacatgcg tggtggtgga cgtgagccac gaagaccctg aggtcaagtt caactggtac     840

gtggacggcg tggaggtgca taatgccaag acaaagccgc gggaggagca gtacaacagc     900

acgtaccgtg tggtcagcgt cctcaccgtc ctgcaccagg actggctgaa tggcaaggag     960

tacaagtgca aggtctccaa caaaggcctc ccaagctcca tcgagaaaac catctccaaa    1020

gccaaagggc agccccgaga accacaggtg tacaccctgc ctccatcccg ggatgagctg    1080

accaagaacc aggtcagcct gacctgcctg gtcaaaggct tctatcccag cgacatcgcc    1140

gtggagtggg agagcaatgg gcagccggag aacaactaca agaccacgcc tcccgtgctg    1200

gactccgacg gctccttctt cctctacagc aagctcaccg tggacaagag caggtggcag    1260

caggggaacg tcttctcatg ctccgtgatg catgaggctc tgcacaacca ctacacgcag    1320

aagagcctct ccctgtctcc gggtaaa                                        1347

<210>  110
<211>  449
<212>  PRT
<213>  artificial sequence

<400>  110

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Pro Val Ala Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Gly Leu Pro Ser Ser Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  111
<211>  342
<212>  DNA
<213>  artificial sequence

<400>  111
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac 180

gacaccgtgg agaacctgat catcctggcc aacaacagcc tgagcagcaa cggcaacgtg 240

accgagagcg gctgcaagga gtgcgaggag ctggaggaga agaacatcaa ggagttcctg 300

cagagcttcg tgcacatcgt gcagatgttc atcaacacca gc 342

<210> 112
<211> 114
<212> PRT
<213> artificial sequence

<400> 112

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser

<210> 113
<211> 578
<212> PRT
<213> artificial sequence

<400> 113

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Gln Val Gln Leu Gln Glu Ser Gly Pro Gly Leu Val Lys Pro
    130                 135                 140

Ser Glu Thr Leu Ser Leu Thr Cys Thr Val Ser Gly Gly Ser Val Ser
145                 150                 155                 160

Ser Gly Asp Tyr Tyr Trp Thr Trp Ile Arg Gln Ser Pro Gly Lys Gly
                165                 170                 175

Leu Glu Trp Ile Gly His Ile Tyr Tyr Ser Gly Asn Thr Asn Tyr Asn
            180                 185                 190

Pro Ser Leu Lys Ser Arg Leu Thr Ile Ser Ile Asp Thr Ser Lys Thr
        195                 200                 205

Gln Phe Ser Leu Lys Leu Ser Ser Val Thr Ala Ala Asp Thr Ala Ile
    210                 215                 220

Tyr Tyr Cys Val Arg Asp Arg Val Thr Gly Ala Phe Asp Ile Trp Gly
225                 230                 235                 240

Gln Gly Thr Met Val Thr Val Ser Ser Ala Ser Thr Lys Gly Pro Ser
                245                 250                 255

Val Phe Pro Leu Ala Pro Ser Ser Lys Ser Thr Ser Gly Gly Thr Ala
            260                 265                 270

Ala Leu Gly Cys Leu Val Lys Asp Tyr Phe Pro Glu Pro Val Thr Val
        275                 280                 285

Ser Trp Asn Ser Gly Ala Leu Thr Ser Gly Val His Thr Phe Pro Ala
    290                 295                 300

Val Leu Gln Ser Ser Gly Leu Tyr Ser Leu Ser Ser Val Val Thr Val
305                 310                 315                 320

Pro Ser Ser Ser Leu Gly Thr Gln Thr Tyr Ile Cys Asn Val Asn His
                325                 330                 335

Lys Pro Ser Asn Thr Lys Val Asp Lys Lys Val Glu Pro Lys Ser Cys
            340                 345                 350

Asp Lys Thr His Thr Cys Pro Pro Cys Pro Ala Pro Pro Val Ala Gly
        355                 360                 365

Pro Ser Val Phe Leu Phe Pro Pro Lys Pro Lys Asp Thr Leu Met Ile
    370                 375                 380

Ser Arg Thr Pro Glu Val Thr Cys Val Val Val Asp Val Ser His Glu
385                 390                 395                 400

Asp Pro Glu Val Lys Phe Asn Trp Tyr Val Asp Gly Val Glu Val His
                405                 410                 415

Asn Ala Lys Thr Lys Pro Arg Glu Glu Gln Tyr Asn Ser Thr Tyr Arg
            420                 425                 430

Val Val Ser Val Leu Thr Val Leu His Gln Asp Trp Leu Asn Gly Lys
        435                 440                 445

Glu Tyr Lys Cys Lys Val Ser Asn Lys Gly Leu Pro Ser Ser Ile Glu
    450                 455                 460

Lys Thr Ile Ser Lys Ala Lys Gly Gln Pro Arg Glu Pro Gln Val Tyr
465                 470                 475                 480

Thr Leu Pro Pro Ser Arg Asp Glu Leu Thr Lys Asn Gln Val Ser Leu
                485                 490                 495

Thr Cys Leu Val Lys Gly Phe Tyr Pro Ser Asp Ile Ala Val Glu Trp
            500                 505                 510

Glu Ser Asn Gly Gln Pro Glu Asn Asn Tyr Lys Thr Thr Pro Pro Val
        515                 520                 525

Leu Asp Ser Asp Gly Ser Phe Phe Leu Tyr Ser Lys Leu Thr Val Asp
    530                 535                 540

Lys Ser Arg Trp Gln Gln Gly Asn Val Phe Ser Cys Ser Val Met His
545                 550                 555                 560

Glu Ala Leu His Asn His Tyr Thr Gln Lys Ser Leu Ser Leu Ser Pro
                565                 570                 575

Gly Lys

<210>  114
<211>  578
<212>  PRT
<213>  artificial sequence

<400>  114

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Asp Tyr Tyr Trp Thr Trp Ile Arg Gln Ser Pro Gly Lys Gly Leu Glu
        35                  40                  45

Trp Ile Gly His Ile Tyr Tyr Ser Gly Asn Thr Asn Tyr Asn Pro Ser
    50                  55                  60

Leu Lys Ser Arg Leu Thr Ile Ser Ile Asp Thr Ser Lys Thr Gln Phe
65                  70                  75                  80

Ser Leu Lys Leu Ser Ser Val Thr Ala Ala Asp Thr Ala Ile Tyr Tyr
                85                  90                  95

Cys Val Arg Asp Arg Val Thr Gly Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
        180                 185                 190

Ser Ser Leu Gly Thr Gln Thr Tyr Ile Cys Asn Val Asn His Lys Pro
    195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
        260                 265                 270

Glu Val Lys Phe Asn Trp Tyr Val Asp Gly Val Glu Val His Asn Ala
    275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
        340                 345                 350

Pro Pro Ser Arg Asp Glu Leu Thr Lys Asn Gln Val Ser Leu Thr Cys
    355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala

420                 425                 430

Leu His Asn His Tyr Thr Gln Lys Ser Leu Ser Leu Ser Pro Gly Lys
        435                 440                 445

Gly Gly Gly Gly Ser Gly Gly Gly Gly Ser Gly Gly Gly Gly Ser Gly
    450                 455                 460

Asn Trp Val Asn Val Ile Ser Asp Leu Lys Lys Ile Glu Asp Leu Ile
465                 470                 475                 480

Gln Ser Met His Ile Asp Ala Thr Leu Tyr Thr Glu Ser Asp Val His
                485                 490                 495

Pro Ser Cys Lys Val Thr Ala Met Lys Cys Phe Leu Leu Glu Leu Gln
            500                 505                 510

Val Ile Ser Leu Glu Ser Gly Asp Ala Ser Ile His Asp Thr Val Glu
        515                 520                 525

Asn Leu Ile Ile Leu Ala Asn Asn Ser Leu Ser Ser Asn Gly Asn Val
    530                 535                 540

Thr Glu Ser Gly Cys Lys Glu Cys Glu Glu Leu Glu Glu Lys Asn Ile
545                 550                 555                 560

Lys Glu Phe Leu Gln Ser Phe Val His Ile Val Gln Met Phe Ile Asn
                565                 570                 575

Thr Ser

<210>  115
<211>  344
<212>  PRT
<213>  artificial sequence

<400>  115

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly

50                  55                  60

Ser Gly Ser Gly Thr Asp Phe Thr Phe Thr Ile Ser Ser Leu Gln Pro
65                  70                  75                  80

Glu Asp Ile Ala Thr Tyr Phe Cys Gln His Phe Asp His Leu Pro Leu
                85                  90                  95

Ala Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Gly Gly Gly Gly Ser Gly Gly Gly Gly Ser
    210                 215                 220

Gly Gly Gly Gly Ser Gly Asn Trp Val Asn Val Ile Ser Asp Leu Lys
225                 230                 235                 240

Lys Ile Glu Asp Leu Ile Gln Ser Met His Ile Asp Ala Thr Leu Tyr
                245                 250                 255

Thr Glu Ser Asp Val His Pro Ser Cys Lys Val Thr Ala Met Lys Cys
            260                 265                 270

Phe Leu Leu Glu Leu Gln Val Ile Ser Leu Glu Ser Gly Asp Ala Ser
        275                 280                 285

Ile His Asp Thr Val Glu Asn Leu Ile Ile Leu Ala Asn Asp Ser Leu
    290                 295                 300

Ser Ser Asn Gly Asn Val Thr Glu Ser Gly Cys Lys Glu Cys Glu Glu
305                 310                 315                 320

Leu Glu Glu Lys Asn Ile Lys Glu Phe Leu Gln Ser Phe Val His Ile
                325                 330                 335

Val Gln Met Phe Ile Asn Thr Ser
            340

<210>  116
<211>  308
<212>  PRT
<213>  artificial sequence

<400>  116

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Asp Tyr Tyr Trp Thr Trp Ile Arg Gln Ser Pro Gly Lys Gly Leu Glu
        35                  40                  45

Trp Ile Gly His Ile Tyr Tyr Ser Gly Asn Thr Asn Tyr Asn Pro Ser
    50                  55                  60

Leu Lys Ser Arg Leu Thr Ile Ser Ile Asp Thr Ser Lys Thr Gln Phe
65                  70                  75                  80

Ser Leu Lys Leu Ser Ser Val Thr Ala Ala Asp Thr Ala Ile Tyr Tyr
                85                  90                  95

Cys Val Arg Asp Arg Val Thr Gly Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Gly Gly Gly Gly Ser Gly Gly Gly Gly Ser Gly Gly Gly
225                 230                 235                 240

Gly Ser Gly Ile Thr Cys Pro Pro Pro Met Ser Val Glu His Ala Asp
                245                 250                 255

Ile Trp Val Lys Ser Tyr Ser Leu Tyr Ser Arg Glu Arg Tyr Ile Cys
            260                 265                 270

Asn Ser Gly Phe Lys Arg Lys Ala Gly Thr Ser Ser Leu Thr Glu Cys
        275                 280                 285

Val Leu Asn Lys Ala Thr Asn Val Ala His Trp Thr Thr Pro Ser Leu
    290                 295                 300

Lys Cys Ile Arg
305

<210>  117
<211>  357
<212>  PRT
<213>  artificial sequence

<400>  117

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Asp Tyr Tyr Trp Thr Trp Ile Arg Gln Ser Pro Gly Lys Gly Leu Glu
        35                  40                  45

Trp Ile Gly His Ile Tyr Tyr Ser Gly Asn Thr Asn Tyr Asn Pro Ser
    50                  55                  60

Leu Lys Ser Arg Leu Thr Ile Ser Ile Asp Thr Ser Lys Thr Gln Phe
65                  70                  75                  80

Ser Leu Lys Leu Ser Ser Val Thr Ala Ala Asp Thr Ala Ile Tyr Tyr
                85                  90                  95

Cys Val Arg Asp Arg Val Thr Gly Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Gly Gly Gly Gly Ser Gly Gly Gly Gly Ser Gly Gly Gly
225                 230                 235                 240

Gly Ser Gly Asn Trp Val Asn Val Ile Ser Asp Leu Lys Lys Ile Glu
                245                 250                 255

Asp Leu Ile Gln Ser Met His Ile Asp Ala Thr Leu Tyr Thr Glu Ser
            260                 265                 270

Asp Val His Pro Ser Cys Lys Val Thr Ala Met Lys Cys Phe Leu Leu
        275                 280                 285

Glu Leu Gln Val Ile Ser Leu Glu Ser Gly Asp Ala Ser Ile His Asp
    290                 295                 300

Thr Val Glu Asn Leu Ile Ile Leu Ala Asn Asp Ser Leu Ser Ser Asn
305                 310                 315                 320

Gly Asn Val Thr Glu Ser Gly Cys Lys Glu Cys Glu Glu Leu Glu Glu
                325                 330                 335

Lys Asn Ile Lys Glu Phe Leu Gln Ser Phe Val His Ile Val Gln Met
            340                 345                 350

Phe Ile Asn Thr Ser
        355

<210> 118
<211> 295
<212> PRT
<213> artificial sequence

<400> 118

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Phe Cys Gln His Phe Asp His Leu Pro Leu
                85                  90                  95

Ala Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Gly Gly Gly Gly Ser Gly Gly Gly Gly Ser
    210                 215                 220

Gly Gly Gly Gly Ser Gly Ile Thr Cys Pro Pro Pro Met Ser Val Glu
225                 230                 235                 240

His Ala Asp Ile Trp Val Lys Ser Tyr Ser Leu Tyr Ser Arg Glu Arg
                245                 250                 255

Tyr Ile Cys Asn Ser Gly Phe Lys Arg Lys Ala Gly Thr Ser Ser Leu
            260                 265                 270

Thr Glu Cys Val Leu Asn Lys Ala Thr Asn Val Ala His Trp Thr Thr
        275                 280                 285

Pro Ser Leu Lys Cys Ile Arg
    290                 295

<210>  119
<211>  580
<212>  PRT
<213>  artificial sequence

<400>  119

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser Gly Gly Gly Gly Ser Gly Gly Gly Gly Ser Gly Gly Gly Gly
        115                 120                 125

Ser Gly Glu Val Gln Leu Val Glu Ser Gly Gly Gly Leu Val Gln Pro
    130                 135                 140

Gly Gly Ser Leu Arg Leu Ser Cys Ala Ala Ser Gly Phe Asn Ile Lys
145                 150                 155                 160

Asp Thr Tyr Ile His Trp Val Arg Gln Ala Pro Gly Lys Gly Leu Glu
                165                 170                 175

Trp Val Ala Arg Ile Tyr Pro Thr Asn Gly Tyr Thr Arg Tyr Ala Asp
            180                 185                 190

Ser Val Lys Gly Arg Phe Thr Ile Ser Ala Asp Thr Ser Lys Asn Thr
        195                 200                 205

Ala Tyr Leu Gln Met Asn Ser Leu Arg Ala Glu Asp Thr Ala Val Tyr
    210                 215                 220

Tyr Cys Ser Arg Trp Gly Gly Asp Gly Phe Tyr Ala Met Asp Tyr Trp
225                 230                 235                 240

Gly Gln Gly Thr Leu Val Thr Val Ser Ser Ala Ser Thr Lys Gly Pro
                245                 250                 255

Ser Val Phe Pro Leu Ala Pro Ser Ser Lys Ser Thr Ser Gly Gly Thr
            260                 265                 270

Ala Ala Leu Gly Cys Leu Val Lys Asp Tyr Phe Pro Glu Pro Val Thr
        275                 280                 285

Val Ser Trp Asn Ser Gly Ala Leu Thr Ser Gly Val His Thr Phe Pro
    290                 295                 300

Ala Val Leu Gln Ser Ser Gly Leu Tyr Ser Leu Ser Ser Val Val Thr
305                 310                 315                 320

Val Pro Ser Ser Ser Leu Gly Thr Gln Thr Tyr Ile Cys Asn Val Asn
                325                 330                 335

His Lys Pro Ser Asn Thr Lys Val Asp Lys Lys Val Glu Pro Lys Ser
            340                 345                 350

Cys Asp Lys Thr His Thr Cys Pro Pro Cys Pro Ala Pro Glu Leu Leu
        355                 360                 365

Gly Gly Pro Ser Val Phe Leu Phe Pro Pro Lys Pro Lys Asp Thr Leu
    370                 375                 380

Met Ile Ser Arg Thr Pro Glu Val Thr Cys Val Val Val Asp Val Ser
385                 390                 395                 400

His Glu Asp Pro Glu Val Lys Phe Asn Trp Tyr Val Asp Gly Val Glu
                405                 410                 415

Val His Asn Ala Lys Thr Lys Pro Arg Glu Glu Gln Tyr Asn Ser Thr
            420                 425                 430

Tyr Arg Val Val Ser Val Leu Thr Val Leu His Gln Asp Trp Leu Asn
        435                 440                 445

Gly Lys Glu Tyr Lys Cys Lys Val Ser Asn Lys Ala Leu Pro Ala Pro
    450                 455                 460

Ile Glu Lys Thr Ile Ser Lys Ala Lys Gly Gln Pro Arg Glu Pro Gln
465                 470                 475                 480

Val Tyr Thr Leu Pro Pro Ser Arg Asp Glu Leu Thr Lys Asn Gln Val
                485                 490                 495

Ser Leu Thr Cys Leu Val Lys Gly Phe Tyr Pro Ser Asp Ile Ala Val
            500                 505                 510

Glu Trp Glu Ser Asn Gly Gln Pro Glu Asn Asn Tyr Lys Thr Thr Pro
        515                 520                 525

Pro Val Leu Asp Ser Asp Gly Ser Phe Phe Leu Tyr Ser Lys Leu Thr
    530                 535                 540

Val Asp Lys Ser Arg Trp Gln Gln Gly Asn Val Phe Ser Cys Ser Val
545                 550                 555                 560

Met His Glu Ala Leu His Asn His Tyr Thr Gln Lys Ser Leu Ser Leu
                565                 570                 575

Ser Pro Gly Lys
            580

<210>  120
<211>  531
<212>  PRT
<213>  artificial sequence

<400>  120

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Gly Gly Gly Gly Ser Gly Gly Gly Gly Ser Gly Gly Gly Gly Ser
65                  70                  75                  80

Gly Glu Val Gln Leu Val Glu Ser Gly Gly Gly Leu Val Gln Pro Gly
                85                  90                  95

Gly Ser Leu Arg Leu Ser Cys Ala Ala Ser Gly Phe Asn Ile Lys Asp
            100                 105                 110

Thr Tyr Ile His Trp Val Arg Gln Ala Pro Gly Lys Gly Leu Glu Trp
        115                 120                 125

Val Ala Arg Ile Tyr Pro Thr Asn Gly Tyr Thr Arg Tyr Ala Asp Ser
    130                 135                 140

Val Lys Gly Arg Phe Thr Ile Ser Ala Asp Thr Ser Lys Asn Thr Ala
145                 150                 155                 160

Tyr Leu Gln Met Asn Ser Leu Arg Ala Glu Asp Thr Ala Val Tyr Tyr
                165                 170                 175

Cys Ser Arg Trp Gly Gly Asp Gly Phe Tyr Ala Met Asp Tyr Trp Gly
            180                 185                 190

Gln Gly Thr Leu Val Thr Val Ser Ser Ala Ser Thr Lys Gly Pro Ser
        195                 200                 205

Val Phe Pro Leu Ala Pro Ser Ser Lys Ser Thr Ser Gly Gly Thr Ala
    210                 215                 220

Ala Leu Gly Cys Leu Val Lys Asp Tyr Phe Pro Glu Pro Val Thr Val
225                 230                 235                 240

Ser Trp Asn Ser Gly Ala Leu Thr Ser Gly Val His Thr Phe Pro Ala

245                 250                 255

Val Leu Gln Ser Ser Gly Leu Tyr Ser Leu Ser Ser Val Val Thr Val
            260                 265                 270

Pro Ser Ser Ser Leu Gly Thr Gln Thr Tyr Ile Cys Asn Val Asn His
        275                 280                 285

Lys Pro Ser Asn Thr Lys Val Asp Lys Lys Val Glu Pro Lys Ser Cys
    290                 295                 300

Asp Lys Thr His Thr Cys Pro Pro Cys Pro Ala Pro Glu Leu Leu Gly
305                 310                 315                 320

Gly Pro Ser Val Phe Leu Phe Pro Pro Lys Pro Lys Asp Thr Leu Met
                325                 330                 335

Ile Ser Arg Thr Pro Glu Val Thr Cys Val Val Val Asp Val Ser His
            340                 345                 350

Glu Asp Pro Glu Val Lys Phe Asn Trp Tyr Val Asp Gly Val Glu Val
        355                 360                 365

His Asn Ala Lys Thr Lys Pro Arg Glu Glu Gln Tyr Asn Ser Thr Tyr
    370                 375                 380

Arg Val Val Ser Val Leu Thr Val Leu His Gln Asp Trp Leu Asn Gly
385                 390                 395                 400

Lys Glu Tyr Lys Cys Lys Val Ser Asn Lys Ala Leu Pro Ala Pro Ile
                405                 410                 415

Glu Lys Thr Ile Ser Lys Ala Lys Gly Gln Pro Arg Glu Pro Gln Val
            420                 425                 430

Tyr Thr Leu Pro Pro Ser Arg Asp Glu Leu Thr Lys Asn Gln Val Ser
        435                 440                 445

Leu Thr Cys Leu Val Lys Gly Phe Tyr Pro Ser Asp Ile Ala Val Glu
    450                 455                 460

Trp Glu Ser Asn Gly Gln Pro Glu Asn Asn Tyr Lys Thr Thr Pro Pro
465                 470                 475                 480

Val Leu Asp Ser Asp Gly Ser Phe Phe Leu Tyr Ser Lys Leu Thr Val
                485                 490                 495

Asp Lys Ser Arg Trp Gln Gln Gly Asn Val Phe Ser Cys Ser Val Met
            500                 505                 510

His Glu Ala Leu His Asn His Tyr Thr Gln Lys Ser Leu Ser Leu Ser
        515                 520                 525

Pro Gly Lys
    530

## Description

### Technical Field

The present disclosure belongs to the field of bio-pharmaceuticals, and particularly relates to an immunocytokine in which IL-15 and IL-15Ra are fused to antibody or an immunocytokine in which IL-15 is fused to antibody, and preparation for the same, and uses thereof.

### Background

Over the past few years, immunotherapy has been used to treat various type of human tumors. Immunotherapy attempts to harness the power of the immune system to attack or kill malignant tumor cells while leaving healthy tissues intact. Immune system has the ability to identify and eliminate malignant tumor cells, however, tumors have developed a variety of mechanisms to escape immune surveillance. The challenge of immunotherapy is to develop strategies capable of effectively and safely augmenting anti-tumor responses of the body. the tumor immunotherapy strategies currently developed mainly include cytokine therapy, adoptive cell transfer therapy, cancer vaccines, and monoclonal antibody. The most promising of these strategies are those stimulating or activating the tumor-specific responses of T cells or NK that exerting the killing effect on tumors [The Potential and Promise of IL-15 in Immuno-Oncogenic Therapies].

IL-15 is a cytokine with a structure similar to IL-2, which can effectively regulate the development, proliferation and activation of effector, natural killer cells (NK), and memory CD8 + T cells. IL-15Ra is a transmembrane protein with high affinity to IL-15. After binding to IL-15, IL-15Ra is trans-presented to the surface of NK cells or T cells expressing IL2R beta and gamma chains, promoting proliferation and activation of these cells, and enhancing tumor-killing activity. Moreover, different from IL-2, IL-15 will not lead to the activation of Treg cells, and is currently undergoing extensive preclinical and clinical study as a tumor immunotherapy agent.

The complex (IL-15 superagonist) formed by IL-15 and IL-15Ra further accelerates the study of IL-15-based tumor immnuotherapy. Compared with the monomer IL-15, IL-15 superagonists have prominent advantages, such as higher blood circulation concentration, longer in vivo half-life and enhanced ability to stimulate NK and CD8T cells. Currently, there are two main types of IL-15 superagonists currently under development: RLI (e.g., Cytune's CYP0150) formed by the fusion of IL-15 with IL-15Ra via a linker, and intramolecular complexes (e.g., NVS NIZ985, Altor ALT-803, and Xencor XmAb24306) formed by IL-15 and IL-15Ra. CYP0150, is easily cleared in vivo, and has a short half-life due to the absence of Fc, requiring frequent administration. The structure of NIZ985 and ALT-803, in which IL-15 and IL-15Ra form complex through intermolecular interaction, has unstable structure, leading to complicated preparation process. XmAb24306 with IL-15 and IL-15Ra fused to N-terminus of Fc, respectively, has a longer in vivo half-life due to the stable structure in which IL-15 and IL-15Ra fused to N-terminus of Fc, respectively, forming intramolecular complex. A large number of preclinical studies have indicated that the physiological and anti-tumor activity of IL-15 superagonists are greatly enhanced when comparing to that of natural IL-15. Some studies have indicated that immunotherapy such as Nivolumab (anti-PD-1 monoclonal antibody) and Rituximab (anti-CD20 monoclonal antibody) can effectively enhance the anti-tumor effect of ALT-803 in patients with progressive tumor. Moreover, the systemic administration of the IL-15 superagonist can bias the expansion of pro-inflammatory (CD11b_{high}CD27_{high}) NK cell subsets in lymphoid organs, and mass secretion of proinflammatory factors such as IFNγ, leading to immune-related adverse effects such as hypothermia, weight loss, liver damage.

The inventor finds that the immunocytokine, in which IL-15 and IL-15Ra are fused with antibody targeting tumor antigen, respectively, may specifically target tumor microenvironment. In tumor microenvironment, the complex formed by IL-15/IL15Ra in the immunocytokine may activate immune cells within or in the vicinity of a tumor, thereby exerting the specific killing effect of T cells or NK cells on the tumor while preventing immune-related adverse effects induced by the systemic hyperactivation of T cells or NK cells.

### Summary

The inventor finds that the immunocytokine, in which IL-15 and IL-15Ra are fused to an antibody targeting therapy-related cell surface antigen, respectively, can effectively and specifically target tumor microenvironment, and activate immune cells within or in the vicinity of a tumor, exerting specific killing effects on tumor while preventing immune-related adverse effects induced by the systemic hyperactivation of NK cells.

One aspect of the present disclosure provides an immunocytokine, which includes:
(A) interleukin-15 (IL-15);
(B) interleukin-15 receptor-a subunit (IL-15Ra);
(C) an antibody targeting a therapy-related cell surface antigen;
wherein, IL-15 is fused to the N-terminal of the heavy chain variable region of the antibody directly or via a linker, and IL-15Ra is fused to N-terminal of the light chain variable region of the antibody directly or via a liner; or, the IL-15 is fused to N-terminal of the light chain variable region of the antibody, and IL-15Ra is fused to N-terminal of the heavy chain variable region of the antibody directly or via a linker; or, the IL-15 is fused to C-terminal of the light chain constant region (CL) of the antibody directly or via a linker, and IL-15Ra is fused to C-terminal of the heavy chain constant region (CH1) of the antibody directly or via a linker; or the IL-15 is fused to C-terminal of the heavy chain constant region (CH1) of the antibody directly or via a linker, and IL-15Ra is, fused to a C-terminal of the light chain constant region (CL) of the antibody directly or via a linker. In some embodiments, the linker is a cleavable or non-cleavable. In some embodiments, the linker is independently selected from, but not limited to GGGGSGGGGSGGGGSG, GSPLGVRGS, GSPLGVR, PLGVR, GGGGSGPLGVRGGGGSG or GGGGSGPLGVR and the like.

In some embodiments, IL-15 is a mammalian IL-15, preferably, a primate IL-15, more preferably, a human IL-15. In some embodiments, IL-15 is a wild-type IL-15 or IL-15 derivative. In some embodiments, IL-15 has a nucleotide sequence as shown in SEQ ID NO: 71 or an amino acid sequence as shown in SEQ ID NO: 72; in some embodiments, IL-15 has a nucleotide sequence as shown in SEQ ID NO: 111 or an amino acid sequence as shown in SEQ ID NO: 112. In some other embodiments, IL-15 has an amino acid sequence which has a percentage of identity of 85%-100% with SEQ ID NO: 72 or SEQ ID NO: 112.

In some embodiments, the IL-15Ra is IL-15Ra. In some embodiments, the IL-15Ra is an IL-15Ra Sushi domain. In some embodiments, IL-15Ra is a variant or derivative of IL-15Ra Sushi domain. In some other embodiments, IL-15Ra has a nucleotide sequence as shown in SEQ ID NO: 73 or an amino acid sequence as shown in SEQ ID NO: 74. In some other embodiments, IL-15Ra has a nucleotide sequence as shown in SEQ ID NO: 87 or an amino acid sequence as shown in SEQ ID NO: 88. In some other embodiments, IL-15Ra has an amino acid sequence which has a percentage of identity of 85%-100% with SEQ ID NO: 74 or SEQ ID NO: 88.

In some other embodiments, the therapy-related cell surface antigen is a CD antigen. In some other embodiments, the CD antigen is selected from but not limited to, CD19, CD20, CD47, CD40, CD73, CD33, CD38, CD123, CD30, CD3, CD22, CD25, CD133, CD27, CD39, CD138, CD46, CD56, CD70, CD32, CD11b, CD135, CD171, CD174, CD147, CD155, CD16, CD162, CD16a, CD200, CD21, CD28, CD44, CD52, CD54, CD7, CD80, CD88, CD13, CD130, CD150, CD160, CD200R1, CD267, CD29, CD3E, CD4, CD51 or CD8, or the like.

In some other embodiments, the therapy-related cell surface antigen is an immune checkpoint protein. In some other embodiments, the immune checkpoint protein is selected from but not limited to PD-1, PD-L1, CTLA-4, LAG-3, OX40, CD28, CD40, CD47, CD70, CD80, CD122, GTIR, A2AR, B7-H3 (CD276), B7-H4, IDO, KIR, Tim-3 or 4-1BB(CD137), or the like. In some other embodiments, the immune checkpoint protein is PD-1. In some other embodiments, the antibody targeting the immune checkpoint protein PD-1 has sequences of HCDR1, HCDR2 and HCDR3 contained in heavy chain as shown in SEQ ID NO: 76, and sequences of LCDR1, LCDR2 and LCDR3 contained in light chain as shown in SEQ ID NO: 78; or has a VH sequence contained in the heavy chain as shown in SEQ ID NO: 76, and a VL sequence contained in the light chain as shown in SEQ ID NO: 78. In some embodiments, the antibody targeting the immune checkpoint protein PD-1 has a heavy chain nucleotide sequence as shown in SEQ ID NO: 75 and a light chain nucleotide sequence as shown in SEQ ID NO: 77; or has a heavy chain amino acid sequence as shown in SEQ ID NO: 76 and a light chain amino acid sequence as shown in SEQ ID NO: 78.

In some other embodiments, the immune checkpoint protein is PD-L1. In some other embodiments, antibody targeting the immune checkpoint protein PD-L1 has sequences of HCDR1, HCDR2 and HCDR3 contained in heavy chain as shown in SEQ ID NO: 36, and sequences of LCDR1, LCDR2 and LCDR3 contained in light chain as shown in SEQ ID NO: 38; or has sequences of HCDR1, HCDR2 and HCDR3 contained in heavy chain as shown in SEQ ID NO: 80, and sequences of LCDR1, LCDR2 and LCDR3 contained in light chain as shown in SEQ ID NO: 82. In some other embodiments, the antibody targeting the immune checkpoint protein PD-L1 has a VH sequence contained in an amino acid sequence as shown in SEQ ID NO: 36, and a VL sequence contained in an amino acid sequence as shown in SEQ ID NO: 38; or has a VH sequence contained in the heavy chain as shown in SEQ ID NO: 80, and a VL sequence contained in the light chain as shown in SEQ ID NO: 82. In some embodiments, the antibody targeting the immune checkpoint protein PD-L1 has a heavy chain nucleotide sequence as shown in SEQ ID NO: 35 and a light chain nucleotide sequence as shown in SEQ ID NO: 37; or has a heavy chain amino acid sequence as shown in SEQ ID NO: 36 and a light chain amino acid sequence as shown in SEQ ID NO: 38. In some embodiments, the antibody targeting the immune checkpoint protein PD-L1 has a heavy chain nucleotide sequence as shown in SEQ ID NO: 79 and a light chain nucleotide sequence as shown in SEQ ID NO: 81; or has a heavy chain amino acid sequence as shown in SEQ ID NO: 80 and a light chain amino acid sequence as shown in SEQ ID NO: 82.

In some other embodiments, the therapy-related cell surface antigen is a tumor surface antigen. In some other embodiments, the tumor surface antigen is selected from but not limited to EGFR, HER2, HER3, HER4, NY-ESO-1, GPC-3, CLL-1, BCMA, GD2, EpCAM, a chemical chemokine receptor family (CCR1, CCR2, CCR3, CCR4, CCR5, CCR6, CCR7, CCR8, CCR9, CCR10, CCL27, CCL28, CX3CR1, CXCR1, CXCR2, CXCR3, CXCR4, CXCR5, CXCR6), a mucin family (MUC1, MUC2, MUC3A, MUC3B, MUC4, MUC5AC, MUC5B, MUC6, MUC7, MUC8, MUC12, MUC13, MUC15, MUC16, MUC17, MUC19, MUC20), PSMA, CEA, HDAC6, Mesothelin, TERT, TLR, TLR9, TLR4, CD33, GITR, Survivin, CD123, TIGIT, fibroblast growth factor receptors (FGFR), vascular endothelial growth factors (FLT1, KDR/Flk-1, and VEGFR-3), hepatocyte growth factor receptors (HGFR), nerve growth factor receptors (NGFR), insulin-like growth factor receptors (IGFR), platelet-derived growth factor receptors (PDGFR), and hormone receptors (melanocortin 1 receptors (MC1R, MSHR), and the like.

In some other embodiments, the tumor surface antigen is EGFR. In some other embodiments, the antibody targeting EGFR has sequences of HCDR1, HCDR2 and HCDR3 contained in heavy chain as shown in SEQ ID NO: 32, and sequences of LCDR1, LCDR2 and LCDR3 contained in light chain as shown in SEQ ID NO: 34; or has a VH sequence contained in the heavy chain as shown in SEQ ID NO: 32, and a VL sequence contained in the light chain as shown in SEQ ID NO: 34. In some embodiments, the antibody targeting the tumor surface antigen EGFR has a heavy chain nucleotide sequence as shown in SEQ ID NO: 29 and a light chain nucleotide sequence as shown in SEQ ID NO: 33; or has a heavy chain amino acid sequence as shown in SEQ ID NO: 30 and a light chain amino acid sequence as shown in SEQ ID NO: 34. In some embodiments, the antibody targeting the tumor surface antigen EGFR has a heavy chain nucleotide sequence as shown in SEQ ID NO: 31 and a light chain nucleotide sequence as shown in SEQ ID NO: 33; or has a heavy chain amino acid sequence as shown in SEQ ID NO: 32 and a light chain amino acid sequence as shown in SEQ ID NO: 34.

In some other embodiments, the tumor surface antigen is HER2. In some other embodiments, the antibody targeting HER2 has sequences of HCDR1, HCDR2 and HCDR3 contained in heavy chain amino as shown in SEQ ID NO: 18, and sequences of LCDR1, LCDR2 and LCDR3 contained in light chain as shown in SEQ ID NO: 20; or the antibody targeting HER2 has a VH sequence contained in the heavy chain as shown in SEQ ID NO: 18, and a VL sequence contained in the light chain as shown in SEQ ID NO: 20. In some embodiments, the antibody targeting HER2 has a heavy chain nucleotide sequence as shown in SEQ ID NO: 17 and a light chain nucleotide sequence as shown in SEQ ID NO: 19; or has a heavy chain amino acid sequence as shown in SEQ ID NO: 18 and a light chain amino acid sequence as shown in SEQ ID NO: 20.

In some other embodiments, the cell surface antigen is selected from a virus-associated antigen. In some other embodiments, the virus-associated antigen is selected from but not limited to RSV F, HPV E6, HPV E7, HPV L2, HPV 16, HPV E6/7, HPV L1, HPV16 E6, HPV16 E7, and the like. In some other embodiments, the cell surface antigen is RSV F. In some other embodiments, the antibody targeting an F protein of a RSV virus has sequences of HCDR1, HCDR2 and HCDR3 contained in the heavy chain as shown in SEQ ID NO: 54, and sequences of LCDR1, LCDR2 and LCDR3 contained in the light chain as shown in SEQ ID NO: 56; or has a VH sequence contained in the heavy chain as shown in SEQ ID NO: 54, and a VL sequence contained in the light chain as shown in SEQ ID NO: 56. In some embodiments, the antibody targeting a RSV F protein of a virus-associated antigen has a heavy chain nucleotide sequence as shown in SEQ ID NO: 53 and a light chain nucleotide sequence as shown in SEQ ID NO: 55; or has a heavy chain amino acid sequence as shown in SEQ ID NO: 54 and a light chain amino acid sequence as shown in SEQ ID NO: 56.

In some embodiments, the immunocytokine comprises a heavy chain and a light chain having the following amino acid sequences, respectively: SEQ ID NO: 2 and SEQ ID NO: 8; SEQ ID NO: 6 and SEQ ID NO: 4; SEQ ID NO: 68 and SEQ ID NO: 8; SEQ ID NO: 70 and SEQ ID NO: 4; SEQ ID NO: 10 and SEQ ID NO: 12; SEQ ID NO: 14 and SEQ ID NO: 16; SEQ ID NO: 22 and SEQ ID NO: 24; SEQ ID NO: 26 and SEQ ID NO: 28; SEQ ID NO: 40 and SEQ ID NO: 24; SEQ ID NO: 44 and SEQ ID NO: 28; SEQ ID NO: 46 and SEQ ID NO: 48; SEQ ID NO: 42 and SEQ ID NO: 58; SEQ ID NO: 84 and SEQ ID NO: 86; SEQ ID NO: 60 and SEQ ID NO: 62; SEQ ID NO: 64 and SEQ ID NO: 66; SEQ ID NO: 115 and SEQ ID NO: 116; SEQ ID NO: 117 and SEQ ID NO: 118; SEQ ID NO: 119 and SEQ ID NO: 12; SEQ ID NO: 120 and SEQ ID NO: 16. A person skilled in the art will readily understands that the "heavy chain" of the immunocytokine in the present disclosure refers to a polypeptide chain containing IL-15 or IL-15Ra-fused heavy chain variable region of an antibody targeting a therapy-related cell surface antigen, and the "light chain" of the immunocytokine refers to a polypeptide chain containing IL-15 or IL-15Ra-fused light chain variable region of an antibody targeting a therapy-related cell surface antigen.

In some embodiments, the immunocytokine comprises a heavy chain and a light chain having the following amino acid sequences, respectively: SEQID NO:1 and SEQ ID NO: 7; SEQ ID NO: 5 and SEQ ID NO: 3; SEQ ID NO: 67 and SEQ ID NO: 7; SEQ ID NO: 69 and SEQ ID NO: 3; SEQ ID NO: 9 and SEQ ID NO: 11; SEQ ID NO: 13 and SEQ ID NO: 15; SEQ ID NO: 21 and SEQ ID NO: 23; SEQ ID NO: 25 and SEQ ID NO: 27; SEQ ID NO: 39 and SEQ ID NO: 23; SEQ ID NO: 43 and SEQ ID NO: 27; SEQ ID NO: 45 and SEQ ID NO: 47; SEQ ID NO: 41 and SEQ ID NO: 57; SEQ ID NO: 83 and SEQ ID NO: 85; SEQ ID NO: 59 and SEQ ID NO: 61; SEQ ID NO: 63 and SEQ ID NO: 65.

One aspect of the present disclosure provides an immunocytokine, and the immunocytokine includes:
(A) interleukin-15 (IL-15);
(B) an antibody targeting a therapy-related cell surface antigen;
where, IL-15 is fused to N-terminal of the heavy chain constant region and/or N-terminal of the light chain variable region of the antibody directly or via a linker; or IL-15 is fused to C-terminal of the heavy chain constant region and/or a C terminal of the light chain constant region of the antibody.

In some embodiments, the linker is cleavable or non-cleavable. In some embodiments, the linker is independently selected from but not limited GGGGSGGGGSGGGGSG, GSPLGVRGS, GSPLGVR, PLGVR, GGGGSGPLGVRGGGGSG or GGGGSGPLGVR.

In some embodiments, IL-15 is a mammalian IL-15, preferably, a primate IL-15, more preferably, a human IL-15. In some embodiments, the IL-15 is wild-type IL-15 or IL-15 derivatives. In some embodiments, the IL-15 has a nucleotide sequence as shown in SEQ ID NO: 71 or an amino acid sequence as shown in SEQ ID NO: 72; in some embodiments, the IL-15 has a nucleotide sequence as shown in SEQ ID NO: 111 or an amino acid sequence as shown in SEQ ID NO: 112. In some other embodiments, the IL-15 has an amino acid sequence which has a percentage of identity of 85%-100% with SEQ ID NO: 72 or SEQ ID NO: 112.

In some other embodiments, the therapy-related cell surface antigen is a tumor surface antigen. In some other embodiments, the tumor surface antigen is selected from but not limited to EGFR, HER2, HER3, HER4, NY-ESO-1, GPC-3, CLL-1, BCMA, GD2, EpCAM, a chemical chemokine receptor family (CCR1, CCR2, CCR3, CCR4, CCR5, CCR6, CCR7, CCR8, CCR9, CCR10, CCL27, CCL28, CX3CR1, CXCR1, CXCR2, CXCR3, CXCR4, CXCR5, CXCR6), a mucin family (MUC1, MUC2, MUC3A, MUC3B, MUC4, MUC5AC, MUC5B, MUC6, MUC7, MUC8, MUC12, MUC13, MUC15, MUC16, MUC17, MUC19, MUC20), PSMA, CEA, HDAC6, Mesothelin, TERT, TLR, TLR9, TLR4, CD33, GITR, Survivin, CD123, TIGIT, fibroblast growth factor receptors (FGFR), vascular endothelial growth factors (FLT1, KDR/Flk-1, and VEGFR-3), hepatocyte growth factor receptors (HGFR), nerve growth factor receptors (NGFR), insulin-like growth factor receptors (IGFR), platelet-derived growth factor receptors (PDGFR), and hormone receptors (melanocortin 1 receptor (MC1R, MSHR), and the like.

In some other embodiments, the tumor surface antigen is EGFR. In some other embodiments, the antibody targeting EGFR has sequences of HCDR1, HCDR2 and HCDR3 contained in the heavy chain as shown in SEQ ID NO: 32, and sequences of LCDR1, LCDR2 and LCDR3 contained in the light chain as shown in SEQ ID NO: 34; or has a VH sequence contained in the heavy chain as shown in SEQ ID NO: 32, and a VL sequence contained in the light chain as shown in SEQ ID NO: 34. In some embodiments, the antibody targeting the tumor surface antigen EGFR has a heavy chain nucleotide sequence as shown in SEQ ID NO: 29 and a light chain nucleotide sequence as shown in SEQ ID NO: 33; or has a heavy chain amino acid sequence as shown in SEQ ID NO: 30 and a light chain amino acid sequence as shown in SEQ ID NO: 34. In some embodiments, the antibody targeting EGFR has a heavy chain nucleotide sequence as shown in SEQ ID NO: 31 and a light chain nucleotide sequence as shown in SEQ ID NO: 33; or has a heavy chain amino acid sequence as shown in SEQ ID NO: 32 and a light chain amino acid sequence as shown in SEQ ID NO: 34.

In some other embodiments, the tumor surface antigen is HER2. In some other embodiments, the antibody targeting HER2 has sequences of HCDR1, HCDR2 and HCDR3 contained in the heavy chain as shown in SEQ ID NO: 18, and sequences of LCDR1, LCDR2 and LCDR3 contained in the light chain as shown in SEQ ID NO: 20; or the antibody targeting HER2 has a VH sequence contained in the heavy chain as shown in SEQ ID NO: 18, and a VL sequence contained in the light chain as shown in SEQ ID NO: 20. In some embodiments, the antibody targeting HER2 has a heavy chain nucleotide sequence as shown in SEQ ID NO: 17 and a light chain nucleotide sequence as shown in SEQ ID NO: 19; or has a heavy chain amino acid sequence as shown in SEQ ID NO: 18 and a light chain amino acid sequence as shown in SEQ ID NO: 20.

In some embodiments, the immunocytokine comprises a heavy chain and a light chain having the following amino acid sequences, respectively: SEQ ID NO: 90 and SEQ ID NO: 34; SEQ ID NO: 92 and SEQ ID NO: 34; SEQ ID NO: 94 and SEQ ID NO: 34; SEQ ID NO: 96 and SEQ ID NO: 34; SEQ ID NO: 98 and SEQ ID NO: 20; SEQ ID NO: 100 and SEQ ID NO:20; SEQ ID NO: 110 and SEQ ID NO: 102; SEQ ID NO: 110 SEQ ID NO: 104; SEQ ID NO: 98 and SEQ ID NO: 102; SEQ ID NO: 98 and SEQ ID NO: 104; SEQ ID NO: 100 and SEQ ID NO: 102; SEQ ID NO: 100 and SEQ ID NO: 104; SEQ ID NO: 106 and SEQ ID NO: 20; SEQ ID NO: 110 and SEQ ID NO: 108; SEQ ID NO: 106 and SEQ ID NO: 108; SEQ ID NO: 113 and SEQ ID NO: 34; SEQ ID NO: 114 and SEQ ID NO: 34.

In some embodiments, the immunocytokine comprises a heavy chain and a light chain having the following amino acid sequences, respectively: SEQ ID NO: 89 and SEQ IDNO: 33; SEQ ID NO: 91 and SEQ ID NO: 33; SEQ ID NO: 93 and SEQ ID NO: 33; SEQ ID NO: 95 and SEQ ID NO: 33; SEQ ID NO: 97 and SEQ ID NO: 19; SEQ ID NO: 99 and SEQ ID NO: 19; SEQ ID NO: 109 and SEQ ID NO: 101; SEQ ID NO: 109 and SEQ ID NO: 103; SEQ ID NO: 97 and SEQ ID NO: 101; SEQ ID NO: 97 and SEQ ID NO: 103; SEQ ID NO: 99 and SEQ ID NO: 101; SEQ ID NO: 99 and SEQ ID NO: 103; SEQ ID NO: 105 and SEQ ID NO: 19; SEQ ID NO: 109 and SEQ ID NO: 107; SEQ ID NO: 105 and SEQ ID NO: 107.

In one aspect, the present disclosure provides a nucleic acid encoding immunocytokine as described above.

In one aspect, the present disclosure provides a vector containing the nucleic acid sequence of the immunocytokine as described above.

In one aspect, the present disclosure provides a host cell containing the vector as described above.

In one aspect, the present disclosure provides a pharmaceutical composition containing a pharmaceutically acceptable carrier or formulation and the immunocytokine as described above.

In a further aspect, the present disclosure provides a method for treating inflammatory disease or cancer in a subject in need thereof, where the method includes administering to the subject a therapeutically effective amount of a composition containing the immunocytokine in a pharmaceutically acceptable form.

### Brief Description of the Drawings

Drawings of the description constituting the present application are used to provide a further understanding to the prevent disclosure. The exemplary embodiments and description of the prevent disclosure are intended to explain the prevent disclosure, and don't constitute an improper limitation of the present disclosure. In the drawings:
FIG. 1 is an SDS-PAGE image showing antibody-based immunocytokine constructs (names are referring to Table 1). In each picture of FIG. 1, M denotes a protein marker, A denotes BSIC-01; B denotes BSIC-10; C denotes BSIC-09; D denotes BSIC-05; E denotes BSIC-06; and F denotes BSIC-03. "-" denotes sample loaded without beta-mercaptoethanol; "+" denotes sample loaded with beta-mercaptoethanol.
FIG. 2 (FIG. 2A- FIG. 2F) shows the gel chromatography result of the antibody-based immunocytokine, where FIG.2A denotes BSIC-03; FIG.2B denotes BSIC-05; FIG.2C denotes BSIC-06; FIG.2D denotes BSIC-09; FIG.2E denotes BSIC-10; and FIG.2F denotes BSIC-24.
FIG. 3 shows the ELISA result of the antibody-based immunocytokine binding to IL-15 (FIG. 3A) or IL-15Ra Sushi domain (FIG. 3B), wherein positive 1 denotes IL-15Ra Sushi-Fc fusion protein (Novoprotein); and positive 2 denotes IL-15-Fc fusion protein (GenScript).
FIG. 4 shows the results of Mo7e cell proliferation promoted by antibody-based immunocytokine, where positive 3 denotes a heterodimer formed by IL-15 and IL-15Ra Sushi-Fc via noncovalent interaction.

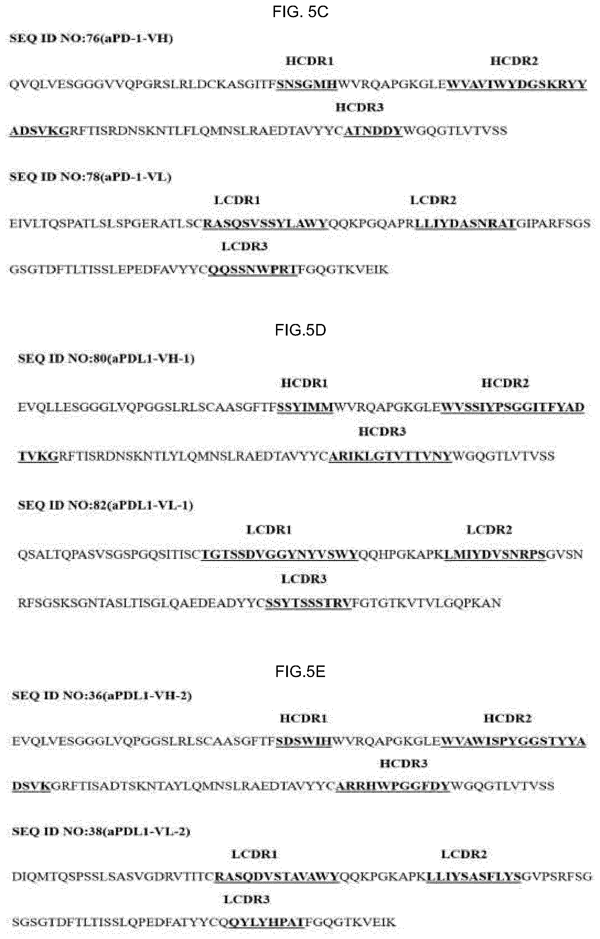

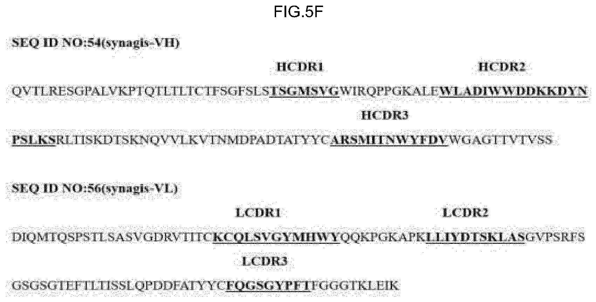

FIG. 5 shows antibody heavy chain and light chain variable regions of aEGFR (FIG.5A), aHER2 (FIG.5B), aPD1 (FIG.5C), aPDL1 (FIG.5D and FIG.5E), and synagis (FIG.5F), the underlined portion denotes complementary determining regions (CDRs).
FIG. 6 shows the proliferation result of lymphocyte from spleen (6A) and PBMC (6B) stimulated by antibody-based immunocytokine , where positive 3 denotes a heterodimer formed by IL-15 and IL-15Ra Sushi-Fc via noncovalent interaction; negative denotes DPBS.
FIG. 7 shows ELISA result that the antibody-based immunocytokine binds to antigens *in vitro*; where 7A denotes binding of the immunocytokine to hEGFR; 7B denotes binding of the immunocytokine to hPD-L1; and 7C denotes binding of the immunocytokine to hHER2.

### Detailed Description of the Embodiments

The present disclosure will be further described herein by citing the following definitions and references to examples. All the contents of the patents and publications mentioned herein, including all the sequences disclosed in these patents and publications, are hereby incorporated by reference.

The "immunocytokines" mentioned herein refer to fusion of cytokines with antibodies.

The term "antibody" herein is used in its broadest sense and includes various kinds of antibody structures, including but not limited to monoclonal antibodies and their derivatives and engineered antibodies, including, for example, multispecific antibodies (such as, DVD-Ig, CrossMab, ART-Ig, FIT-Ig, Duobody and other bispecific antibodies, trispecific antibodies) and antibody fragments, including but not limited to, Fab, Fab', (Fab')₂, Fv, Diabody, and the like as long as they exhibit the desired binding activity to the therapy-related cell surface antigen, and have a heavy chain variable region and light chain variable region capable of fusion with IL-15 and IL-15Ra at N-terminal. In certain circumstances, for example, when IL-15 or IL-15Ra is fused to C-terminal of a light chain CL or heavy chain CH1, the antibody may also contain the heavy chain constant region or light chain constant region or a portion thereof. In such a circumstance, the antibody may be, for example, in the form of Fab.

The term "CDR" refers to a complementary determining region within the variable region of an immunoglobulin. There are three CDRs in each variable region of the heavy chain and light chain, named CDR1, CDR2 and CDR3, respectively. The term "CDR group" refers to a group of three CDRs that appear in a single variable region capable of binding to an antigen. The exact boundaries of these CDRs have been defined differently according to different systems. The system described by Kabat (Kabat, *et al,* (1987) and (1991)) not only provides an exact residue numbering system applicable to any variable region of antibodies or binding proteins, but also provides exact residue boundaries defining three CDRs in each heavy chain or light chain sequence. These CDRs can be referred to as Kabat CDR. Chothia and colleagues (Chothia and Lesk (1987) J. Mol. Biol. 196:901-917; Chothia, et al, (1989) Nature 342:877-883) have found that certain sub-portions in Kabat CDRs adopt almost the same peptide framework conformation even though there is great diversity at the amino acid sequence level. These sub-portions are named L1, L2 and L3 or H1, H2 and H3, where "L" and "H" refer to light chain and heavy chain regions, respectively. These regions may be referred to as Chothia CDRs, which have borders that overlap with Kabat CDRs. Other boundaries defining CDRs that overlap with Kabat CDRs have been described by Padlan (1995) FASEB J. 9:133-139 and MacCallum (1996) J. Mol. Biol. 262(5):732-45). There are other CDR boundary definitions that may not strictly follow one of the systems herein, but will still overlap with Kabat CDR, although they can be shortened or lengthened in view of the predictions or experiments that a specific residue or residue group or even the whole CDR does not significantly influence antigen binding. The methods of CDR definition by Kabat and Contact are combined herein (see details on http: //www.bioinf.ore.uk/abs/).

The term "homology" or "identity" refers to the identity of primary sequence between two polymer molecules (for example, between two nucleic acid molecules (e.g., between two DNA molecules or two RNA molecules) or two polypeptide molecules). When a certain position of the primary sequence in these two molecules is occupied by a same monomeric c subunit, for example, if a certain position in each molecule of two DNA molecules is occupied by adenine, the two molecules are homologous or same at that position. Identity between two sequences varies directly with the number of matching positions or homologous positions. For example, if half of the positions in two sequences (for example, 5 positions in a polymer of 10 subunits in length) are homologous, the two sequences are 50% homologous (also called 50% homology or 50% identity); if 90% of the positions (for example, 9 positions in 10 positions) are matched or homologous, the two sequences are 90% homologous (also called 90% homology or 90% identity).

The "IL-15" of the present disclosure is a mammalian interleukin 15 (IL-15), preferably, a primate IL-15, more preferably a human IL-15. The "IL-15" of the present disclosure refers to an IL-15 derivative or variant which has an amino acid sequence having at least a percentage of identity of 85% (namely, equivalent to about 20 amino acid changes), preferably at least a percentage of identity of 99% (change of about 1 amino acid) to the amino acid sequence as shown in SEQ ID NO: 72 while still maintaining equivalent physiological activity to that of a wild-type IL-15. A person skilled in the art can identify such a derivative based on the knowledge and the technical teaching of the disclosure. It should also be understood that natural amino acids may be substituted by chemically modified amino acids. Usually, such chemically modified amino acids have an improved polypeptide half-life (see WO/2015/131994). With regard to the definition of the "percentage of identity" between the two amino acid sequences used previously, see WO/2015/131994.

Preferably, the IL-15 derivative or variant may be an IL-15 agonist or superagonist. A person skilled in the art can identify the IL-15 agonist or superagonist based on the method of the prior art. As examples of the IL-15 agonists or superagonists, those disclosed in WO2005/085282 or Zhu, et al. (J.Immunol, vol. 183 (6), p:3598-607, 2009) can be cited.

More preferably, the IL-15 agonist or superagonist includes a substitution selected from the following group consisting L45D, L45E, S51D, L52D, N72D, N72E, N72A, N72S, N72Y and N72P (the amino acid positions above are referring to human IL-15 as shown in SEQ ID NO.: 72).

The "IL-15Ra Sushi domain" of the present disclosure has the general meaning well known in the art. The Sushi domain is the Sushi domain of mammalian IL-15Ra, preferably, Sushi domain of a primate IL-15Ra, and more preferably, Sushi domain of a human IL-15Ra. Preferably, the sushi domain containing human IL-15Ra has an amino acid sequence as shown in SEQ ID NO.: 74.

As used herein, the term "derivative of the IL-15 sushi domain" in the present disclosure refers to an amino acid sequence with at least a percentage of identity of 85% (namely, equivalent to about 10 amino acid changes), preferably a derivative with at least a percentage of identity of 99% (about 1 amino acid change) to the amino acid sequence as shown in SEQ ID NO: 74. A person skilled in the art can identify such a derivative based on his personal knowledge and the technical teaching of the disclosure. It should also be understood that natural amino acids may be substituted by chemically modified amino acids. Usually, such chemically modified amino acids have improved polypeptide half-life (see WO/2015/131994). With regard to the definition of the "percentage of identity" between the two amino acid sequences used previously, see, for example, WO/2015/131994.

The "therapy-related cell surface antigen" of the present disclosure refers to an antigen expressed on the surface of a cell that can be targeted for IL-15-mediated treatment of diseases. The "therapy-related cell surface antigen" of the present disclosure includes CD antigens, immune checkpoint antigens, tumor-associated antigens, tumor specific antigens, and virus-induced tumor antigens, and may be selected from but not limited to the following antigens: CD19, PD-1, PD-L1, HER2, STAT3, STEAP1, CTLA-4, IDO, NY-ESO-1, CD40, CSF1R, BCMA, MUC1, ADORA2A, CD20, GD2, TLR7, WT1, IFNAR1, CD47; Neoantigen, EGFR, LAG-3, OX40, PSMA, Mesothelin, TERT, TLR, TLR9, 4-1BB, IL2R, TLR4, CD33, GITR, HPV E6, Survivin, CD123, TIGITTIM-3, CD73, HPV E7, TLR3, CD38, EBV, STING, CD22, GPC3, HDAC1, CXCR4, GMCSFR, CD30, CEACAM5, HDAC6, HPV, CD3, MAGE-A3, TNF, PSA, CD25, CEA, EPCAM, CMV, IL12, PRAME, IL12R, 5T4, Beta catenin, CCR2, PMEL, CXCL12, IGF1, CD46, CXCR1, GMCSF, IL15R, ROR1, TGFBR2, CCR4, FLT-3, FOLR1, GCSFR, ICOS, JAK2, KRAS, VISTA, CD133, CD27, CD39, CEACAM6, NKG2D, STAT5, TGFB1, TLR2, USP7, ANG1, ANG2, B7-H3, CLEC12A, IL13RA2, RIG-I, TRP2, VEGF, AFP, Alpha-Gal, COX-2, EPHA2, gp96, MUC16, p53, TGF-β, CD138, CDw136, CS1, CXCR2, EGFRvIII, Gelactin-3, Globo H, GR, IFNAR2, IFNGR1, IL6, JAK1, MLANA, RAS, SLAMF7, TDO, TGFB2, TLR8, ALK, Arginase, CCR1, CD56, CD70, FAP, GD3, IDH1, IL6R, IRAK4, MAGE-A4, MERTK, MIF, PSCA, PTGER4, SIRPA, TGFB, TGFBR1, ACPP, ADORA2B, AR, Brachyury, CA19-9, CD32, CEACAM1, Gastrin, HDAC, HPV L2, IFNAR, IFNGR, IGF1R, IGF2, IL15, IL17R, IL1B, IL7R, JAK, MAGE-A, MAGE-A1, MAGE-A6, P38, , RORC, TLR5, VEGFR2, ADORA3, ATRT, B7-H4, c-KIT, CCR7, CD11b, CD135, CD171, CD174, CDH3, CX3CR1, Gelactin-1, GM3, HLA-A2, HSP70, IL10, IL17, IL2RB, JAK3, MDA5, NKG2A, PBF, PVRIG, SPAM1, URLC10, VEGFR1, ABCB5, ADABP, ADAM17, ADP, AEG1, Alpha-lactalbumin, AMHR2, ASPH, AXL, BCL2, BTE6-LX-8b, BTE6-X-15-7, Carbohydrate antigens, CCL20, CCL3, CCNB1, CD147, CD155, CD16, CD162, CD16a, CD200, CD21, CD28, CD44, CD52, CD54, CD7, CD80, CD88, Claudin 18, cMET, COX2, CSF1, CTCFL, CXCR5, CXCR7, E1A, EIF2AK3, ERG, FGF2, FN1, GC, GM2, gpA33, HBV, Hemagglutinin, HER3, HILPDA, HLA-DR, HMW-MAA, HP59, HPV 16, HPV E6/7, HPV L1, HSP105, HSP65, HVEM, Hyaluronan, IL13RA1, IL2, IL21R, IL8, KIF20A, KIR2DL1, KIR2DL3, LXR, MAGE-A10, MAGE-C2, MammaglobinA, MAPK, MICA, MiHA, MMP-11, MVP, Myeloblastin, N-Myc, NKp46, NLRP3, NR2F6, Oncofetal Antigen, P2RX7, RhoC, SIM-2, SSTR2, SSX2, STAT1, STn, TAG72, TAMA, TFDP3, TGFBR, TSA, TYK2, Tyrosinase, VEGFA, 5' Nucleotidase (Ecto 5' Nucleotidase or CD73 or NT5E or EC 3.1.3.5), ADAM9, AIM2, B7-H6, BAFF-R, BAI1, BARD1, BOB-1, CA9, Cancer testis antigen, CB2, CBLB, CCR9, CD13, CD130, CD150, CD160, CD200R1, CD267, CD29, CD3E, CD4, CD51, CD8, CGEN-XXXX, Claudin 6, CLEC2D, COX, COX-1, CPEB4, CPEG4, CRBN, CRLF2, CSPG4, CTA, CXCL1, CXCR3, Cytosine deaminase, DCK, DKK1, DLL3, DR3, DR5, EBNA3C, EGF, EGFR5, ELVAL4, EPHA3, EPS8, EVI1, FAIM-3, FasR, FCU1, FLT3, FOLR, FOXM1, FSHR, Galectin-3, GalNAc, GARP, Gelactin-9, Gelatcin-1/3/9, GLD18, GNRHR, GP160, GP73, H3.3K27M, HAGE, HDAC2, HDAC8, HPV16 E6, HPV16 E7, HSP, Hypoxia, ICAM, ICAM7, IDO1, IFNG, IFNGR2, IGF2R, IGFBP2, IGK, IL10RA, IL12RB1, IL13, IL13R, IL13Ralpha2, IL15RA, IL17A, IL17B, IL1A, IL1R1, IL1R3, IL21, IL22R, IL27R, IL2RA, IL35, IL9R, Integrin beta-7, IRAK1, ITGB5, Kappa myeloma antigen, KIR2DL2, Kynurenine, L1CAM, Lambda Myeloma Antigen, LAMP, LLO, LXRA, LXRB, Mas receptor, MG7, MHCI, MHCII, MIC, MOSPD2, MRP-3, MRP1, MRP3765, muGNTP01, MYB, MYBL2, NFAT, NGcGM3, Nrf2, p38 MAP Kinase (EC 2.7.11.24), P55, PAM4, PAP, PASD1, PCDH18, PD-L2, PI3K-delta, POTE, PPT, Protein tolemerase, PTGER2, RANKL, RBL001, RNF43, ROR2, S100A9, SEREX, SLAMF1, STAT, TACSTD2, TASTD2, TDO2, TEM, thymidine kinase, Thymidylate synthase, TIE2, TIMP3, TM4SF5, TOP1, TRBC1, TRBC2, TRIF, Tryptophan, TSHR, TWEAK, UTA2-1, VDBP, VRP, VSIG-4, XAGE1, XAGE1A, ZP1, and ZP3.

The term "linker" of the present disclosure refers to two amino acid residues or a polypeptide with two or more amino acid residues used to fusing two polypeptides. Such a linker is well known in the art (see, for example, Holliger, et al. (1993) Proc. Natl. Acad. Sci. USA 90:6444-6448; Poljak, et al. (1994) Structure 2:1121-1123). Suitably, the non-immunogenic linker includes, for example, GS, (G4S)n, (SG4)n, (G4S)n or G4(SG4)n peptide linker. "n" is generally 1-10, and usually 2-4. In some embodiments, the linker may be cleavable linker, for example, a self-cleavable, enzymatically cleavable or chemically cleavable. The entire linker in WO2011/034605 is incorporated herein by reference. Enzymatical cleavage may be performed by, but no limited to endopeptidases or exopeptidases. Non-limiting examples of endopeptidases include MMP2 (matrix metalloproteinase 2), urokinase, Lys-C, Asp-N, Arg-C, V8, Glu-C, chymotrypsin, trypsin, pepsase, papain, thrombin, Genenase, factor Xa, TEV (tobacco etch virus cysteine protease), enterokinase, HRV C3 (human rhinovirus C3 protease), ininogenase, *Bacillus subtilis* protease-like proprotein convertase (for example, Furin (PC1), PC2 or PC3) and N-arginine dibasic convertase. Non-limiting examples of exopeptidases include carboxypeptidase A, carboxypeptidase B, carboxypeptidase D, carboxypeptidase E (also called carboxypeptidase H), carboxypeptidase M, carboxypeptidase N or carboxypeptidase Z.

In some embodiments, chemical cleavage may be performed by hydroxylamine, N-chlorosuccinimide, N-bromo succinimide or cyanogen bromide.

### Examples

It should be indicated that in the case of no conflict, examples in the present application are only illustrative, and are not intended to limit the present disclosure in any way.

### Examples

### Example 1 Construction of an antibody-based immunocytokine

Gene segments encoding heavy chain (H) and light chain (L) of anti-EGFR antibody (aEGFR), Palivizumab (Syn), anti-PD-1 antibody (aPD-1), anti-PD-L1 antibody (aPD-L1), anti-HER2 antibody (aHER2), IL-15, IL-15Ra, or IL-15RaSushi were synthesized, respectively. IL-15 was fused to N-terminal of heavy chain variable region of the antibody or C-terminal of CH1, while IL-15Ra was fused to N-terminal of light chain variable region or C-terminal of light chain, or IL-15 was fused to N-terminal of light chain variable region or a C-terminal of light chain, while IL-15Ra was fused to N-terminal of heavy chain variable region or C-terminal of CH1 by standard molecular biology techniques. All the sequences were verified by sequencing (see sequences in Table 1).

**Table 1 Sequences of the antibody-based immunocytokine**

| Construct | Protein | Compositio n | Nucleotide sequence Seq ID No: | Amino acid sequence Seq ID No: |
|---|---|---|---|---|
| BSIC-1 | IL-15-SynH(null): IL-15RaSushi-SynL | Chain-1 | 1 | 2 |
| | | Chain-2 | 7 | 8 |
| BSIC-2 | IL-15RaShi-SynH(null): IL-15-SynL | Chain-1 | 5 | 6 |
| | | Chain-2 | 3 | 4 |
| BSIC-3 | IL-15-aHER2H(null): IL-15RaSushi-aHER2L | Chain-1 | 9 | 10 |
| | | Chain-2 | 11 | 12 |
| BSIC-4 | IL-15RaSushi-aHER2H(null): IL-15-aHER2L | Chain-1 | 13 | 14 |
| | | Chain-2 | 15 | 16 |
| BSIC-5 | IL-15-aEGFRH: IL-15RaSushi-aEGFRL | Chain-1 | 21 | 22 |
| | | Chain-2 | 23 | 24 |
| BSIC-6 | IL-15RaSushi-aEGFRH: IL-15-aEGFRL | Chain-1 | 25 | 26 |
| | | Chain-2 | 27 | 28 |
| BSIC-7 | IL-15-aEGFRH(null): IL-15RaSushi-aEGFRL | Chain-1 | 39 | 40 |
| | | Chain-2 | 41 | 42 |
| BSIC-8 | IL-15RaSushi-aEGFRH(null): IL15-aEGFRL | Chain-1 | 43 | 44 |
| | | Chain-2 | 27 | 28 |
| BSIC-9 | IL-15-aPDL1H(null): IL-15RaSushi-aPDL1L | Chain-1 | 45 | 46 |
| | | Chain-2 | 47 | 48 |
| BSIC-10 | IL-15RaSushi-aPDL1H(null): IL15-aPDL1L | Chain-1 | 49 | 50 |
| | | Chain-2 | 51 | 52 |
| BSIC-11 | IL-15RaSushi-aPDL1H(null): IL-15-aPDLIL | Chain-1 | 87 | 88 |
| | | Chain-2 | 57 | 58 |
| BSIC-12 | IL-15-aPD1H(null): IL-15RaSushi-aPD1L | Chain-1 | 83 | 84 |
| | | Chain-2 | 85 | 86 |
| BSIC-13 | Syn(null) | Chain-1 | 53 | 54 |
| | | Chain-2 | 55 | 56 |
| BSIC-14 | IL-15-aPDL1H: IL-15RaSushi-aPDL1L | Chain-1 | 59 | 60 |
| | | Chain-2 | 61 | 62 |
| BSIC-15 | IL-15RaSushi-aPDL1H(null): IL-15-aPDL1L | Chain-1 | 63 | 64 |
| | | Chain-2 | 65 | 66 |
| BSIC-16 | IL-15-SynH: IL-15RaSushi-SynL | Chain-1 | 67 | 68 |
| | | Chain-2 | 7 | 8 |
| BSIC-17 | IL-15RaSushi-SynH: IL-15-SynL | Chain-1 | 69 | 70 |
| | | Chain-2 | 3 | 4 |
| BSIC-18 | aPDL1-1 | Chain-1 | 79 | 80 |
| | | Chain-2 | 81 | 82 |
| BSIC-19 | aHER2 | Chain-1 | 17 | 18 |
| | | Chain-2 | 19 | 20 |
| BSIC-20 | aEGFR | Chain-1 | 29 | 30 |
| | | Chain-2 | 33 | 34 |
| BSIC-21 | aPD1 | Chain-1 | 75 | 76 |
| | | Chain-2 | 77 | 78 |
| BSIC-22 | aEGFR(null) | Chain-1 | 31 | 32 |
| | | Chain-2 | 33 | 34 |
| BSIC-23 | aPDL1(null) | Chain-1 | 35 | 36 |
| | | Chain-2 | 37 | 38 |
| BSIC-24 | IL-15-16GS-aHER2H | Chain-1 | 19 | 20 |
| | | Chain-2 | 9 | 10 |
| BSIC-25 | IL-15-16GS-aHER2L | Chain-1 | 17 | 18 |
| | | Chain-2 | 15 | 16 |
| BSIC-26 | IL-15RaSushi-SynL | Chain-1 | 53 | 54 |
| | | Chain-2 | 7 | 8 |
| BSIC-27 | IL-15RaSushi-SynH(null) | Chain-1 | 5 | 6 |
| | | Chain-2 | 55 | 56 |
| BSIC-28 | IL-15-16GS-SynH(null) | Chain-1 | 1 | 2 |
| | | Chain-2 | 55 | 56 |
| BSIC-29 | IL-15-MP2GS-aEGFRH(null) | Chain-1 | 89 | 90 |
| | | Chain-2 | 33 | 34 |
| BSIC-30 | IL-15-MP2-aEGFRH(null) | Chain-1 | 91 | 92 |
| | | Chain-2 | 33 | 34 |
| BSIC-31 | aEGFRH(null)-MP2GS-IL-15 | Chain-1 | 93 | 94 |
| | | Chain-2 | 33 | 34 |
| BSIC-32 | aEGFRH(null)MP2-IL-15 | Chain-1 | 95 | 96 |
| | | Chain-2 | 33 | 34 |
| BSIC-33 | IL-15WT-MP2GS-aHER2H(n ull) | Chain-1 | 97 | 98 |
| | | Chain-2 | 19 | 20 |
| BSIC-34 | IL-15WT-MP2-aHER2H(null) | Chain-1 | 99 | 100 |
| | | Chain-2 | 19 | 20 |
| BSIC-35 | IL-15WT-MP2GS-aHER2L | Chain-1 | 109 | 110 |
| | | Chain-2 | 101 | 102 |
| BSIC-36 | IL-15WT-MP2-aHER2L | Chain-1 | 109 | 110 |
| | | Chain-2 | 103 | 104 |
| BSIC-37 | IL-15WT-MP2GS-aHER2H(n ull): IL-15WT-MP2GS-aHER2L | Chain-1 | 97 | 98 |
| | | Chain-2 | 101 | 102 |
| BSIC-38 | IL-15WT-MP2GS-aHER2H(n ull): IL-15WT-MP2-aHER2L | Chain-1 | 97 | 98 |
| | | Chain-2 | 103 | 104 |
| BSIC-39 | IL-15WT-MP2-aHER2H(null) : IL-15WT-MP2GS-aHER2L | Chain-1 | 99 | 100 |
| | | Chain-2 | 101 | 102 |
| BSIC-40 | IL-15WT-MP2-aHER2H(null) : IL-15WT-MP2-aHER2L | Chain-1 | 99 | 100 |
| | | Chain-2 | 103 | 104 |
| BSIC-41 | IL-15WT-16GS-aHER2H(null ) | Chain-1 | 105 | 106 |
| | | Chain-2 | 19 | 20 |
| BSIC-42 | IL-15WT-16GS-aHER2L | Chain-1 | 109 | 110 |
| | | Chain-2 | 107 | 108 |
| BSIC-43 | IL-15WT-16GS-aHER2H(null ): IL-15WT-16GS-aHER2L | Chain-1 | 105 | 106 |
| | | Chain-2 | 107 | 108 |
| BSIC-44 | hIL15WT-16GS-aEGFRH | Chain-1 | / | 113 |
| | | Chain-2 | / | 34 |
| BSIC-45 | aEGFRH-16GS-IL15WT | Chain-1 | / | 114 |
| | | Chain-2 | / | 34 |
| BSIC-46 | aEGFR-FabL-hIL15RSUSHI:a EGFR-FabH-hIL15 | Chain-1 | / | 115 |
| | | Chain-2 | / | 116 |
| BSIC-47 | aEGFR-FabL-hIL15RSUSHI:a EGFR-FabH-hIL15 | Chain-1 | / | 117 |
| | | Chain-2 | / | 118 |
| BSIC-48 | IL15-aHER2H: IL15RaSushi-aHER2L | Chain-1 | / | 119 |
| | | Chain-2 | 11 | 12 |
| BSIC-49 | IL15RaSushi-aHER2H: IL15-aHER2L | Chain-1 | / | 120 |
| | | Chain-2 | 15 | 16 |
| IL-15 | IL-15 | / | 71 | 72 |
| IL-15RaSushi | IL-15RaSushi | / | 73 | 74 |
| IL-15Ra | IL-15Ra | / | 87 | 88 |
| IL-15WT | | | 111 | 112 |

| | | | | |
|---|---|---|---|---|
| Note: null denotes that Fc had no ADCC and CDC function or had weakened ADCC and CDC functions. | | | | |

### Example 2 Expression, purification and size-exclusion chromatography (SEC) of the antibody-based immunocytokine

The heavy chain and light chain expression vectors for immunocytokine constructed in Example 1 were transiently co-transfected into Expi 293 cells (ThermoFisher)with a molar ratio of 1:1: 40 ml Expi293 (3-4×10⁶ cell/ml) were seeded in a 125 ml cell culture flask, 80 ug plasmid were diluted with 2 ml Opti-MEM (Invitrogen), and then added to 2 ml Opti-MEM containing 120 µl PEI(Polysciences). After standing at room temperature for 30 min, the plasmid-PEI mixture was added to the cell culture for culture at 125 rpm, 37°C, 5% CO₂. Cell culture supernatant was collected at 96h after transfection, purified by Protein A Resin (Genscript), and then analyzed by SDS-PAGE.

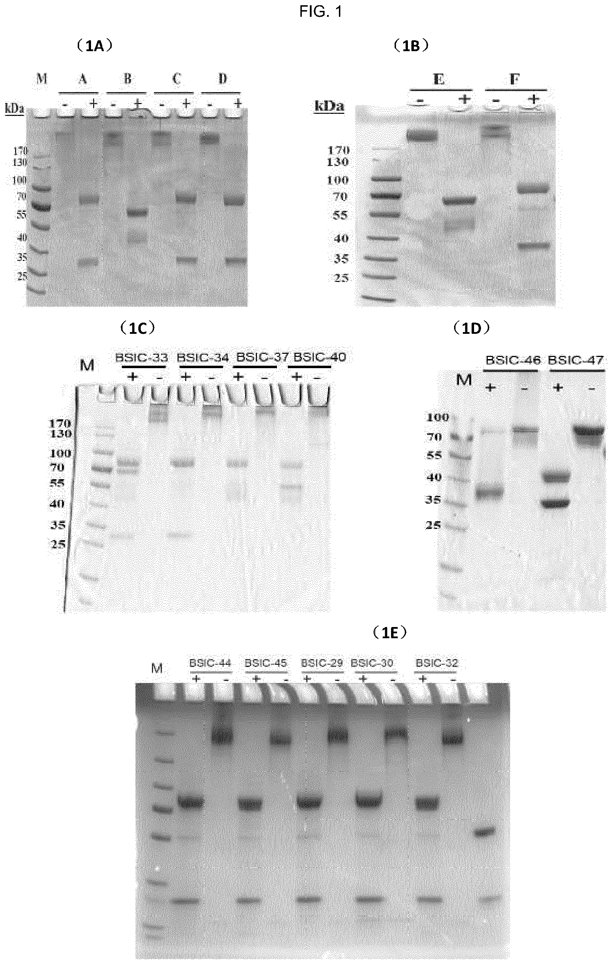

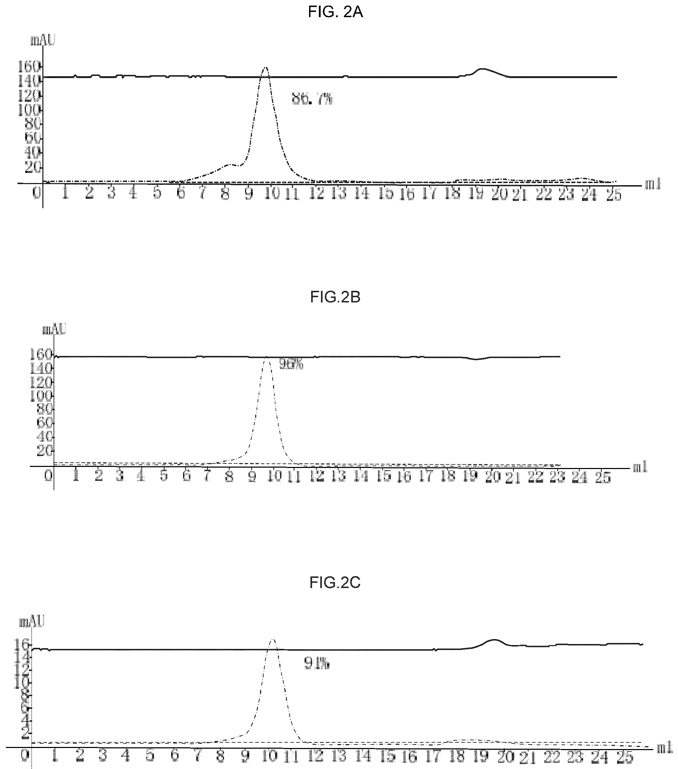

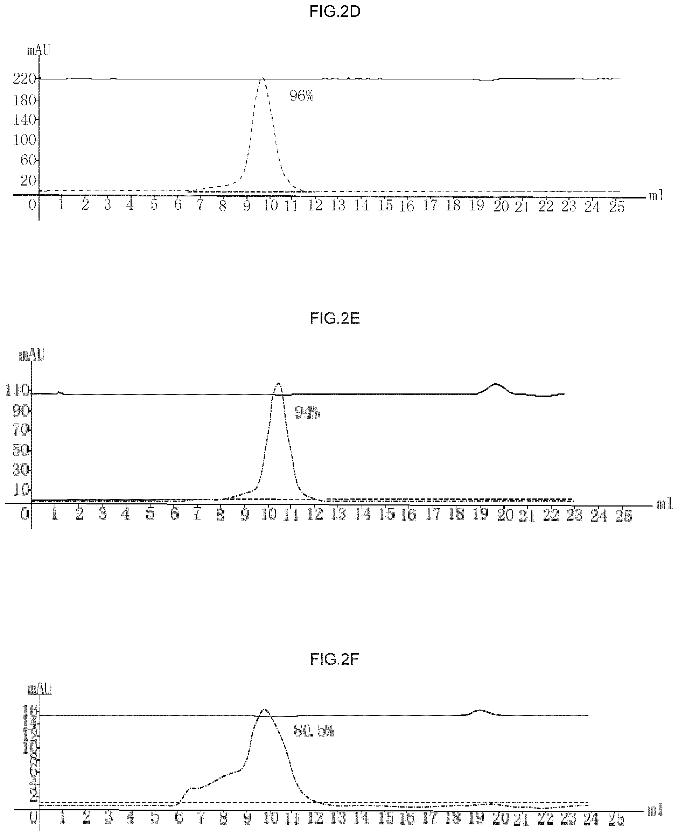

The purified immunocytokine by Protein A resin was subjected to chromatographic analysis via GE's AKTA, and the chromatographic column used was: Superdex 200 Increase 10/300 GL size exclusion chromatographic column. The solution for the size exclusion chromatography was PBS buffer (0.010 M phosphate buffer, 0.0027 M KCI, 0.14 M NaCl, pH=7.4). SDS in FIG. 1 and chromatogram in FIG. 2 indicated that the immunocytokine complex had considerable purity and can be effectively separated by a chromatographic column.

### Example 3 In vitro activity evaluation of the antibody-based immunocytokine

### 3.1 IL-15 or IL-15RaSushi binding activity

hIL-15 (SinoBiological) or IL-15RaSushi (Miltenyi) (100 ng/well) (DPBS buffer, pH=7.4) was coated in a 96-well plate and incubated overnight at 4°C. After blocked by DPBST containing 2% skimmed milk powder for 1 h at room temperature, and washed for 3 times with DPBS containing 0.05% Tween-20, the gradient diluted immunocytokines were added and incubation for 2 h at room temperature. After washed for 4-5 times with DPBS containing 0.05% Tween-20, HRP conjugated anti-human kappa light chain (Cat. A18853, Thermo Fisher Scientific, 1:2000) secondary antibody was added for incubation for 2 h at room temperature, then washed for 4-5 times with DPBS containing 0.05% Tween-20. TMB reagent (BioLegend) was used for color development, and readings was performed at OD450. Data was analyzed by nonlinear regression with a log(agonist) vs. response model via Prizm Graphpad software.

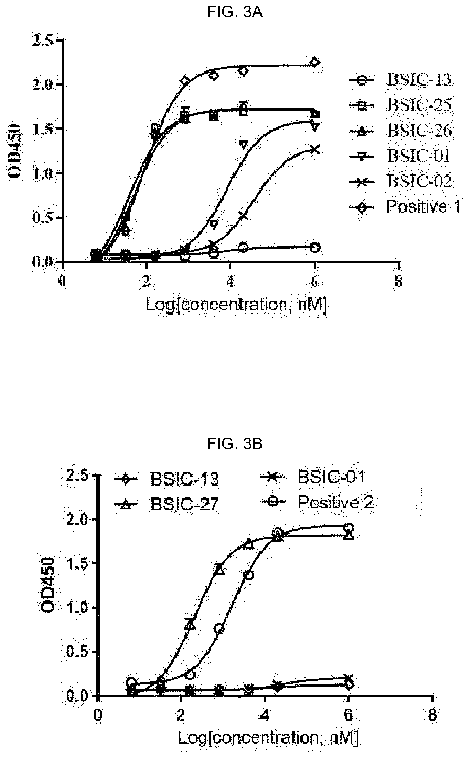

As shown in FIG. 3, BSIC-01 (IL15-SynH(null): IL15RaSushi-SynL) and BSIC-02 (IL15RaShi-SynH(null): IL15-SynL) had relatively low (3A) affinity to IL-15 when compared to positive-1(IL-15RaSushi-Fc), indicating that the IL-15 and IL-15RaSushi in BSIC-01 (IL 15-SynH(null): IL15RaSushi-SynL) or BSIC-02 (IL 15RaShi-SynH(null): IL 15-SynL) formed a stable complex. BSIC-01 (IL15-SynH(null): IL 15RaSushi-SynL) had relatively low (3B) affinity to IL-15 consistent with the framework antibody BSIC-13Syn(null) when compared to positive-2(IL-15-Fc), indicating that IL-15 and IL-15RaSushi in BSIC-01 (IL 15-SynH(null): IL 15RaSushi-SynL) formed a very stable intramolecular complex. BSIC-27 (IL-15RaSushi-SynH(null)) had a higher affinity to IL-15RaSushi.

### 3.2 Antigen binding activity

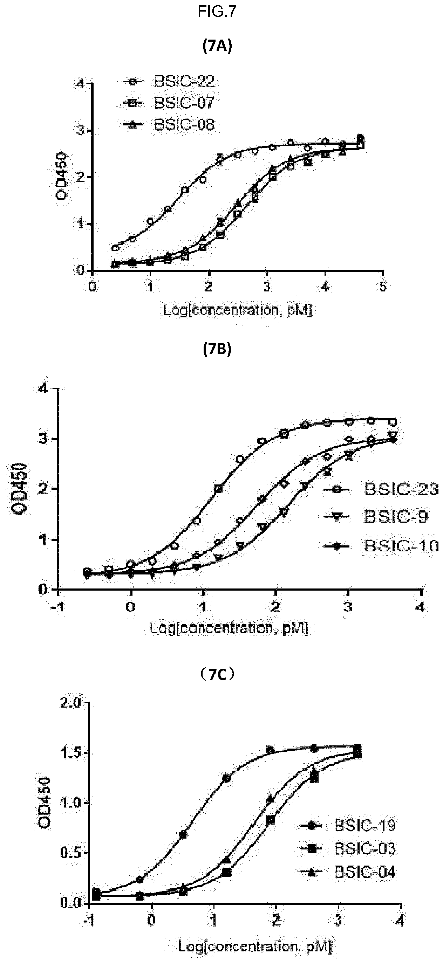

hEGFR-his ( SinoBiological), hHER2(ACRO) or hPDLI (100 ng/well) were coated in a 96-well plate and incubated overnight at 4°C. After blocked by PBST (0.5% Tween-20 in PBS) containing 2% skim milk powder for 1 h at room temperature, the gradient diluted immunocytokines were added and incubated for 2 h at room temperature. After washed for 4-5 times with PBST containing 2% skim milk powder, HRP anti-human kappa light chain (Invitorgen) secondary antibody was added and incubated for 1 h at room temperature, then washed for 4-5 times with PBST containing 2% skim milk powder. Color development was performed with TMB reagent (BioLegend, Cat. 421101), and readings were performed at 450 nm. Or color development was performed with QuantaBlu fluorescent peroxidase substrate (Life technologies, Cat. 15169), and reading was performed at 325 nm and 420 nm. Data was analyzed by nonlinear regression with a specific binding model via Prizm Graphpad software. As shown in Figure 7; the antibody-based immunocytokine had better affinity to antigens.

### Example 4 Mo7e proliferation-promoting assay

Mo7e cells (Cell Resource Center, IBMS, CAMS/PUMC) were cultured in RPMI 1640 medium with 10% fetal bovine serum, 10 ng/ml GM-CSF(Peprotech), and two antibiotics (penicillin-streptomycin). Cell viability should exceed 95% as detected by trypan blue before assay. Cells were washed for twice with RPMI 1640(10%FBS+ two antibiotics (penicillin-streptomycin)), pended to density of 40,000/100 µl, and then added (50µl/well) to a 96-well plate (opaque black bottom). RPMI 1640(10%FBS+ two antibiotics (penicillin-streptomycin)) medium containing the immunocytokines was prepared according to the pre-set concentration. The prepared medium containing the immunocytokine was added(50µl/well) to the 96-well plate (three replicates for each gradient). Afterwards, the 96-well plate was gently shaken to make the cytokine cells distributed evenly, and then incubated. 72 h later, color development was performed by MTS (Promega), and reading was performed at OD490. Or color development was performed by Cell TiterGLO (Promega), and luminescent was read. Data was analyzed by nonlinear regression with a log(agonist) vs. response model via Prizm Graphpad software.

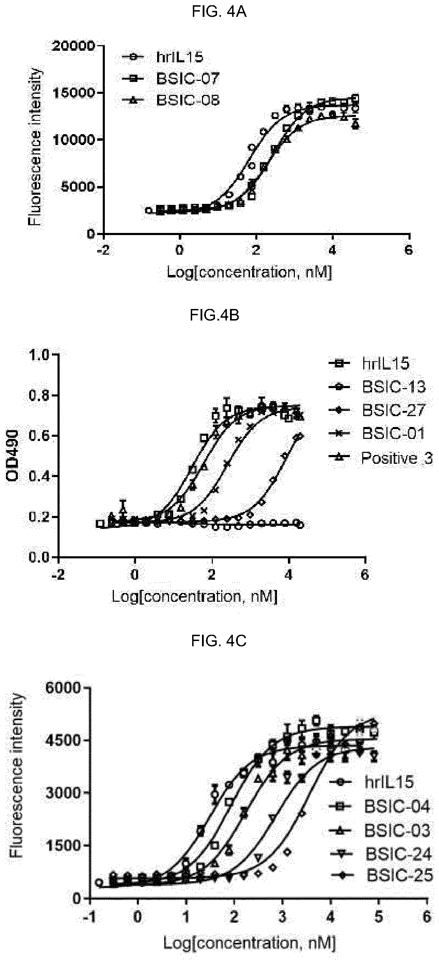

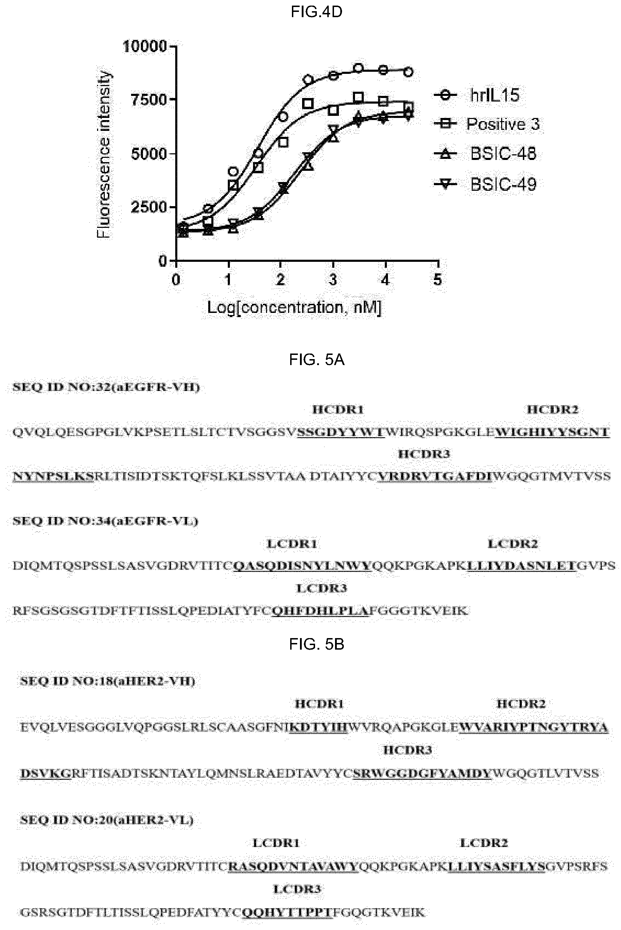

As shown in FIG. 4. The proliferation-promoting effect of BSIC-01 (IL 15-SynH(null): IL15RaSushi-SynL) on Mo7e proliferation was weaker than that of positive-3 (in which IL-15 and IL-15RaSushi-Fc formed a heterodimer via noncovalent interaction) or rhIL-15, but stronger than BSIC-27 (FIG. 4A), indicating that the intramolecular complex formed by IL-15 and IL-15Rasushi via noncovalent interaction may effectively promote the proliferation of Mo7e cells. BSIC-07 (IL15-aEGFRH(null): IL 15RaSushi-aEGFRL) and BSIC-08(IL15RaSushi-aEGFRH(null): IL 15-aEGFRL) had similar proliferation-promoting effect on Mo7e, although slightly weaker than that of rhIL-15. The promoting effect of BSIC-03 (IL15-aHER2H(WT): IL15RaSushi-aHER2L) and BSIC-04 (IL15RaSushi-aHER2(WT): IL15-aHER2L) on Mo7e proliferation was stronger than that of BSIC-24 (IL-15-aHER2H(WT)) or IL-15-aHER2L(WT) (IL-15-aHER2H(WT)), consistent with results from Palivizumab or aEGFR-based constructs.

### Example 5 Lymphocyte activation in vivo

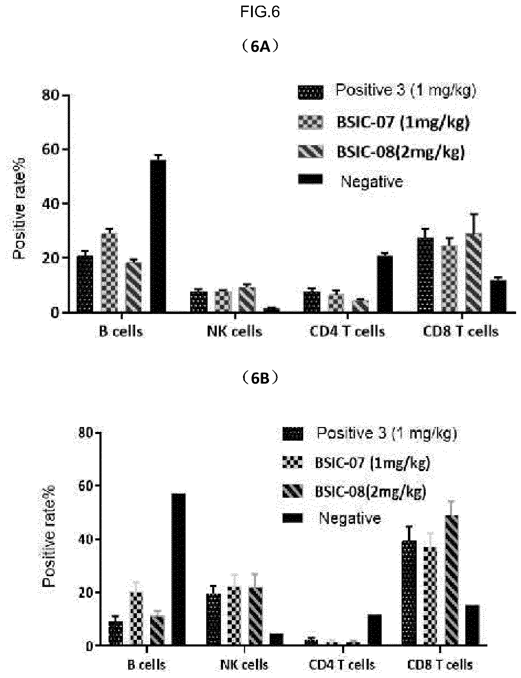

DPBS or immunocytokine was intraperitoneally injected into female C57BL/6 mice (6-7 weeks). Four days later, venous blood was collected from the orbital socket to isolate PBMCs, at the same time, mice were sacrificed and the spleens were taken for preparation of -splenocytes suspension The population percentage of different immune cells in PBMC and spleen was analyzed by flow cytometry with antibodies as follows: PE rat anti-mouse CD19, APC rat anti-mouse CD45, FITC rat anti-mouse CD335, FITC rat anti-mouse CD3, PE rat anti-mouse CD4, PE rat anti-mouse CD8, TruStain FcX plus (anti-mouse CD 16/32)(Biolegend). As shown in FIG. 6, the antibody-based immunocytokine could effectively activate CD8+T cells and NK cells in mice.

## Claims

1. An immunocytokine, comprising:
(A) interleukin-15 (IL-15);
(B) interleukin-15 receptor-a subunit (IL-15Ra);
(C) an antibody targeting a therapy-related cell surface antigen;
wherein:
IL-15 is fused to N-terminal of heavy chain variable region of the antibody with or without a linker while IL-15Ra is fused to N-terminal of light chain variable region of the antibody with or without a linker; or,
IL-15 is fused to N-terminal of light chain variable region of the antibody with or without a linker while IL-15Ra is fused to N-terminal of heavy chain variable region of the antibody with or without a linker; or
IL-15 is fused to C-terminal of light chain constant region (CL) of the antibody with or without a linker while IL-15Ra is fused to C-terminal of heavy chain constant region (CH1) of the antibody with or without a linker; or
IL-15 is fused to C-terminal of heavy chain constant region (CH1) of the antibody with or without a linker while IL-15Ra is fused to a C-terminal of light chain constant region (CL) of the antibody with or without a linker.

2. The immunocytokine of claim 1, wherein IL-15 is a wild-type IL-15 or an IL-15 derivative.

3. The immunocytokine of claim 2, wherein the IL-15 has an amino acid sequence which has a percentage of identity of 85%-100% with SEQ ID NO: 72 or SEQ ID NO: 112, or consists of such an amino acid sequence.

4. The immunocytokine of any one of claims 1-3, wherein IL-15Ra is a full-length IL-15Ra, sushi domain of IL-15Ra or a derivative of IL-15Ra Sushi domain.

5. The immunocytokine of any one of claim 1-3, wherein IL-15Ra has an amino acid sequence which has a percentage of identity of 85%-100% with SEQ ID NO: 74 or SEQ ID NO: 88, or consists of such an amino acid sequence.

6. The immunocytokine of any one of claim 1-5, wherein the therapy-related cell surface antigen is an immune checkpoint protein or a tumor antigen.

7. The immunocytokine of claim 6, wherein the therapy-related cell surface antigen is selected from: an epidermal growth factor receptor family (EGFR, HER2, HER3, HER4), PD-1, PD-L1, STEAP1, CTLA-4, 4-1BB (CD137), OX40, CD28, CD40, CD47, CD70, CD80, CD 122, GTIR, A2AR, B7-H3 (CD276), B7-H4, IDO, KIR, Tim-3, NY-ESO-1, GPC3, CLL-1, BCMA, a mucin family (MUC1, MUC2, MUC3A, MUC3B, MUC4, MUC5AC, MUC5B, MUC6, MUC7, MUC8, MUC12, MUC13, MUC15, MUC16, MUC17, MUC19, MUC20), CD19, CD20, CD22, CD30, CD33, CD52, a chemical chemokine receptor family (CCR1, CCR2, CCR3, CCR4, CCR5, CCR6, CCR7, CCR8, CCR9, CCR10, CCL27, CCL28, CX3CR1, CXCR1, CXCR2, CXCR3, CXCR4, CXCR5, CXCR6), PSMA, CEA, HDAC6, EpCAM, Mesothelin, TERT, TLR, TLR9, TLR4, CD33, GITR, Survivin CD123, TIGIT, TIM-3, CD73, fibroblast growth factor receptors (FGFR), vascular endothelial growth factors (FLT1, KDR/F1k-1, and VEGFR-3), hepatocyte growth factor receptors (HGFR), nerve growth factor receptors (NGFR), insulin-like growth factor receptors (IGFR), platelet-derived growth factor receptors (PDGFR), and hormone receptors (melanocortin 1 receptors (MC1R, MSHR).

8. The immunocytokine of claim 6, wherein the therapy-related cell surface antigen is selected from EGFR, HER2, PD-1, PD-L1, CLL-1, GPC-3, RSVF protein, CD19, CD20, CD22, CD30, CD33 or CD52.

9. The immunocytokine of claim 8, wherein the antibody targeting the therapy-related cell surface antigen is an antibody targeting EGFR, wherein the antibody has sequences of HCDR1, HCDR2 and HCDR3 contained in heavy chain as shown in SEQ ID NO: 32, and sequences of LCDR1, LCDR2 and LCDR3 contained in light chain as shown in SEQ ID NO: 34; or has a VH sequence contained in the heavy chain as shown in SEQ ID NO: 32, and a VL sequence contained in the light chain e as shown in SEQ ID NO: 34.

10. The immunocytokine of claim 8, wherein the antibody targeting the therapy-related cell surface antigen is an antibody targeting HER2, wherein the antibody has sequences of HCDR1, HCDR2 and HCDR3 contained in heavy chain as shown in SEQ ID NO: 18, and sequences of LCDR1, LCDR2 and LCDR3 contained in light chain as shown in SEQ ID NO: 20; or has a VH sequence contained in the heavy chain as shown in SEQ ID NO: 18, and a VL sequence contained in the light chain as shown in SEQ ID NO: 20.

11. The immunocytokine of claim 8, wherein the antibody targeting the therapy-related cell surface antigen is an antibody targeting PD-1, wherein the antibody has sequences of HCDR1, HCDR2 and HCDR3 contained in heavy chain as shown in SEQ ID NO: 76, and sequences of LCDR1, LCDR2 and LCDR3 contained in light chain as shown in SEQ ID NO: 78; or has a VH sequence contained in the heavy chain as shown in SEQ ID NO: 76, and a VL sequence contained in the light chain as shown in SEQ ID NO: 78.

12. The immunocytokine of claim 8, wherein the antibody targeting the therapy-related cell surface antigen is an antibody targeting PD-L1, wherein the antibody has sequences of HCDR1, HCDR2 and HCDR3 contained in heavy chain as shown in SEQ ID NO: 36, and sequences of LCDR1, LCDR2 and LCDR3 contained in light chain as shown in SEQ ID NO: 38; or has sequences HCDR1, HCDR2 and HCDR3 contained in heavy chain as shown in SEQ ID NO: 80, and sequences of LCDR1, LCDR2 and LCDR3 contained in light chain as shown in SEQ ID NO: 82; or has a VH sequence contained in the amino acid sequence as shown in SEQ ID NO: 36, and a VL sequence contained in the amino acid sequence as shown in SEQ ID NO: 38; or has a VH sequence contained in the heavy chain as shown in SEQ ID NO: 80, and a VL sequence contained in the light chain as shown in SEQ ID NO: 82.

13. The immunocytokine of claim 8, wherein the antibody targeting the therapy-related cell surface antigen is an antibody targeting an F protein of a RSV virus, wherein the antibody has sequences of HCDR1, HCDR2 and HCDR3 contained in heavy chain as shown in SEQ ID NO: 54, and sequences of LCDR1, LCDR2 and LCDR3 contained in light chain as shown in SEQ ID NO: 56; or has a VH sequence contained in the heavy chain as shown in SEQ ID NO: 54, and a VL sequence contained in the light chain as shown in SEQ ID NO: 56.

14. The immunocytokine any one of claims 1-13, wherein the antibody targeting the therapy-related cell surface antigen further comprises an Fc fragment.

15. The immunocytokine of claim 14, wherein the Fc fragment is selected from human IgG1, IgG2, IgG3 or IgG4.

16. The immunocytokine of any one of claims 1-15, wherein the linker is a cleavable linker or non-cleavable linker.

17. The immunocytokine of claim 16, wherein the linker is independently selected from GGGGSGGGGSGGGGSG, GSPLGVRGS, GSPLGVR, PLGVR, GGGGSGPLGVRGGGGSG or GGGGSGPLGVR.

18. The immunocytokine any one of claims 1-17, wherein the immunocytokine comprises a heavy chain and a light chain having the following amino acid sequences, respectively: SEQ ID NO: 2 and SEQ ID NO: 8; SEQ ID NO: 6 and SEQ ID NO: 4; SEQ ID NO: 68 and SEQ ID NO: 8; SEQ ID NO: 70 and SEQ ID NO: 4; SEQ ID NO: 10 and SEQ ID NO: 12; SEQ ID NO: 14 and SEQ ID NO: 16; SEQ ID NO: 22 and SEQ ID NO: 24; SEQ ID NO: 26 and SEQ ID NO: 28; SEQ ID NO: 40 and SEQ ID NO: 24; SEQ ID NO: 44 and SEQ ID NO: 28; SEQ ID NO: 46 and SEQ ID NO: 48; SEQ ID NO: 42 and SEQ ID NO: 58; SEQ ID NO: 84 and SEQ ID NO: 86; SEQ ID NO: 60 and SEQ ID NO: 62; SEQ ID NO: 64 and SEQ ID NO: 66; SEQ ID NO: 115 and SEQ ID NO: 116; SEQ ID NO: 117 and SEQ ID NO: 118; SEQ ID NO: 119 and SEQ ID NO: 12; SEQ ID NO: 120 and SEQ ID NO: 16; SEQ ID NO: 90 and SEQ ID NO: 34; SEQ ID NO: 92 and SEQ ID NO: 34; SEQ ID NO: 94 and SEQ ID NO: 34; SEQ ID NO: 96 and SEQ ID NO: 34; SEQ ID NO: 98 and SEQ ID NO: 20; SEQ ID NO: 100 and SEQ ID NO: 20; SEQ ID NO: 110 and SEQ ID NO: 102; SEQ ID NO: 110 and SEQ ID NO: 104; SEQ ID NO: 98 and SEQ ID NO: 102; SEQ ID NO: 98 and SEQ ID NO: 104; SEQ ID NO: 100 and SEQ ID NO: 102; SEQ ID NO: 100 and SEQ ID NO: 104; SEQ ID NO: 106 and SEQ ID NO: 20; SEQ ID NO: 110 and SEQ ID NO: 108; SEQ ID NO: 106 and SEQ ID NO: 108; SEQ ID NO: 113 and SEQ ID NO: 34; SEQ ID NO: 114 and SEQ ID NO: 34.

19. A nucleic acid, encoding the immunocytokine of any one of claims 1-18.

20. A vector, comprising the nucleic acid of claim 19.

21. A host cell, comprising the vector of claim 20.

22. A pharmaceutical composition, comprising a pharmaceutically acceptable carrier or a formulation and the immunocytokine of any one of claims 1-18, the nucleic acid of claim 19, or the vector of claim 20.

23. A method for treating an inflammatory disease or a cancer of a subject in need thereof, wherein the method comprises administering the subject a therapeutically effective amount of a composition, and the composition comprises the immunocytokine of any one of claims 1-18 in a pharmaceutically acceptable form.
